# EUROPEAN PATENT SPECIFICATION

(11) **EP 3 468 563 B1**
(45) Date of publication and mention of the grant of the patent: **13.09.2023**
(21) Application number: 17810952.6
(22) Date of filing: 07.06.2017
(51) Int. Cl.: A61K 31/7088, A61K 38/54, A61K 45/06, A61K 49/00, C07F 9/54, C07F 9/572, C07F 9/6539, A61P 39/06, A61K 31/06, A61K 47/54

(54) **COMPOUNDS FOR DELIVERING GLUTATHIONE TO A TARGET AND METHODS OF MAKING AND USING THE SAME**
VERBINDUNGEN ZUR ABGABE VON GLUTATHION AN EIN TARGET SOWIE VERFAHREN ZUR HERSTELLUNG UND VERWENDUNG DAVON
COMPOSÉS POUR L'ADMINISTRATION DE GLUTATHION À UNE CIBLE ET PROCÉDÉS POUR LES PRÉPARER ET LES UTILISER

(30) Priority: 09.06.2016 US 201662348030 P
(43) Date of publication of application: 17.04.2019
(73) Proprietor: Oregon State University, Corvallis, OR 97331-2140 (US)
(72) Inventor: BECKMAN, Joseph, S., Corvallis, OR 97331-2140 (US); HAGEN, Tory, M., Corvallis, OR 97331-2140 (US); BEILBY, Pamela, R., Corvallis, OR 97331-2140 (US)
(74) Representative: J A Kemp LLP
(86) International application number: PCT/US2017/036393
(87) International publication number: WO 2017/214297

(56) References cited:
- WO-A1-2013/177293
- US-A1- 2004 063 640
- US-A1- 2011 305 751
- US-A1- 2016 129 070
- US-A1- 2016 129 070
- SHEU S S ET AL: "Targeting antioxidants to mitochondria: A new therapeutic direction", BIOCHIMICA ET BIOPHYSICA ACTA. MOLECULAR BASIS OF DISEASE, AMSTERDAM, NL, vol. 1762, no. 2, 1 February 2006 (2006-02-01), pages 256-265, XP027998206, ISSN: 0925-4439, DOI: 10.1016/J.BBADIS.2005.10.007 [retrieved on 2006-02-01]
- UTHPALA SENEVIRATNE ET AL: "Mechanism-Based Triarylphosphine-Ester Probes for Capture of Endogenous RSNOs", JOURNAL OF THE AMERICAN CHEMICAL SOCIETY, vol. 135, no. 20, 22 May 2013 (2013-05-22) , pages 7693-7704, XP055667633, ISSN: 0002-7863, DOI: 10.1021/ja401565w
- ERIKA BECHTOLD ET AL: "Water-Soluble Triarylphosphines as Biomarkers for Protein S -Nitrosation", ACS CHEMICAL BIOLOGY, vol. 5, no. 4, 16 April 2010 (2010-04-16), pages 405-414, XP055667634, ISSN: 1554-8929, DOI: 10.1021/cb900302u
- ELLEN M. SHULMAN-ROSKES ET AL: "The Partitioning of Phosphoramide Mustard and Its Aziridinium Ions among Alkylation and P-N Bond Hydrolysis Reactions", JOURNAL OF MEDICINAL CHEMISTRY, vol. 41, no. 4, 1 February 1998 (1998-02-01), pages 515-529, XP055667635, US ISSN: 0022-2623, DOI: 10.1021/jm9704659
- MICHAEL P. GAMCSIK ET AL: "NMR studies of the conjugation of mechlorethamine with glutathione", JOURNAL OF MEDICINAL CHEMISTRY, vol. 33, no. 3, 1 March 1990 (1990-03-01), pages 1009-1014, XP055399288, US ISSN: 0022-2623, DOI: 10.1021/jm00165a019
- MURPHY MICHAEL P ET AL: "Targeting antioxidants to mitochondria by conjugation to lipophilic cations", ANNUAL REVIEW OF PHARMACOLOGY AND TOXICOLOGY, ANNUAL REVIEW INC., PALO ALTO, CA, US, vol. 47, 1 January 2007 (2007-01-01), pages 629-656, XP009109550, ISSN: 0362-1642, DOI: 10.1146/ANNUREV.PHARMTOX.47.120505.105110
- YANG et al.: "Does Vitamin E Prevent or Promote Cancer?", Cancer Prevention Research, vol. 5, 3 April 2012 (2012-04-03), pages 701-705, XP055459345, DOI: doi:10.1158/1940-6207.CAPR-12-0045
- FISCHLER et al.: "Chain-like assembly of gold nanoparticles on artificial DNA templates via", Chemical Communications, 29 November 2007 (2007-11-29), pages 169-171, XP055459347,
- Ivana Cacciatore ET AL: "Prodrug Approach for Increasing Cellular Glutathione Levels", Molecules, vol. 15, no. 3, 3 March 2010 (2010-03-03), pages 1242-1264, XP055271483, DOI: 10.3390/molecules15031242

## Description

### FIELD

The present disclosure concerns certain compounds suitable for use in delivering glutathione to various biological target areas, pharmaceutical compositions comprising such compounds, the compounds and pharmaceutical compositions for use in methods of treatment, and methods of making such compounds.

### BACKGROUND

Glutathione (GSH) is the most abundant non-protein thiol and cellular antioxidant. It is present in millimolar concentrations in almost all mammalian cells. Glutathione is a tripeptide that plays a prominent role in redox cycling, protein folding, regulation of cellular proliferation, and metabolism of heavy metals. Glutathione is synthesized in the cytosol in a two-step, ATP-mediated process. Glutamate-cysteine ligase catalyzes the rate-limiting first step, the conjugation of glutamate and cysteine to form γ-glutamylcysteine. The generation of glutathione follows through the linkage of glycine to γ-glutamylcysteine, which is catalyzed by glutathione synthase. Glutathione exists in millimolar concentrations in the cytoplasm and generally (e.g., 96-99%) in a reduced state. Small amounts of glutathione are bound to proteins through mixed disulfides or as the disulfide GSSG. The cell contains three additional compartmentalized pools of glutathione in the nucleus, endoplasmic reticulum, and mitochondria.

Mitochondria demonstrate the delicate balance between proper function and redox homeostasis. As the primary consumer of oxygen, mitochondria can generate reactive oxygen species (ROS) leading to oxidative damage and subsequent mitochondrial dysfunction. Mitochondrial dysfunction has been implicated in biological aging and a wide variety of pathological processes, including Parkinson's disease, amyotrophic lateral sclerosis, and cardiovascular disease. Mitochondrial glutathione plays a role in mitigating oxidative stress generated during normal aerobic metabolism, as well as responding to additional reactive oxidative species (ROS) created under toxic insult or other pathological conditions.

The decline in mitochondrial glutathione often precedes or is coincident with loss of cristae, electron transport deficits, oxidative damage and redox perturbations, and directly lowers the threshold for mitochondrial-driven apoptosis. Conventional methods for delivering glutathione have a long clinical history of preventing toxicological insult when GSH becomes limiting; however, GSH precursors (e.g. N-acetylcysteine) or GSH-esters primarily increase cytosolic GSH. Thus, current therapies do not sustainably remediate mitochondrial GSH loss with age, or under toxicological insults that target mitochondria. The lack of mitochondrial GSH remediation thus represents a distinct barrier to limiting the degree of mitochondrial decay and its attendant consequences on many disease processes. Accordingly, it is clear that the straightforward route of providing amino acid precursors of GSH (e.g. N-acetylcysteine), GSH esters, or antioxidants to "spare" GSH does not reliably sustain steady-state levels in mitochondria even when cytosolic GSH can be remediated. Compounds and methods of making such compounds that can be used as delivery agents of GSH, such as through a membrane structure, are needed in the art.

Sheu et al. (Biochimica et Biophysica Acta 1762 (2006) 256-265) is a review article discussing cellular reactive oxygen species metabolism and its role in pathophysiology, the currently existing antioxidants and possible reasons why they are not effective in ameliorating oxidative stress-mediated diseases, and developments in mitochondrially targeted antioxidants and their future promise for disease treatment.

Murphy et al. (Annu. Rev. Pharmacol. Toxicol. 2007 47:629-56) is a review article relating to work conducted on mitochondria-targeted antioxidants.

US 2016/129070 relates to compositions and methods for the treatment of cancer comprising administering an effective amount of a composition comprising a GSSG and a carrier thereof, such as a liposome.

WO 2013/177293 relates to compounds said to be useful in methods for detecting a reactive metabolite in a sample, e.g., wherein the metabolite is generated from the metabolism of a test compound, and wherein the metabolite and the compound react to form a detectable adduct, e.g., detectable by mass spectrometry.

Cacciatore et al. (Molecules 2010, 15, 1242-1264) is a review article that discusses different strategies for increasing GSH levels by supplying reversible bioconjugates able to cross the cellular membrane more easily than GSH and to provide a source of thiols for GSH synthesis.

### SUMMARY

Disclosed herein are embodiments of compounds that can be used to increase and/or maintain glutathione levels in a cell, subject, or sample by facilitating delivery of glutathione (and/or glutathione precursor compounds) to particular targets within the cell, subject, or sample. The compounds comprise at least one targeting moiety that facilitates delivery of the compound. In particular disclosed embodiments, the targeting moiety facilitates transport of the compounds through membranes.

Also disclosed herein are embodiments of methods of making the compounds and compositions comprising the compounds. Methods of using the compounds also are disclosed. In particular disclosed embodiments, the compounds are for use to treat, ameliorate, and/or prevent a disease or condition associated with low or reduced glutathione levels. In exemplary embodiments, the low or reduced glutathione levels are attributed to low mitochondrial levels of glutathione.

Specifically, the present invention is as defined in the appended set of claims.

The foregoing and other objects, features, and advantages of the present disclosure will become more apparent from the following detailed description, which proceeds with reference to the accompanying figures.

### BRIEF DESCRIPTION OF THE DRAWINGS

FIG. 1 is a mass spectrum for triphenylphosphonium tetraethyl glutathione disulfide (Et₄GSSG-TPP₂); the peak at m/z 707.42 is a doubly-charged peak that reflects an actual mass of 1415 (not including the mass of the counter ion).
FIG. 2 is a chromatogram showing an HPLC trace, which illustrates that triphenylphosphonium (TPP) GSH (line labeled "TPP-GSH") accumulates in cardiac mitochondria along with authentic GSH (line labeled "GSH").
FIG. 3 is a bar graph illustrating % mitochondrial GSH replenishment for two different concentrations of TPP-GSH (0.1 µm and 10 µm).
FIG. 4 is a graph of GSH as a function of 3-hydroxy-4-pentenoate ("3HP") (µM), which illustrates the impact of 3HP on mitochondrial (represented with "■") and cytosolic (represented with "●") GSH.
FIG. 5 is a bar graph illustrating the impact of 3HP on mitochondrial GSH levels in young rat liver versus mitochondrial GSH in young and old rat liver.
FIG. 6 is a graph of oxygen consumption rate ("OCR") as a function of time (minutes) illustrating that 3HP-dependent GSH loss does not alter (1) basal respiration, (2) ATP turnover, (3) proton leak, or (4) non-mitochondrial respiration; however, respiratory capacity is increasingly lost with higher levels of 3HP treatment indicating that through the loss of mitochondrial GSH, cells can no longer respond to increasing energy demands.
FIG. 7 is a graph of percent viability as a function of menadione (µM), which illustrates that hepatocytes from old rats are nearly twice as susceptible to menadione as cells from young animals (LC₅₀ = 232 µM in old versus 401 µM in young; n=6, p<0.05); this higher vulnerability is coincident with an age-related loss of mitochondrial GSH (as illustrated by the insert in FIG. 7, labeled as "A").
FIG. 8 illustrates that pretreating cells with 50 µM TPP-GSH 30 minutes prior to menadione protects against hepatotoxicity; the LC₅₀ after TPP-GSH treatment is no different than *untreated* cells from young rats (n=1).

### DETAILED DESCRIPTION

### I. Explanation of Terms

The following explanations of terms are provided to better describe the present disclosure and to guide those of ordinary skill in the art in the practice of the present disclosure. As used herein, "comprising" means "including" and the singular forms "a" or "an" or "the" include plural references unless the context clearly dictates otherwise. The term "or" refers to a single element of stated alternative elements or a combination of two or more elements, unless the context clearly indicates otherwise.

Although the steps of some of the disclosed methods are described in a particular, sequential order for convenient presentation, it should be understood that this manner of description encompasses rearrangement, unless a particular ordering is required by specific language set forth below. For example, steps described sequentially may in some cases be rearranged or performed concurrently. Additionally, the description sometimes uses terms like "produce" and "provide" to describe the disclosed methods. These terms are high-level abstractions of the actual steps that are performed. The actual steps that correspond to these terms will vary depending on the particular implementation and are readily discernible by one of ordinary skill in the art.

Unless explained otherwise, all technical and scientific terms used herein have the same meaning as commonly understood to one of ordinary skill in the art to which this disclosure belongs. Although methods and compounds similar or equivalent to those described herein can be used in the practice or testing of the present disclosure, suitable methods and compounds are described below. The compounds, methods, and examples are illustrative only and not intended to be limiting, unless otherwise indicated. Other features of the disclosure are apparent from the following detailed description and the claims.

Unless otherwise indicated, all numbers expressing quantities of components, molecular weights, percentages, temperatures, times, and so forth, as used in the specification or claims are to be understood as being modified by the term "about." Accordingly, unless otherwise indicated, implicitly or explicitly, the numerical parameters set forth are approximations that can depend on the desired properties sought and/or limits of detection under standard test conditions/methods. When directly and explicitly distinguishing embodiments from discussed prior art, the embodiment numbers are not approximates unless the word "about" is recited. Furthermore, not all alternatives recited herein are equivalents.

Compounds disclosed herein may contain one or more asymmetric elements such as stereogenic centers, stereogenic axes and the like, e.g., asymmetric carbon atoms, so that the chemical conjugates can exist in different stereoisomeric forms. These compounds can be, for example, racemates or optically active forms. For compounds with two or more asymmetric elements, these compounds can additionally be mixtures of diastereomers. For compounds having asymmetric centers, all optical isomers in pure form and mixtures thereof are encompassed. In these situations, the single enantiomers, i.e., optically active forms can be obtained by asymmetric synthesis, synthesis from optically pure precursors, or by resolution of the racemates. Resolution of the racemates can also be accomplished, for example, by conventional methods such as crystallization in the presence of a resolving agent, or chromatography, using, for example a chiral HPLC column. All forms are contemplated herein regardless of the methods used to obtain them.

All forms (for example solvates, optical isomers, enantiomeric forms, polymorphs, free compound and salts) of an active agent may be employed either alone or in combination.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., Stereochemistry of Organic Compounds (1994) John Wiley & Sons, Inc., New York. Many organic compounds exist in optically active forms, *i.e*., they have the ability to rotate the plane of plane-polarized light. In describing an optically active compound, the prefixes D and L or R and S are used to denote the absolute configuration of the molecule about its chiral center(s). The prefixes d and l or (+) and (-) are employed to designate the sign of rotation of plane-polarized light by the compound, with (-) or 1 meaning that the compound is levorotatory. A compound prefixed with (+) or d is dextrorotatory

To facilitate review of the various aspects of the disclosure, the following explanations of specific terms and abbreviations are provided:
**Adjuvant:** An excipient that modifies the effect of other agents, typically the active ingredient. Adjuvants are often pharmacological and/or immunological agents. An adjuvant may modify the effect of an active ingredient by increasing an immune response. An adjuvant may also act as a stabilizing agent for a formulation. Exemplary adjuvants include, but are not limited to, aluminum hydroxide, alum, aluminum phosphate, killed bacteria, squalene, detergents, cytokines, paraffin oil, and combination adjuvants, such as freund's complete adjuvant or freund's incomplete adjuvant.

**Aldehyde:** -C(O)H.

**Aliphatic:** A hydrocarbon, or a radical thereof, having at least one carbon atom to 50 carbon atoms, such as one to 25 carbon atoms, or one to ten carbon atoms, and which includes alkanes (or alkyl), alkenes (or alkenyl), alkynes (or alkynyl), including cyclic versions thereof, and further including straight- and branched-chain arrangements, and all stereo and position isomers as well.

**Aliphatic-aryl:** An aryl group that is or can be coupled to a compound disclosed herein, wherein the aryl group is or becomes coupled through an aliphatic group.

**Aliphatic-heteroaryl:** A heteroaryl group that is or can be coupled to a compound disclosed herein, wherein the heteroaryl group is or becomes coupled through an aliphatic group.

**Alkenyl:** An unsaturated monovalent hydrocarbon having at least two carbon atoms to 50 carbon atoms, such as two to 25 carbon atoms, or two to ten carbon atoms, and at least one carbon-carbon double bond, wherein the unsaturated monovalent hydrocarbon can be derived from removing one hydrogen atom from one carbon atom of a parent alkene. An alkenyl group can be branched, straight-chain, cyclic (*e.g.,* cycloalkenyl), *cis,* or *trans* (*e.g., E* or *Z*).

**Alkoxy:** -O-alkyl, with exemplary instances including, but not limited to, methoxy, ethoxy, n-propoxy, isopropoxy, *n*-butoxy, *t*-butoxy, *sec*-butoxy, *n*-pentoxy.

**Alkyl:** A saturated monovalent hydrocarbon having at least one carbon atom to 50 carbon atoms, such as one to 25 carbon atoms, or one to ten carbon atoms, wherein the saturated monovalent hydrocarbon can be derived from removing one hydrogen atom from one carbon atom of a parent compound (*e.g.,* alkane). An alkyl group can be branched, straight-chain, or cyclic (*e.g.,* cycloalkyl).

**Alkynyl:** An unsaturated monovalent hydrocarbon having at least two carbon atoms to 50 carbon atoms, such as two to 25 carbon atoms, or two to ten carbon atoms and at least one carbon-carbon triple bond, wherein the unsaturated monovalent hydrocarbon can be derived from removing one hydrogen atom from one carbon atom of a parent alkyne. An alkynyl group can be branched, straight-chain, or cyclic (*e.g.,* cycloalkynyl).

**Alkylaryl/Alkenylaryl/Alkynylaryl:** An aryl group that is or can be coupled to a compound disclosed herein, wherein the aryl group is or becomes coupled through an alkyl, alkenyl, or alkynyl group, respectively.

**Alkylheteroaryl/Alkenylheteroaryl/Alkynylheteroaryl:** A heteroaryl group that is or can be coupled to a compound disclosed herein, wherein the heteroaryl group is or becomes coupled through an alkyl, alkenyl, or alkynyl group, respectively.

**Amide:** -C(O)NR^{a}R^{b} wherein each of R^{a} and R^{b} independently is selected from hydrogen, aliphatic, heteroaliphatic, aryl, heteroaryl, or any combination thereof.

**Amine:** -NR^{a}R^{b}, wherein each of R^{a} and R^{b} independently is selected from hydrogen, alkyl, alkenyl, alkynyl, aryl, heteroalkyl, heteroalkenyl, heteroalkynyl, heteroaryl, and any combination thereof.

**Aryl:** An aromatic carbocyclic group comprising at least five carbon atoms to 15 carbon atoms, such as five to ten carbon atoms, having a single ring or multiple condensed rings, which condensed rings can or may not be aromatic provided that the point of attachment is through an atom of the aromatic carbocyclic group. Aryl groups may be substituted with one or more groups other than hydrogen, such as aliphatic, heteroaliphatic, aryl, heteroaryl, other functional groups, or any combination thereof.

**Carboxyl:** -C(O)OR^{a}, wherein R^{a} is alkyl, alkenyl, alkynyl, aryl, heteroalkyl, heteroalkenyl, heteroalkynyl, heteroaryl, hydrogen, and any combination thereof.

**Carrier:** An excipient that serves as a component capable of delivering a compound described herein. In some instances, a carrier can be a suspension aid, solubilizing aid, or aerosolization aid. In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. In some examples, the pharmaceutically acceptable carrier may be sterile to be suitable for administration to a subject (for example, by parenteral, intramuscular, or subcutaneous injection). In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Haloaliphatic:** An aliphatic group wherein one or more hydrogen atoms, such as one to 10 hydrogen atoms, independently is replaced with a halogen atom, such as fluoro, bromo, chloro, or iodo.

**Haloaliphatic-aryl:** An aryl group that is or can be coupled to a compound disclosed herein, wherein the aryl group is or becomes coupled through a haloaliphatic group.

**Haloaliphatic-heteroaryl:** A heteroaryl group that is or can be coupled to a compound disclosed herein, wherein the heteroaryl group is or becomes coupled through a haloaliphatic group.

**Haloalkyl/Haloalkenyl/Haloalkynyl:** An alkyl, alkenyl, or alkynyl group (which can be branched, straight-chain, or cyclic) comprising at least one halogen atom to 20 halogen atoms, such as one to 15 halogen atoms, or one to 5 halogen atoms, which can be selected from, but not limited to bromine, chlorine, fluorine, or iodine.

**Heteroaliphatic:** An aliphatic group comprising at least one heteroatom to 20 heteroatoms, such as one to 15 heteroatoms, or one to 5 heteroatoms, which can be selected from, but not limited to oxygen, nitrogen, sulfur, selenium, phosphorous, and oxidized forms thereof within the group.

**Heteroaliphatic-aryl:** An aryl group that is or can be coupled to a compound disclosed herein, wherein the aryl group is or becomes coupled through a heteroaliphatic group.

**Heteroalkyl/Heteroalkenyl/Heteroalkynyl:** An alkyl, alkenyl, or alkynyl group (which can be branched, straight-chain, or cyclic) comprising at least one heteroatom to 20 heteroatoms, such as one to 15 heteroatoms, or one to 5 heteroatoms, which can be selected from, but not limited to oxygen, nitrogen, sulfur, selenium, phosphorous, and oxidized forms thereof within the group.

**Heteroalkyl-aryl/Heteroalkenyl-aryl/Heteroalkynyl-aryl:** An aryl group that is or can be coupled to a compound disclosed herein, wherein the aryl group is or becomes coupled through a heteroalkyl, heteroalkenyl, or heteroalkynyl group, respectively.

**Heteroalkyl-heteroaryl/Heteroalkenyl-heteroaryl/Heteroalkynyl-heteroaryl:** A heteroaryl group that is or can be coupled to a compound disclosed herein, wherein the aryl group is or becomes coupled through a heteroalkyl, heteroalkenyl, or heteroalkynyl group, respectively.

**Heteroaryl:** An aryl group comprising at least one heteroatom to six heteroatoms, such as one to four heteroatoms, which can be selected from, but not limited to oxygen, nitrogen, sulfur, selenium, phosphorous, and oxidized forms thereof within the ring. Such heteroaryl groups can have a single ring or multiple condensed rings, wherein the condensed rings may or may not be aromatic and/or contain a heteroatom, provided that the point of attachment is through an atom of the aromatic heteroaryl group. Heteroaryl groups may be substituted with one or more groups other than hydrogen, such as aliphatic, heteroaliphatic, aryl, heteroaryl, other functional groups, or any combination thereof.

**Imidoester:** -C(NH₂⁺)OR^{a}, wherein R^{a} is selected from aliphatic, heteroaliphatic, aryl, heteroaryl, and any combination thereof.

**Ketone:** -C(O)R^{a}, wherein R^{a} is selected from aliphatic, heteroaliphatic, aryl, heteroaryl, and any combination thereof.

**Pharmaceutically Acceptable Excipient:** A substance, other than an active ingredient (e.g., a compound described herein), that is included in a formulation of the active ingredient. As used herein, an excipient may be incorporated within particles of a pharmaceutical composition, or it may be physically mixed with particles of a pharmaceutical composition. An excipient can be used, for example, to dilute an active agent and/or to modify properties of a pharmaceutical composition. Excipients can include, but are not limited to, antiadherents, binders, coatings, enteric coatings, disintegrants, flavorings, sweeteners, colorants, lubricants, glidants, sorbents, preservatives, adjuvants, carriers or vehicles. Excipients may be starches and modified starches, cellulose and cellulose derivatives, saccharides and their derivatives such as disaccharides, polysaccharides and sugar alcohols, protein, synthetic polymers, crosslinked polymers, antioxidants, amino acids or preservatives. Exemplary excipients include, but are not limited to, magnesium stearate, stearic acid, vegetable stearin, sucrose, lactose, starches, hydroxypropyl cellulose, hydoxypropyl methylcellulose, xylitol, sorbitol, maltitol, gelatin, polyvinylpyrrolidone (PVP), polyethyleneglycol (PEG), tocopheryl polyethylene glycol 1000 succinate (also known as vitamin E TPGS, or TPGS), carboxy methyl cellulose, dipalmitoyl phosphatidyl choline (DPPC), vitamin A, vitamin E, vitamin C, retinyl palmitate, selenium, cysteine, methionine, citric acid, sodium citrate, methyl paraben, propyl paraben, sugar, silica, talc, magnesium carbonate, sodium starch glycolate, tartrazine, aspartame, benzalkonium chloride, sesame oil, propyl gallate, sodium metabisulphite or lanolin.

**Pharmaceutically Acceptable Salt:** Pharmaceutically acceptable salts of a compound described herein that are derived from a variety of organic and inorganic counter ions as will be known to a person of ordinary skill in the art and include, by way of example only, sodium, potassium, calcium, magnesium, ammonium, tetraalkylammonium, and the like; and when the molecule contains a basic functionality, salts of organic or inorganic acids, such as hydrochloride, hydrobromide, tartrate, mesylate, acetate, maleate, oxalate, and the like. "Pharmaceutically acceptable acid addition salts" are a subset of "pharmaceutically acceptable salts" that retain the biological effectiveness of the free bases while formed by acid partners. In particular, the disclosed compounds form salts with a variety of pharmaceutically acceptable acids, including, without limitation, inorganic acids such as hydrochloric acid, hydrobromic acid, sulfuric acid, nitric acid, phosphoric acid, and the like, as well as organic acids such as formic acid, acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, benzene sulfonic acid, isethionic acid, methane sulfonic acid, ethanesulfonic acid, *p*-toluenesulfonic acid, salicylic acid, xinafoic acid and the like. "Pharmaceutically acceptable base addition salts" are a subset of "pharmaceutically acceptable salts" that are derived from inorganic bases such as sodium, potassium, lithium, ammonium, calcium, magnesium, iron, zinc, copper, manganese, aluminum salts and the like. Exemplary salts are the ammonium, potassium, sodium, calcium, and magnesium salts. Salts derived from pharmaceutically acceptable organic bases include, but are not limited to, salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, dicyclohexylamine, lysine, arginine, histidine, caffeine, procaine, hydrabamine, choline, betaine, ethylenediamine, glucosamine, methylglucamine, theobromine, purines, piperazine, piperidine, *N*-ethylpiperidine, polyamine resins, and the like. Exemplary organic bases are isopropylamine, diethylamine, ethanolamine, trimethylamine, dicyclohexylamine, choline, and caffeine. (See, for example, S. M. Berge, et al., "Pharmaceutical Salts," J. Pharm. Sci., 1977; 66:1-19.)

**Pharmaceutically/Therapeutically Effective Amount:** An amount of a compound sufficient to treat a specified disorder or disease, or to ameliorate or eradicate one or more of its symptoms and/or to prevent the occurrence of the disease or disorder. The amount of a compound which constitutes a "therapeutically effective amount" will vary depending on the compound, the disease state and its severity, the age of the patient to be treated, and the like. The therapeutically effective amount can be determined by a person of ordinary skill in the art.

**Prodrug:** Compounds disclosed herein that are transformed *in vivo* to yield a biologically active compound, particularly the parent compound, for example, by hydrolysis in the gut or enzymatic conversion. Common examples of prodrug moieties include, but are not limited to, pharmaceutically acceptable ester and amide forms of a compound having an active form bearing a carboxylic acid moiety. Examples of pharmaceutically acceptable esters of the compounds of the present disclosure include, but are not limited to, esters of phosphate groups and carboxylic acids, such as aliphatic esters, particularly alkyl esters (for example C₁₋₆alkyl esters). Other prodrug moieties include phosphate esters, such as -CH₂-O-P(O)(OR^{d})₂ or a salt thereof, wherein R^{d} is H or C₁₋₆alkyl. Acceptable esters also include cycloalkyl esters and arylalkyl esters such as, but not limited to benzyl. Examples of pharmaceutically acceptable amides of the compounds of this invention include, but are not limited to, primary amides, and secondary and tertiary alkyl amides (for example with between about one and about six carbons). Amides and esters of disclosed exemplary compounds according to the present disclosure can be prepared according to conventional methods. A thorough discussion of prodrugs is provided in T. Higuchi and V. Stella, "Pro-drugs as Novel Delivery Systems," Vol 14 of the A.C.S. Symposium Series, and in Bioreversible Carriers in Drug Design, ed. Edward B. Roche, American Pharmaceutical Association and Pergamon Press, 1987.

**Subject:** Mammals and other animals, such as humans, companion animals (e.g., dogs, cats, rabbits, etc), utility animals, and feed animals; thus, disclosed methods are applicable to both human therapy and veterinary applications.

**Treating/Treatment:** Treatment of a disease or condition of interest in a subject, particularly a human having the disease or condition of interest, and includes by way of example, and without limitation:
(i) preventing the disease or condition from occurring in a subject, in particular, when such subject is predisposed to the condition but has not yet been diagnosed as having it;
(ii) inhibiting the disease or condition, for example, arresting or slowing its development;
(iii) relieving the disease or condition, for example, causing regression of the disease or condition or a symptom thereof; or
(iv) stabilizing the disease or condition.

As used herein, the terms "disease" and "condition" can be used interchangeably or can be different in that the particular malady or condition may not have a known causative agent (so that etiology has not yet been determined) and it is therefore not yet recognized as a disease but only as an undesirable condition or syndrome, where a more or less specific set of symptoms have been identified by clinicians.

### II. Compounds

Disclosed herein are embodiments of compounds that can be used to deliver glutathione to a target and/or compounds capable of delivering glutathione precursors to a target region. In particular disclosed embodiments, the compounds are glutathione-based compounds. In additional embodiments, the compounds are glutathione-based fragment compounds that can be assembled to form glutathione *in vitro* or *in vivo.* The compounds disclosed herein can be used to increase or replenish glutathione levels in a subject. In particular disclosed embodiments, the compounds disclosed herein are capable of passing through membranes and thus can be used to increase glutathione levels within different biological structures in a cell, subject, or a sample. The compounds can pass through membranes of mitochondria, the endoplasmic reticulum, nuclei, and other biological structures comprising a membrane. In some embodiments, the compounds can associate with DNA and/or RNA. Because the disclosed compounds are capable of permeating or passing through membranes, they can be used to increase and/or maintain glutathione (or glutathione precursor) levels in a particular biological structure. By maintaining and/or increasing glutathione (or glutathione precursor) levels in a subject, the disclosed compounds can be used to treat myriad diseases and/or conditions, which are discussed in more detail herein. In particular disclosed embodiments, the compounds disclosed herein can be used to maintain and/or increase glutathione levels (or glutathione precursor) in the mitochondria or other cellular compartments by specifically and selectively delivering glutathione and/or glutathione precursors to the mitochondria and other cellular compartments.

To assist with providing context to the present invention, compounds that have structures satisfying Formula 1A or 1B below, or structures corresponding to pharmaceutically acceptable salts, zwitterions, or prodrugs thereof, are now described. Where the compound is a pharmaceutically acceptable salt of the formulas illustrated below, the compound includes one or more charged groups derived from removal of at least one of the R groups illustrated below in Formulas 1A and 1B. For example, one or more R groups attached to an oxygen of an illustrated carboxyl group can be removed to provide a negatively charged carboxylate. In yet additional aspects, one or more of the illustrated amine groups (e.g., -NR- or -N(R)₂ groups) can be quaternized to provide a positively charged amine group. In such aspects, the amine can be quaternized by protonation or alkylation of the amine group. In aspects where the compound is a pharmaceutically acceptable salt, one or more counter ions can be present, such as the organic and/or inorganic salts described above for pharmaceutically acceptable salts. In some aspects, the compound can be a zwitterion and therefore comprise a positively charged group and a negatively charged group (e.g., a quaternized nitrogen atom and a carboxylate).

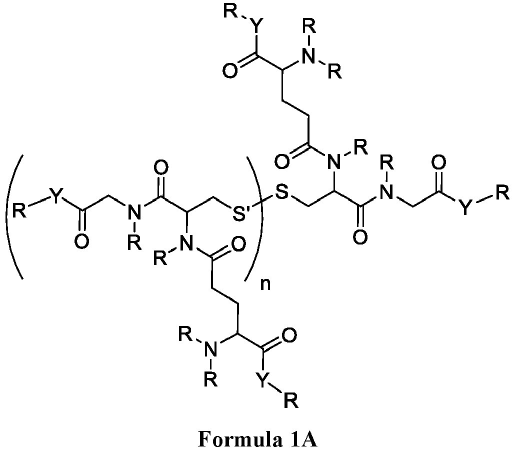

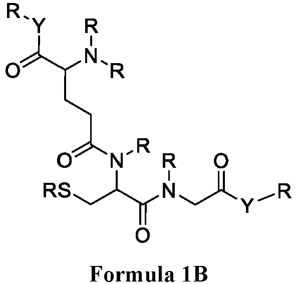

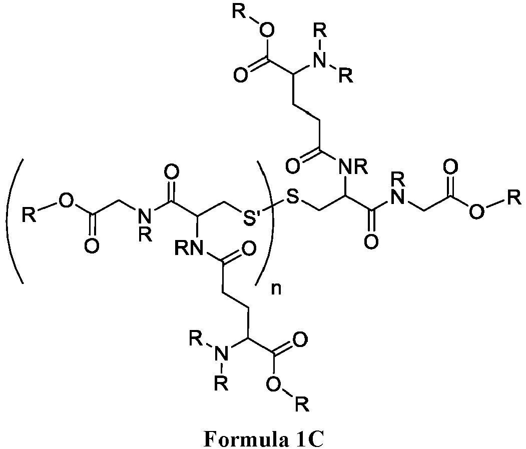

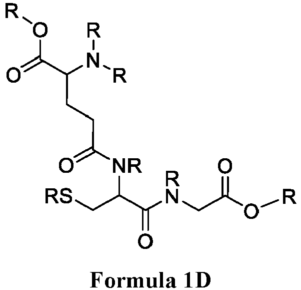

With reference to Formulas 1A-1D, each R independently can be selected from hydrogen, a protecting group, or a linker-X moiety. Suitable protecting groups can be selected from a functional group capable of being bound to and removed (e.g., by displacement of the functional group or from hydrolysis or other cleavage of the group) from an amine group, a thiol group, or a carboxylic acid. In some aspects, protecting groups can be selected from aliphatic groups, aryl groups, heteroaliphatic groups, aliphatic-aryl groups, heteroaryl groups, aliphatic-heteroaryl groups, heteroaliphatic-aryl groups, or heteroaliphatic-heteroaryl groups. With respect to the linker-X moiety, this moiety includes functional groups suitable to facilitate delivery of the compound to a target. In some aspects, the linker can be selected from a carbonyl-containing group, an alkylene oxide, an aliphatic group, an imidoester; or the linker can be generated from a maleimide, a haloacetyl, or a pyridyl disulfide; X can be a targeting moiety capable of promoting or facilitating permeation of the compound through a membrane. With continued reference to Formulas 1A and 1C, n can be 0 or 1. With reference to Formulas 1A and 1B, Y is selected from oxygen or -NR', wherein R' is hydrogen or a protecting group. In particular disclosed aspects, Y is oxygen or -NH.

In some aspects, the targeting moiety can be a moiety capable of increasing delivery of a compound into the cytosol, such as increasing delivery of the compound by a factor of 10 (as compared to a similar compound that does not comprise a targeting moiety) and/or into mitochondria by a factor of 100 (as compared to a similar compound that does not comprise a targeting moiety). In some aspects, if n is 0, then the disulfide moiety formed between S and S' in Formulas 1A and 1C is not included and instead the S atom is bound to an R group selected from hydrogen; a protecting group selected from an aliphatic group, aryl group, heteroaliphatic group, aliphatic-aryl group, heteroaryl group, aliphatic-heteroaryl group, heteroaliphatic-aryl group, or heteroaliphatic-heteroaryl group; a nitroso group; or a linker-X group as described herein (Formulas 1B and 1D).

Compounds satisfying the formulas above can further comprise a counter ion in aspects where X comprises a charged group, thereby providing electronic neutrality. For example, in some aspects, each X independently can be selected from a phosphonium group, an ammonium group, or other such group comprising a positively charged moiety. In particular disclosed aspects, the counter ion used in combination with a hydrophobic, positively charged moiety can be a negatively charged counter ion and typically is a pharmaceutically-acceptable, negatively charged counter ion. Exemplary negatively charged counter ions include, but are not limited to, halogens (e.g., Br⁻, Cl⁻, F⁻, I⁻), sulfonates (e.g., mesylate), sulfates, hydrobromide, acetate, citrate, maleate, tartrate, phosphate, nitrate, salicylate, fumerate, lactate, or the like. In an independent aspect, X is a targeting moiety that is not or is other than a nanoparticle, such as a gold nanoparticle.

In some aspects, each R independently can be selected from hydrogen, alkyl, alkenyl, or alkynyl; heteroalkyl, heteroalkenyl, or heteroalkynyl; heteroalkylaryl, heteroalkenylaryl, or heteroalkynylaryl; heteroalkylheteroaryl, heteroalkenylheteroaryl, or heteroalkynylheteroaryl; alkylheteroaryl, alkenylheteroaryl, or alkynylheteroaryl; a linker-X group selected from -C(O)R^{c}X, -[(CH₂)₂O]ₘX, -C(=NH₂⁺)NR^{c}X, -CH₂C(O)NHR^{c}X, -SR^{c}X, 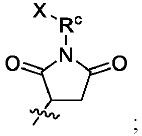 wherein each R^{c} independently can be selected from aliphatic, aryl, heteroaliphatic, aliphatic-aryl, heteroaryl, aliphatic-heteroaryl, heteroaliphatic-aryl, or heteroaliphatic-heteroaryl; each X independently can be selected from -P⁺(R^{d})₃, -N⁺(R^{d})₃, wherein each R^{d} independently can be selected from hydrogen, aliphatic, aryl, or aliphatic-aryl; and each of m independently can be 1 to 30, such as 1 to 20, or 1 to 10, or 1 to 5. In an independent instance, if the linker is an aliphatic linker group and X is a trimethyl-substituted amine (i.e., X = -N⁺(Me)₃), then the aliphatic linker group comprises more than two methylene units, such as 3 to 30 methylene units, or 5 to 30 methylene units.

In particular disclosed aspects, each R shown in the above Formulas 1A-1D independently can be alkyl selected from methyl, ethyl, propyl, butyl, pentyl, hexyl, septyl, octyl, nonyl, decyl; heteroaliphatic selected from alkyl amine (e.g., -ethylamine, -propylamine, -butylamine, or the like); aryl selected from phenyl or naphthyl; or aliphatic-aryl selected from -(CH₂)ₘPh, wherein m is 1 to 20; a linker-X group selected from -C(O)(CH₂)₁₋₃₀(P)⁺Ph₃Br⁻, -C(=NH₂⁺)N(CH₂)₁₋₃₀(P)⁺Ph₃Br-, -CH₂C(O)NH(CH₂)₁₋₃₀(P)⁺Ph₃Br⁻, -S(CH₂)₁₋₃₀(P)⁺Ph₃Br⁻, -(CH₂)₁₋₃₀(P)⁺Ph₃Br⁻, -C(O)[O(CH₂)₂]₁₋₃₀(P)⁺Ph₃Br⁻, -C(=NH₂⁺)NCH₂[O(CH₂)₂]₁₋₃₀(P)⁺Ph₃Br⁻, -CH₂C(O)NH[O(CH₂)₂]₁₋₃₀(P)⁺Ph₃Br⁻, -S[O(CH₂)₂]₁₋₃₀(P)⁺Ph₃Br, -C(O)(CH₂)₁₋₃₀(N)⁺Me₃Br⁻, -C(=NH₂⁺)N(CH₂)₁₋₃₀(N)⁺Me₃Br⁻, -CH₂C(O)NH(CH₂)₁₋₃₀(N)⁺Me₃Br⁻, -S(CH₂)₁₋₃₀(N)⁺Me₃Br⁻, -(CH₂)₃₋₃₀(N)⁺Me₃Br⁻, -C(O)[O(CH₂)₂]₁₋₃₀(N)⁺Me₃Br⁻, -C(=NH₂⁺)NCH₂[O(CH₂)₂]₁₋₃₀(N)⁺Me₃Br-, -CH₂C(O)NH[O(CH₂)₂]₁₋₃₀(N)⁺Me₃Br⁻, -S[O(CH₂)₂]₁₋₃₀(N)⁺Me₃Br⁻,

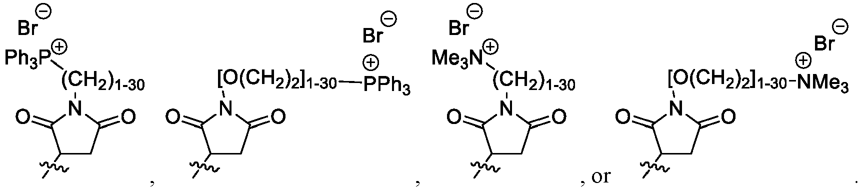

The compounds of the present invention have a structure that satisfies any of Formulas 2A-2D or 5A or 5B.

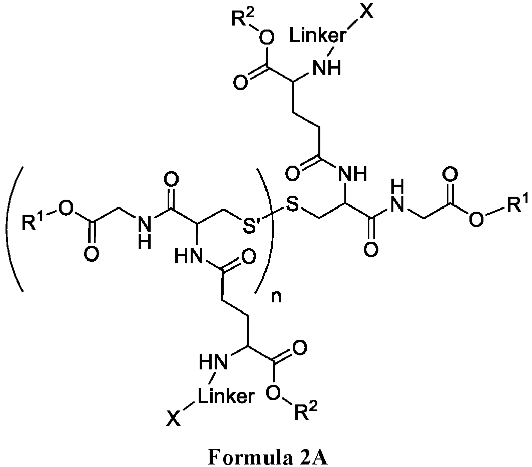

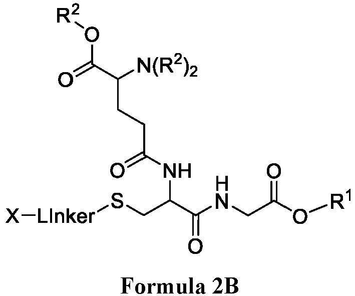

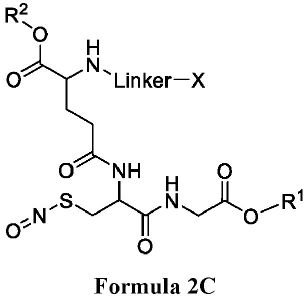

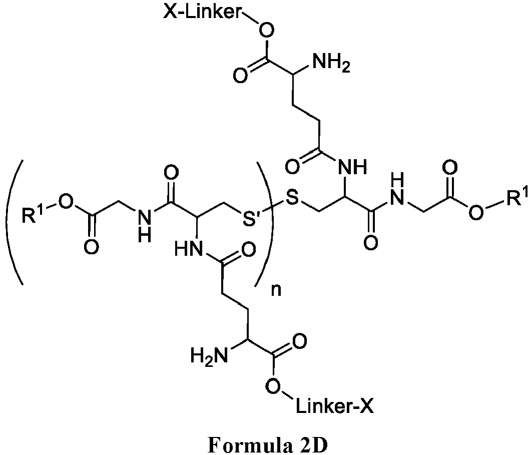

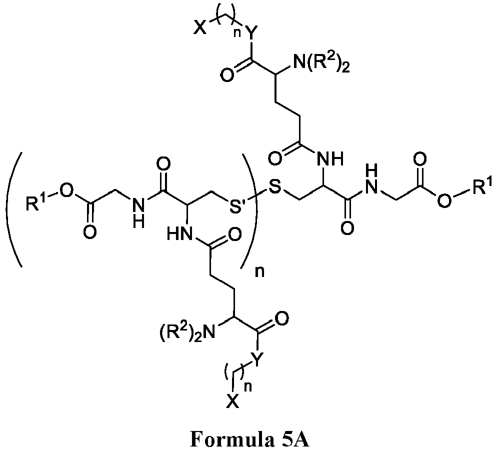

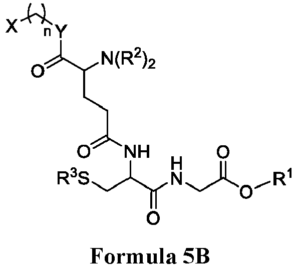

As illustrated in Formulas 2A and 2D, the compounds can be disulfide-containing compounds when n is 1 (thus, both sulfur atoms, S and S' are included). In yet additional embodiments when n is 0, the compounds can be thiol-containing compounds (where the S atom is bound to a hydrogen atom), thio-nitroso groups (e.g., Formula 2C), or thioether-containing compounds or thioester-containing compounds (where the S atom is bound an aliphatic group to provide a thioether-containing compound, or a ketone group to provide a thioester-containing compound).

In some embodiments, the compounds of the present invention can have structures satisfying Formulas 3A, 3B, 4A, or 4B, below. Further compounds, described herein purely to provide context for the present invention, have Formulas 6A or 6B below.

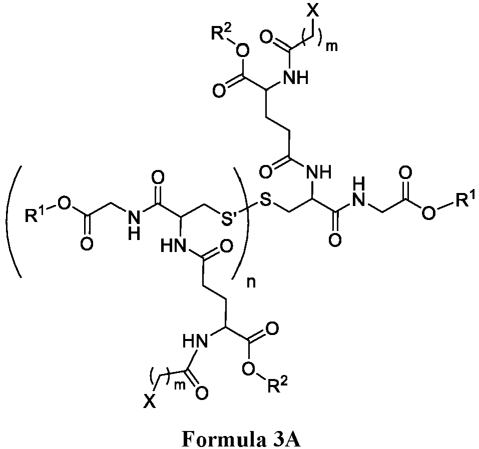

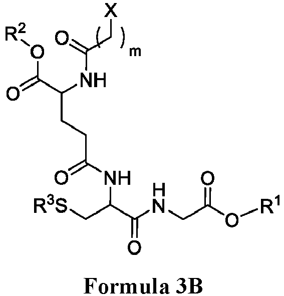

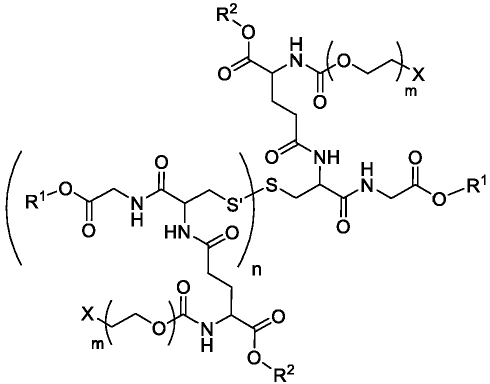

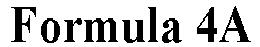

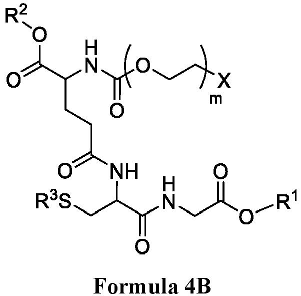

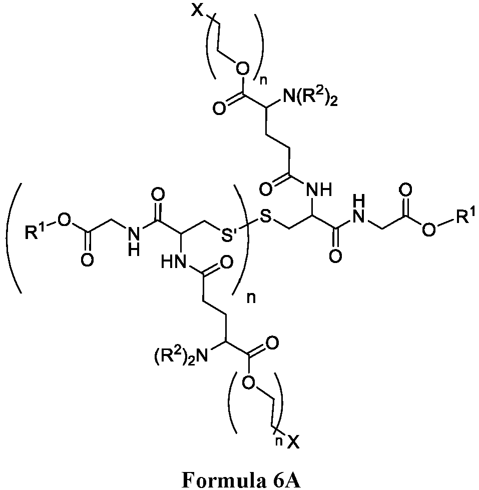

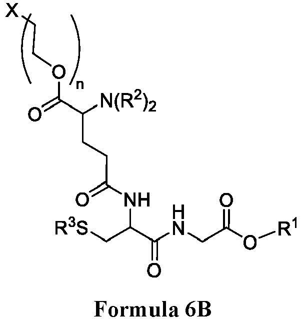

As contemplated by certain formulas above, the compounds can be a dimer compound or a heterodimer compound. In dimeric compounds, the dimer includes two fragments that are joined through the sulfur-sulfur bond illustrated in Formulas 2A, 3A, 4A, 5A, and 6A. Each fragment is the same in dimeric compounds; that is, the two components bound through the disulfide bond are the same. In heterodimeric compounds, the heterodimer similarly includes two fragments that are joined through the sulfur-sulfur bond illustrated in Formulas 2A, 3A, 4A, 5A, and 6A; however, the two fragments are not identical.

Representative compounds satisfying one or more of the formulas above are illustrated and listed below in Table 1.

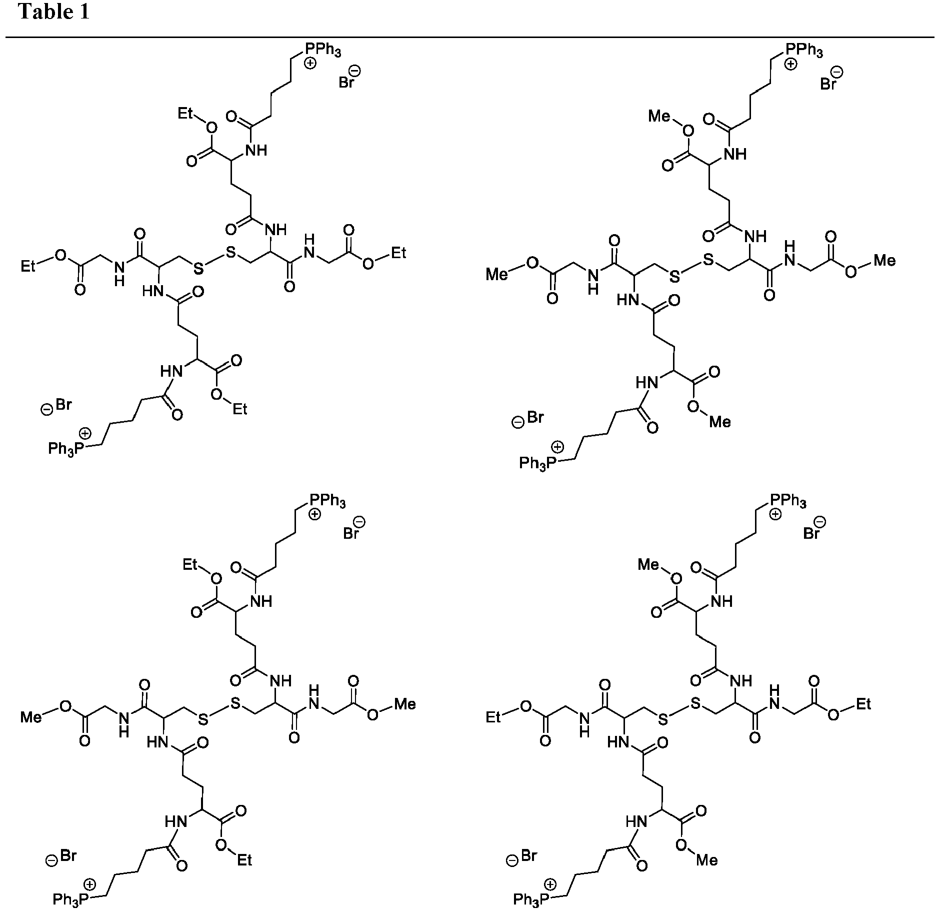

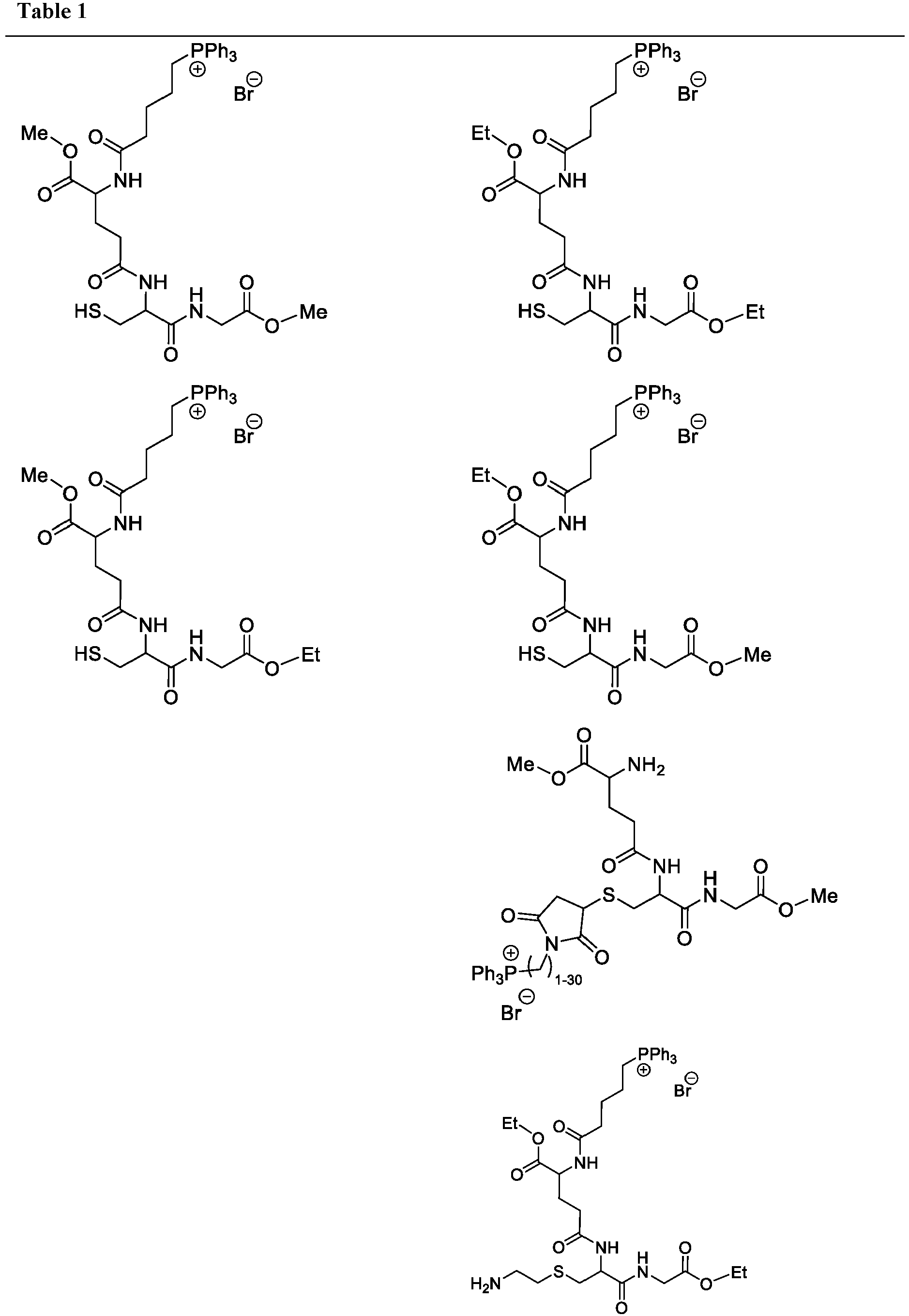

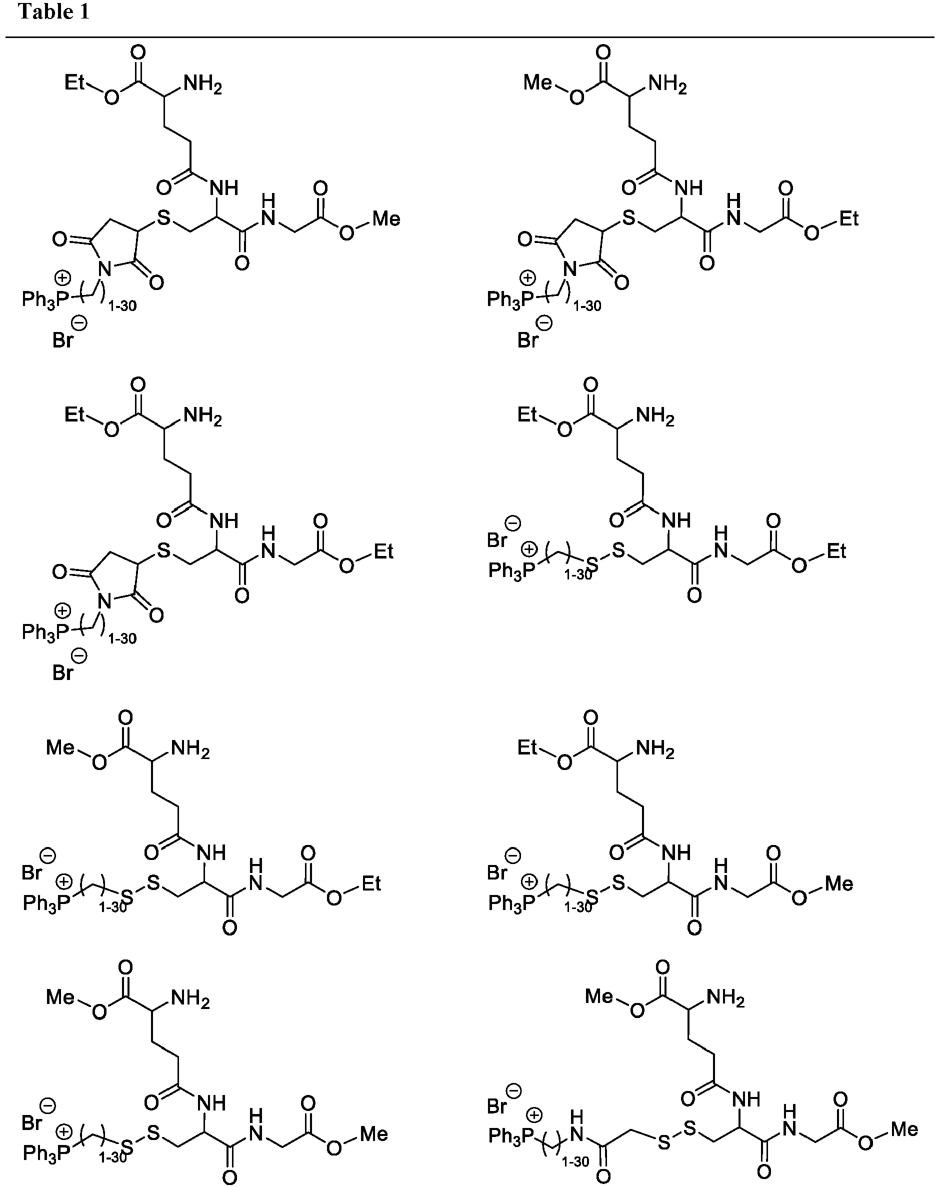

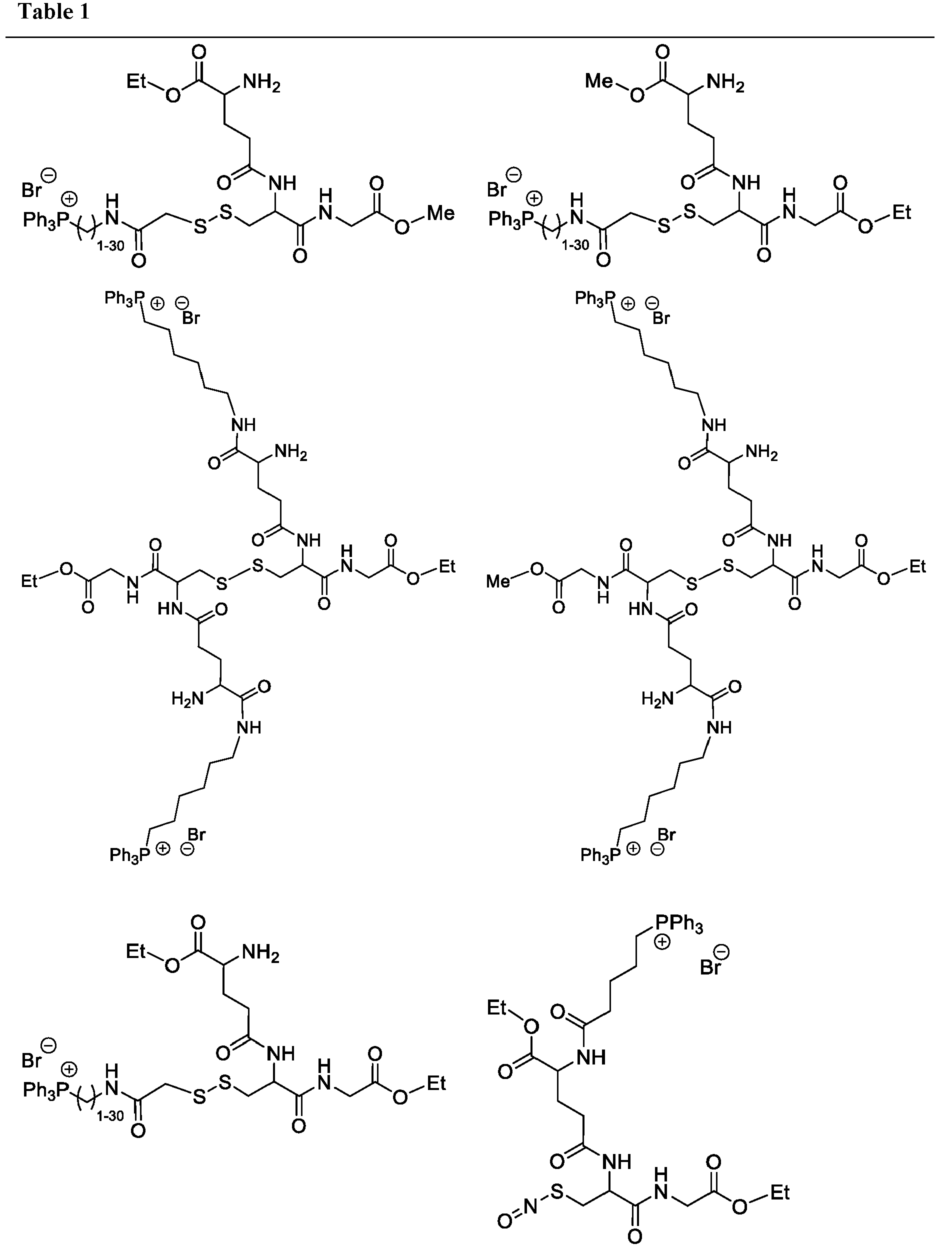

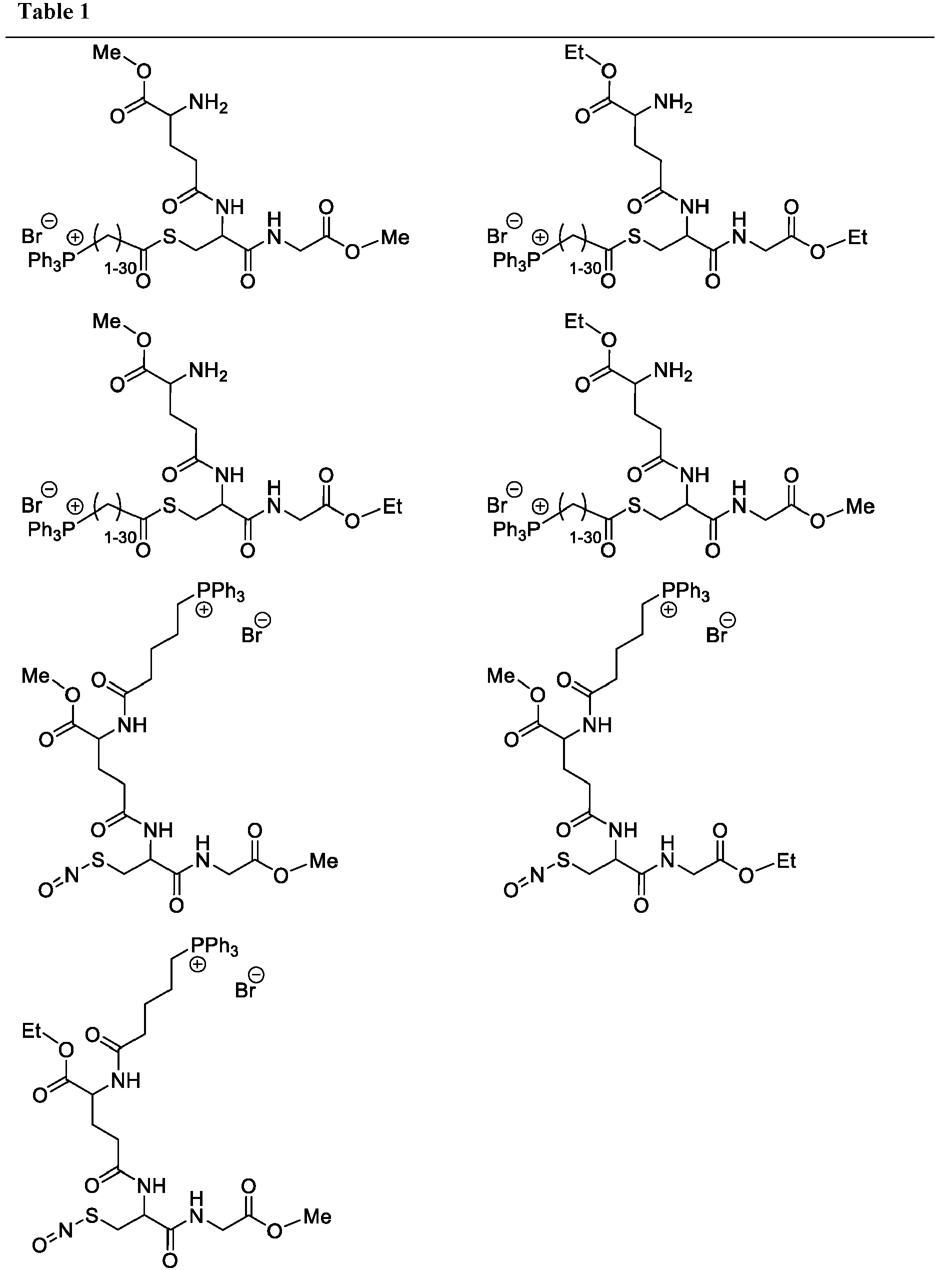

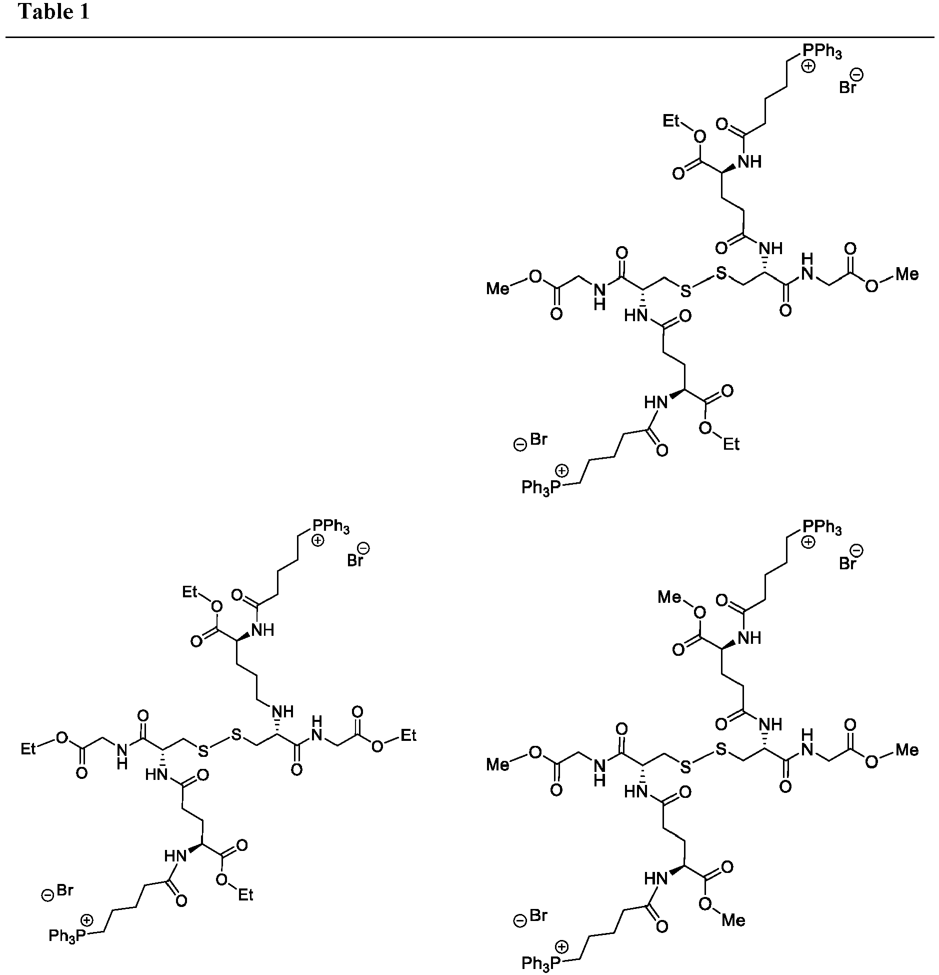

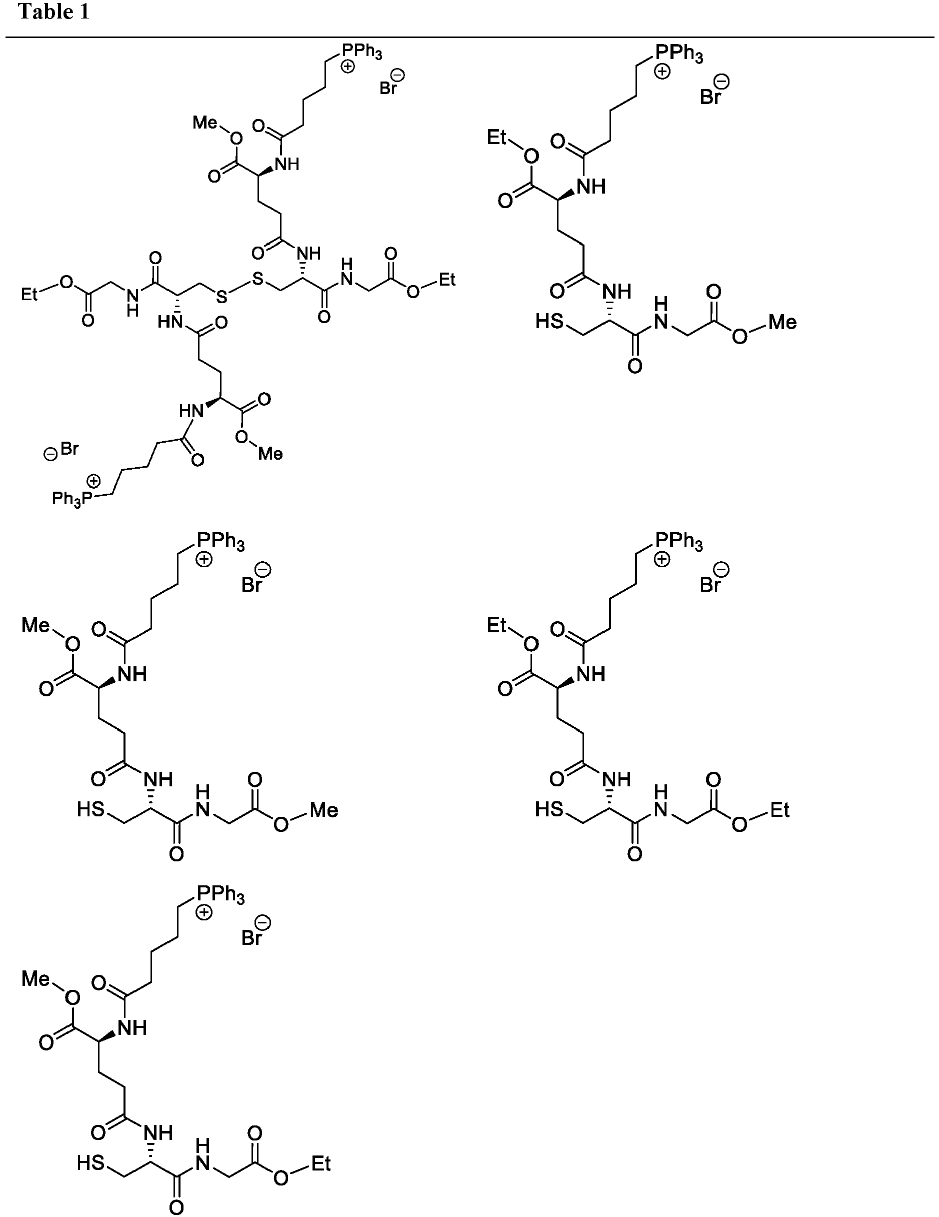

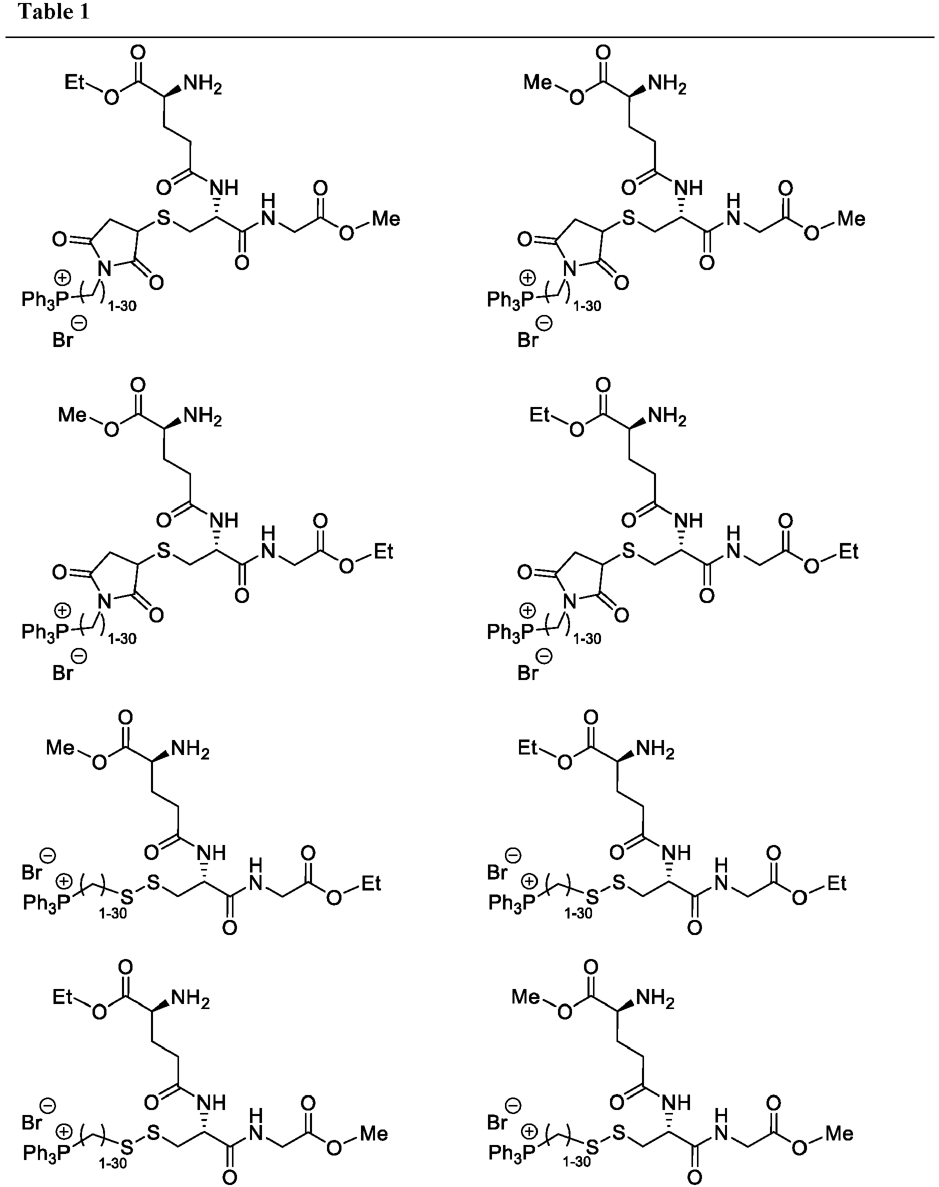

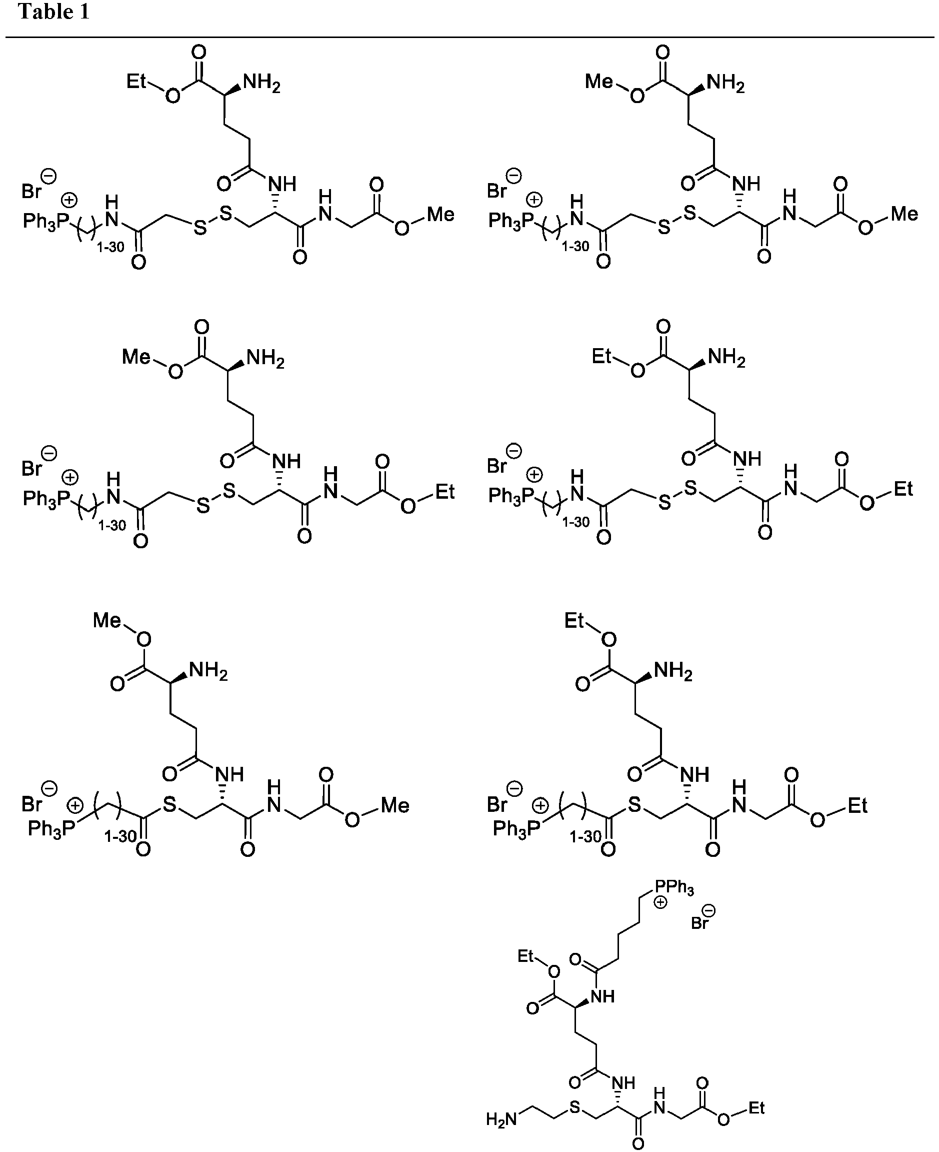

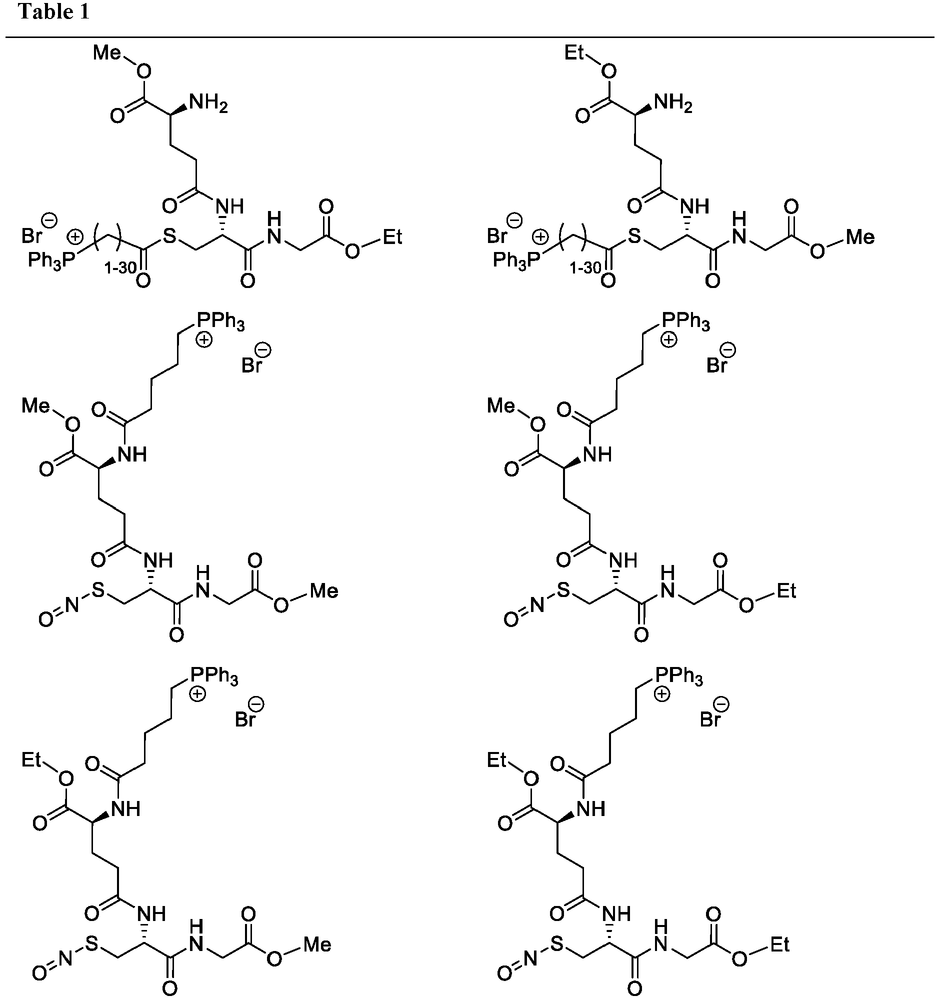

Examples of specific compounds of the disclosure are those selected from the compounds listed below in Table 2 (any compounds outside the scope of the claims are recited for reference only).

**Table 2**

| |
|---|
| ((disulfanediylbis(methylene))bis(13-(ethoxycarbonyl)-4,7,10,15-tetraoxo-3-oxa-6,9,14-triazanonadecane-8,19-diyl))bis(triphenylphosphonium) bromide; |
| ((8*R*,8'*R*,13*S,*13'*S*)-(disulfanediylbis(methylene))bis(13-(ethoxycarbonyl)-4,7,10,15-tetraoxo-3-oxa-6,9,14-triazanonadecane-8,19-diyl))bis(triphenylphosphonium) bromide; |
| ((disulfanediylbis(methylene))bis(12-(methoxycarbonyl)-3,6,9,14-tetraoxo-2-oxa-5,8,13-triazaoctadecane-7,18-diyl))bis(triphenylphosphonium) bromide; |
| ((7*R*,7'*R*,12*S*,12'*S*)-(disulfanediylbis(methylene))bis(12-(methoxycarbonyl)-3,6,9,14-tetraoxo-2-oxa-5,8,13-triazaoctadecane-7,18-diyl))bis(triphenylphosphonium) bromide; |
| (7-(mercaptomethyl)-12-(methoxycarbonyl)-3,6,9,14-tetraoxo-2-oxa-5,8,13-triazaoctadecan-18-yl)triphenylphosphoniumbromide; |
| ((7*R*,12*S*)-7-(mercaptomethyl)-12-(methoxycarbonyl)-3,6,9,14-tetraoxo-2-oxa-5,8,13-triazaoctadecan-18-yl)triphenylphosphoniumbromide; |
| (13-(ethoxycarbonyl)-8-(mercaptomethyl)-4,7,10,15-tetraoxo-3-oxa-6,9,14-triazanonadecan-19-yl)triphenylphosphoniumbromide; |
| ((8*R*,13*S*)-13-(ethoxycarbonyl)-8-(mercaptomethyl)-4,7,10,15-tetraoxo-3-oxa-6,9,14-triazanonadecan-19-yl)triphenylphosphoniumbromide; |
| ((4*S*,9*R*,14*R*,19*S*)-4-acetamido-9,14-bis((2-methoxy-2-oxoethyl)carbamoyl)-19-(methoxycarbonyl)-3,7,21-trioxo-2-oxa-11,12-dithia-8,15,20-triazapentacosan-25-yl)triphenylphosphoniumbromide; |
| ((7*S*,12*R*,17*R*,22*S*)-12,17-bis((2-ethoxy-2-oxoethyl)carbamoyl)-7,22-bls(ethoxycarbonyl)-5,10,24-trioxo-14,15-dithia-6,11,18,23-tetraazaoctacosane-1,28-diyl)bis(triphenylphosphonium) bromide; |
| ((5*S*,10*R*,15*R*,20*S*)-5-acetamido-10,15-bis((2-ethoxy-2-oxoethyl)carbamoyl)-20-(ethoxycarbonyl)-4,8,22-trioxo-3-oxa-12,13-dithia-9,16,21-triazahexacosan-26-yl)triphenylphosphoniumbromide; |
| (13-amino-8-(mercaptomethyl)-4,7,10,14-tetraoxo-3,15-dioxa-6,9-diazaheptadecane-1,17-diyl)bis(triphenylphosphonium)bromide; |
| ((8*R*,13*S*)-13-amino-8-(mercaptomethyl)-4,7,10,14-tetraoxo-3,15-dioxa-6,9-diazaheptadecane-1,17-diyl)bis(triphenylphosphonium)bromide; |
| (16-amino-11-(mercaptomethyl)-7,10,13,17-tetraoxo-23-(triphenyl-14-phosphanyl)-3,6,18,21-tetraoxa-9,12-diazatricosyl)bistriphenylphosphoniumbromide; |
| ((19-amino-14-(mercaptomethyl)-10,13,16,20-tetraoxo-29-(triphenyl-14-phosphanyl)-3,6,9,21,24,27-hexaoxa-12,15-diazanonacosyl)bistriphenylphosphoniumbromide; |
| ((3-((2-(4-amino-5-methoxy-5-oxopentanamido)-3-((2-methoxy-2-oxoethyl)amino)-3-oxopropyl)thio)-2,5-dioxopyrrolidin-1-yl)methyl)triphenylphosphoniumbromide; |
| ((3-(((*R*)-2-((*S*)-4-amino-5-methoxy-5-oxopentanamido)-3-((2-methoxy-2-oxoethyl)amino)-3-oxopropyl)thio)-2,5-dioxopyrrolidin-1-yl)methyl)triphenylphosphonium bromide; |
| (2-(3-((2-(4-amino-5-methoxy-5-oxopentanamido)-3-((2-methoxy-2-oxoethyl)amino)-3-oxopropyl)thio)-2,5-dioxopyrrolidin-1-yl)ethyl)triphenylphosphoniumbromide; |
| (2-(3-(((*R*)-2-((*S*)-4-amino-5-methoxy-5-oxopentanamido)-3-((2-methoxy-2-oxoethyl)amino)-3-oxopropyl)thio)-2,5-dioxopyrrolidin-1-yl)ethyl)triphenylphosphonium bromide; |
| (3-(3-((2-(4-amino-5-methoxy-5-oxopentanamido)-3-((2-methoxy-2-oxoethyl)amino)-3-oxopropyl)thio)-2,5-dioxopyrrolidin-1-yl)propyl)triphenylphosphoniumbromide; |
| (3-(3-(((*R*)-2-((*S*)-4-amino-5-methoxy-5-oxopentanamido)-3-((2-methoxy-2-oxoethyl)amino)-3-oxopropyl)thio)-2,5-dioxopyrrolidin-1-yl)propyl)triphenylphosphonium bromide; |
| (4-(3-((2-(4-amino-5-methoxy-5-oxopentanamido)-3-((2-methoxy-2-oxoethyl)amino)-3-oxopropyl)thio)-2,5-dioxopyrrolidin-1-yl)butyl)triphenylphosphoniumbromide; |
| (4-(3-(((*R*)-2-((S)-4-amino-5-methoxy-5-oxopentanamido)-3-((2-methoxy-2-oxoethyl)amino)-3-oxopropyl)thio)-2,5-dioxopyrrolidin-1-yl)butyl)triphenylphosphonium bromide; |
| (5-(3-((2-(4-amino-5-methoxy-5-oxopentanamido)-3-((2-methoxy-2-oxoethyl)amino)-3-oxopropyl)thio)-2,5-dioxopyrrolidin-1-yl)pentyl)triphenylphosphonium bromide; |
| (5-(3-(((*R*)-2-((S)-4-amino-5-methoxy-5-oxopentanamido)-3-((2-methoxy-2-oxoethyl)amino)-3-oxopropyl)thio)-2,5-dioxopyrrolidin-1-yl)pentyl)triphenylphosphonium bromide; |
| (2-(3-((2-(4-amino-5-ethoxy-5-oxopentanamido)-3-((2-ethoxy-2-oxoethyl)amino)-3-oxopropyl)thio)-2,5-dioxopyrrolidin-1-yl)ethyl)triphenylphosphonium bromide; |
| (2-(3-(((*R*)-2-((*S*)-4-amino-5-ethoxy-5-oxopentanamido)-3-((2-ethoxy-2-oxoethyl)amino)-3-oxopropyl)thio)-2,5-dioxopyrrolidin-1-yl)ethyl)triphenylphosphonium bromide; |
| (3-(3-((2-(4-amino-5-ethoxy-5-oxopentanamido)-3-((2-ethoxy-2-oxoethyl)amino)-3-oxopropyl)thio)-2,5-dioxopyrrolidin-1-yl)propyl)triphenylphosphonium bromide; |
| (3-(3-(((*R*)-2-((*S*)-4-amino-5-ethoxy-5-oxopentanamido)-3-((2-ethoxy-2-oxoethyl)amino)-3-oxopropyl)thio)-2,5-dioxopyrrolidin-1-yl)propyl)triphenylphosphonium bromide; |
| (4-(3-((2-(4-amino-5-ethoxy-5-oxopentanamido)-3-((2-ethoxy-2-oxoethyl)amino)-3-oxopropyl)thio)-2,5-dioxopyrrolidin-1-yl)butyl)triphenylphosphonium bromide; |
| (4-(3-(((*R*)-2-((*S*)-4-amino-5-ethoxy-5-oxopentanamido)-3-((2-ethoxy-2-oxoethyl)amino)-3-oxopropyl)thio)-2,5-dioxopyrrolidin-1-yl)butyl)triphenylphosphonium bromide; |
| (5-(3-((2-(4-amino-5-ethoxy-5-oxopentanamido)-3-((2-ethoxy-2-oxoethyl)amino)-3-oxopropyl)thio)-2,5-dioxopyrrolidin-1-yl)pentyl)triphenylphosphonium bromide; |
| (5-(3-(((*R*)-2-((*S*)-4-amino-5-ethoxy-5-oxopentanamido)-3-((2-ethoxy-2-oxoethyl)amino)-3-oxopropyl)thio)-2,5-dioxopyrrolidin-1-yl)pentyl)triphenylphosphonium bromide; |
| ((3-((2-(4-amino-5-ethoxy-5-oxopentanamido)-3-((2-ethoxy-2-oxoethyl)amino)-3-oxopropyl)thio)-2,5-dioxopyrrolidin-1-yl)methyl)triphenylphosphonium bromide; |
| ((3-(((*R*)-2-((*S*)-4-amino-5-ethoxy-5-oxopentanamido)-3-((2-ethoxy-2-oxoethyl)amino)-3-oxopropyl)thio)-2,5-dioxopyrrolidin-1-yl)methyl)triphenylphosphonium bromide; |
| (((2-(4-amino-5-ethoxy-5-oxopentanamido)-3-((2-ethoxy-2-oxoethyl)amino)-3-oxopropyl)disulfanyl)methyl)triphenylphosphonium bromide; |
| ((((*R*)-2-((S)-4-amino-5-ethoxy-5-oxopentanamido)-3-((2-ethoxy-2-oxoethyl)amino)-3-oxopropyl)disulfanyl)methyl)triphenylphosphonium bromide; |
| (2-((2-(4-amino-5-ethoxy-5-oxopentanamido)-3-((2-ethoxy-2-oxoethyl)amino)-3-oxopropyl)disulfanyl)ethyl)triphenylphosphonium bromide; |
| (2-(((*R*)-2-((*S*)-4-amino-5-ethoxy-5-oxopentanamido)-3-((2-ethoxy-2-oxoethyl)amino)-3-oxopropyl)disulfanyl)ethyl)triphenylphosphonium bromide; |
| (3-((2-(4-amino-5-ethoxy-5-oxopentanamido)-3-((2-ethoxy-2-oxoethyl)amino)-3-oxopropyl)disulfanyl)propyl)triphenylphosphonium bromide; |
| (3-(((*R*)-2-((*S*)-4-amino-5-ethoxy-5-oxopentanamido)-3-((2-ethoxy-2-oxoethyl)amino)-3-oxopropyl)disulfanyl)propyl)triphenylphosphonium bromide; |
| (4-((2-(4-amino-5-ethoxy-5-oxopentanamido)-3-((2-ethoxy-2-oxoethyl)amino)-3-oxopropyl)disulfanyl)butyl)triphenylphosphonium bromide; |
| (4-(((*R*)-2-((*S*)-4-amino-5-ethoxy-5-oxopentanamido)-3-((2-ethoxy-2-oxoethyl)amino)-3-oxopropyl)disulfanyl)butyl)triphenylphosphonium bromide; |
| (5-((2-(4-amino-5-ethoxy-5-oxopentanamido)-3-((2-ethoxy-2-oxoethyl)amino)-3-oxopropyl)disulfanyl)pentyl)triphenylphosphonium bromide; |
| (5-(((*R*)-2-((*S*)-4-amino-5-ethoxy-5-oxopentanamido)-3-((2-ethoxy-2-oxoethyl)amino)-3-oxopropyl)disulfanyl)pentyl)triphenylphosphonium bromide; |
| (((2-(4-amino-5-methoxy-5-oxopentanamido)-3-((2-methoxy-2-oxoethyl)amino)-3-oxopropyl)disulfanyl)methyl)triphenylphosphoniumbromide; |
| ((((*R*)-2-((*S*)-4-amino-5-methoxy-5-oxopentanamido)-3-((2-methoxy-2-oxoethyl)amino)-3-oxopropyl)disulfanyl)methyl)triphenylphosphoniumbromide; |
| (2-((2-(4-amino-5-methoxy-5-oxopentanamido)-3-((2-methoxy-2-oxoethyl)amino)-3-oxopropyl)disulfanyl)ethyl)triphenylphosphoniumbromide; |
| (2-(((*R*)-2-((*S*)-4-amino-5-methoxy-5-oxopentanamido)-3-((2-methoxy-2-oxoethyl)amino)-3-oxopropyl)disulfanyl)ethyl)triphenylphosphoniumbromide; |
| (3-((2-(4-amino-5-methoxy-5-oxopentanamido)-3-((2-methoxy-2-oxoethyl)amino)-3-oxopropyl)disulfanyl)propyl)triphenylphosphoniumbromide; |
| (3-(((*R*)-2-((*S*)-4-amino-5-methoxy-5-oxopentanamido)-3-((2-methoxy-2-oxoethyl)amino)-3-oxopropyl)disulfanyl)propyl)triphenylphosphoniumbromide; |
| (4-((2-(4-amino-5-methoxy-5-oxopentanamido)-3-((2-methoxy-2-oxoethyl)amino)-3-oxopropyl)disulfanyl)butyl)triphenylphosphoniumbromide; |
| (4-(((*R*)-2-((S)-4-amino-5-methoxy-5-oxopentanamido)-3-((2-methoxy-2-oxoethyl)amino)-3-oxopropyl)disulfanyl)butyl)triphenylphosphoniumbromide; |
| (5-((2-(4-amino-5-methoxy-5-oxopentanamido)-3-((2-methoxy-2-oxoethyl)amino)-3-oxopropyl)disulfanyl)pentyl)triphenylphosphoniumbromide; |
| (5-(((*R*)-2-((*S*)-4-amino-5-methoxy-5-oxopentanamido)-3-((2-methoxy-2-oxoethyl)amino)-3-oxopropyl)disulfanyl)pentyl)triphenylphosphoniumbromide; |
| (13-amino-8-((2-methoxy-2-oxoethyl)carbamoyl)-3,10,14-trioxo-15-oxa-5,6-dithia-2,9-diazahexadecyl)triphenylphosphoniumbromide; |
| ((8*R*,13*S*)-13-amino-8-«2-methoxy-2-oxoethyl)carbamoyl)-3,10,14-trioxo-15-oxa-5,6-dithia-2,9-diazahexadecyl)triphenylphosphoniumbromide; |
| (4-amino-9-((2-methoxy-2-oxoethyl)carbamoyl)-3,7,14-trioxo-2-oxa-11,12-dithia-8,15-diazaheptadecan-17-yl)triphenylphosphoniumbromide; |
| ((44*S*,9*R*)-4-amino-9-((2-methoxy-2-oxoethyl)carbamoyl)-3,7,14trioxo-2-oxa-11,12-dithia-8,15-diazaheptadecan-17-yl)triphenylphosphoniumbromide; |
| (4-amino-9-((2-methoxy-2-oxoethyl)carbamoyl)-3,7,14-trioxo-2-oxa-11,12-dithia-8,15-diazaoctadecan-18-yl)triphenylphosphoniumbromide; |
| ((4*S*,9*R*)-4-amino-9-((2-methoxy-2-oxoethyl)carbamoyl)-3,7,14trioxo-2-oxa-11,12-dithia-8,15-diazaoctadecan-18-yl)triphenylphosphoniumbromide; |
| (4-amino-9-((2-methoxy-2-oxoethyl)carbamoyl)-3,7,14-trioxo-2-oxa-11,12-dithia-8,15-diazanonadecan-19-yl)triphenylphosphoniumbromide; |
| ((4*S*,9*R*)-4-amino-9-((2-methoxy-2-oxoethyl)carbamoyl)-3,7,14trioxo-2-oxa-11,12-dithia-8,15-diazanonadecan-19-yl)triphenylphosphoniumbromide; |
| (4-amino-9-((2-methoxy-2-oxoethyl)carbamoyl)-3,7,14-trioxo-2-oxa-11,12-dithia-8,15-diazaicosan-20-yl)triphenylphosphoniumbromide; |
| ((4*S*,9*R*)-4-amino-9-((2-methoxy-2-oxoethyl)carbamoyl)-3,7,14trioxo-2-oxa-11,12-dithia-8,15-diazaicosan-20-yl)triphenylphosphoniumbromide; |
| (13-amino-8-((2-ethoxy-2-oxoethyl)carbamoyl)-3,10,14-trioxo-15-oxa-5,6-dithia-2,9-diazahexadecyl)triphenylphosphoniumbromide; |
| ((8*R*,13*S*)-13-amino-8-((2-ethoxy-2-oxoethyl)carbamoyl)-3,10,14-trioxo-15-oxa-5,6-dithia-2,9-diazahexadecyl)triphenylphosphoniumbromide; |
| (4-amino-9-((2-methoxy-2-oxoethyl)carbamoyl)-3,7,14-trioxo-2-oxa-11,12-dithia-8,15-diazaheptadecan-17-yl)triphenylphosphoniumbromide; |
| ((4*S*,9*R*)-4-amino-9-((2-methoxy-2-oxoethyl)carbamoyl)-3,7,14trioxo-2-oxa-11,12-dithia-8,15-diazaheptadecan-17-yl)triphenylphosphoniumbromide; |
| (4-amino-9-((2-methoxy-2-oxoethyl)carbamoyl)-3,7,14-trioxo-2-oxa-11,12-dithia-8,15-diazaoctadecan-18-yl)triphenylphosphoniumbromide; |
| ((4*S*,9*R*)-4-amino-9-((2-methoxy-2-oxoethyl)carbamoyl)-3,7,14trioxo-2-oxa-11,12-dithia-8,15-diazaoctadecan-18-yl)triphenylphosphoniumbromide; |
| (4-amino-9-((2-methoxy-2-oxoethyl)carbamoyl)-3,7,14-trioxo-2-oxa-11,12-dithia-8,15-diazanonadecan-19-yl)triphenylphosphoniumbromide; |
| ((4*S*,9*R*)-4-amino-9-((2-methoxy-2-oxoethyl)carbamoyl)-3,7,14trioxo-2-oxa-11,12-dithia-8,15-diazanonadecan-19-yl)triphenylphosphoniumbromide; |
| (5-amino-10-((2-ethoxy-2-oxoethyl)carbamoyl)-4,8,15-trioxo-3-oxa-12,13-dithia-9,16-diazahenicosan-21-yl)triphenylphosphoniumbromide; |
| ((5*S*,10*R*)-5-amino-10-((2-ethoxy-2-oxoethyl)carbamoyl)-4,8,15-trioxo-3-oxa-12,13-dithia-9,16-diazahenicosan-21-yl)triphenylphosphoniumbromide; |
| (13-amino-8-((2-ethoxy-2-oxoethyl)carbamoyl)-3,10,14-trioxo-15-oxa-5,6-dithia-2,9-diazaheptadecyl)triphenylphosphoniumbromide; |
| ((8*R*,13*S*)-13-amino-8-((2-ethoxy-2-oxoethyl)carbamoyl)-3,10,14-trioxo-15-oxa-5,6-dithia-2,9-diazaheptadecyl)triphenylphosphoniumbromide; |
| (14-amino-9-((2-ethoxy-2-oxoethyl)carbamoyl)-4,11,15-trioxo-16-oxa-6,7-dithia-3,10-diazaoctadecyl)triphenylphosphoniumbromide; |
| ((9*R*,14*S*)-14-amino-9-((2-ethoxy-2-oxoethyl)carbamoyl)-4,11,15-tn*oxo-16-oxa-6,7-dithia-3,10-diazaoctadecyl)triphenylphosphoniumbromide; |
| (5-amino-10-((2-ethoxy-2-oxoethyl)carbamoyl)-4,8,15-trioxo-3-oxa-12,13-dithia-9,16-diazanonadecan-19-yl)triphenylphosphoniumbromide; |
| ((5*S*,10*R*)-5-amino-10-((2-ethoxy-2-oxoethyl)carbamoyl)-4,8,15-trioxo-3-oxa-12,13-dithia-9,16-diazanonadecan-19-yl)triphenylphosphoniumbromide; |
| (5-amino-10-((2-ethoxy-2-oxoethyl)carbamoyl)-4,8,15-trioxo-3-oxa-12,13-dithia-9,16-diazaicosan-20-yl)triphenylphosphoniumbromide; |
| ((5*S*,10*R*)-5-amino-10-((2-ethoxy-2-oxoethyl)carbamoyl)-4,8,15-trioxo-3-oxa-12,13-dithia-9,16-diazaicosan-20-yl)triphenylphosphoniumbromide; |
| (12-(methoxycarbonyl)-7-((nitrosothio)methyl)-3,6,9,14-tetraoxo-2-oxa-5,8,13-triazaoctadecan-18-yl)triphenylphosphoniumbromide; |
| (12-(ethoxycarbonyl)-7-((nitrosothio)methyl)-3,6,9,14-tetraoxo-2-oxa-5,8,13-triazaoctadecan-18-yl)triphenylphosphoniumbromide; |
| (13-(ethoxycarbonyl)-8-((nitrosothio)methyl)-4,7,10,15-tetraoxo-3-oxa-6,9,14-triazanonadecan-19-yl)triphenylphosphoniumbromide; |
| (13-(methoxycarbonyl)-8-((nitrosothio)methyl)-4,7,10,15-tetraoxo-3-oxa-6,9,14-triazanonadecan-19-yl)triphenylphosphoniumbromide; |
| (2-((2-(4-amino-5-methoxy-5-oxopentanamido)-3-((2-methoxy-2-oxoethyl)amino)-3-oxopropyl)thio)-2-oxoethyl)triphenylphosphoniumbromide; |
| (3-((2-(4-amino-5-methoxy-5-oxopentanamido)-3-((2-methoxy-2-oxoethyl)amino)-3-oxopropyl)thio)-3-oxopropyl)triphenylphosphoniumbromide; |
| (4-((2-(4-amino-5-methoxy-5-oxopentanamido)-3-((2-methoxy-2-oxoethyl)amino)-3-oxopropyl)thio)-4-oxobutyl)triphenylphosphoniumbromide; |
| (5-((2-(4-amino-5-methoxy-5-oxopentanamido)-3-((2-methoxy-2-oxoethyl)amino)-3-oxopropyl)thio)-5-oxopentyl)triphenylphosphoniumbromide; |
| (2-((2-(4-amino-5-ethoxy-5-oxopentanamido)-3-((2-methoxy-2-oxoethyl)amino)-3-oxopropyl)thio)-2-oxoethyl)triphenylphosphoniumbromide; |
| (3-((2-(4-amino-5-ethoxy-5-oxopentanamido)-3-((2-methoxy-2-oxoethyl)amino)-3-oxopropyl)thio)-3-oxopropyl)triphenylphosphoniumbromide; |
| (4-((2-(4-amino-5-ethoxy-5-oxopentanamido)-3-((2-methoxy-2-oxoethyl)amino)-3-oxopropyl)thio)-4-oxobutyl)triphenylphosphoniumbromide; |
| (5-((2-(4-amino-5-ethoxy-5-oxopentanamido)-3-((2-methoxy-2-oxoethyl)amino)-3-oxopropyl)thio)-5-oxopentyl)triphenylphosphoniumbromide; |
| (2-((2-(4-amino-5-methoxy-5-oxopentanamido)-3-((2-ethoxy-2-oxoethyl)amino)-3-oxopropyl)thio)-2-oxoethyl)triphenylphosphoniumbromide; |
| (3-((2-(4-amino-5-methoxy-5-oxopentanamido)-3-((2-ethoxy-2-oxoethyl)amino)-3-oxopropyl)thio)-3-oxopropyl)triphenylphosphoniumbromide; |
| (4-((2-(4-amino-5-methoxy-5-oxopentanamido)-3-((2-ethoxy-2-oxoethyl)amino)-3-oxopropyl)thio)-4-oxobutyl)triphenylphosphoniumbromide; |
| (5-((2-(4-amino-5-methoxy-5-oxopentanamido)-3-((2-ethoxy-2-oxoethyl)amino)-3-oxopropyl)thio)-5-oxopentyl)triphenylphosphoniumbromide; |
| (2-((2-(4-amino-5-ethoxy-5-oxopentanamido)-3-((2-ethoxy-2-oxoethyl)amino)-3-oxopropyl)thio)-2-oxoethyl)triphenylphosphoniumbromide; |
| (3-((2-(4-amino-5-ethoxy-5-oxopentanamido)-3-((2-ethoxy-2-oxoethyl)amino)-3-oxopropyl)thio)-3-oxopropyl)triphenylphosphoniumbromide; |
| (4-((2-(4-amino-5-ethoxy-5-oxopentanamido)-3-((2-ethoxy-2-oxoethyl)amino)-3-oxopropyl)thio)-4-oxobutyl)triphenylphosphoniumbromide; |
| (5-((2-(4-amino-5-ethoxy-5-oxopentanamido)-3-((2-ethoxy-2-oxoethyl)amino)-3-oxopropyl)thio)-5-oxopentyl)triphenylphosphoniumbromide; |
| ((7*R*,12*S*)-12-(methoxycarbonyl)-7-((nitrosothio)methyl)-3,6,9,14-tetraoxo-2-oxa-5,8,13-triazaoctadecan-18-yl)triphenylphosphoniumbromide; |
| ((7*R*,12*S*)-12-(ethoxycarbonyl)-7-((nitrosothio)methyl)-3,6,9,14-tetraoxo-2-oxa-5,8,13-triazaoctadecan-18-yl)triphenylphosphoniumbromide; |
| ((8*R*,13*S*)13-(ethoxycarbonyl)-8-((nitrosothio)methyl)-4,7,10,15-tetraoxo-3-oxa-6,9,14-triazanonadecan-19-yl)triphenylphosphoniumbromide; |
| ((8*R*,13*S*)13-(methoxycarbonyl)-8-((nitrosothio)methyl)-4,7,10,15-tetraoxo-3-oxa-6,9,14-triazanonadecan-19-yl)triphenylphosphoniumbromide; |
| (2-(((*R*)-2-((*S*)-4-amino-5-methoxy-5-oxopentanamido)-3-((2-methoxy-2-oxoethyl)amino)-3-oxopropyl)thio)-2-oxoethyl)triphenylphosphoniumbromide; |
| (3-(((*R*)-2-((*S*)-4-amino-5-methoxy-5-oxopentanamido)-3-((2-methoxy-2-oxoethyl)amino)-3-oxopropyl)thio)-3-oxopropyl)triphenylphosphoniumbromide; |
| (4-(((*R*)-2-((*S*)-4-amino-5-methoxy-5-oxopentanamido)-3-((2-methoxy-2-oxoethyl)amino)-3-oxopropyl)thio)-4-oxobutyl)triphenylphosphoniumbromide; |
| (5-(((*R*)-2-((*S*)-4-amino-5-methoxy-5-oxopentanamido)-3-((2-methoxy-2-oxoethyl)amino)-3-oxopropyl)thio)-5-oxopentyl)triphenylphosphoniumbromide; |
| (2-(((*R*)-2-((S)-4-amino-5-ethoxy-5-oxopentanamido)-3-((2-methoxy-2-oxoethyl)amino)-3-oxopropyl)thio)-2-oxoethyl)triphenylphosphoniumbromide; |
| (3-(((*R*)-2-((*S*)-4-amino-5-ethoxy-5-oxopentanamido)-3-((2-methoxy-2-oxoethyl)amino)-3-oxopropyl)thio)-3-oxopropyl)triphenylphosphonium bromide; |
| (4-(((*R*)-2-((*S*)-(4-amino-5-ethoxy-5-oxopentanamido)-3-((2-methoxy-2-oxoethyl)amino)-3-oxopropyl)thio)-4-oxobutyl)triphenylphosphonium bromide; |
| (5-(((*R*)-2-((*S*)-4-amino-5-ethoxy-5-oxopentanamido)-3-((2-methoxy-2-oxoethyl)amino)-3-oxopropyl)thio)-5-oxopentyl)triphenylphosphonium bromide; |
| (2-(((*R*)-2-((*S*)-4-amino-5-methoxy-5-oxopentanamido)-3-((2-ethoxy-2-oxoethyl)amino)-3-oxopropyl)thio)-2-oxoethyl)triphenylphosphonium bromide; |
| (3-(((*R*)-2-((*S*)-4-amino-5-methoxy-5-oxopentanamido)-3-((2-ethoxy-2-oxoethyl)amino)-3-oxopropyl)thio)-3-oxopropyl)triphenylphosphonium bromide; |
| (4-(((*R*)-2-((*S*)-4-amino-5-methoxy-5-oxopentanamido)-3-((2-ethoxy-2-oxoethyl)amino)-3-oxopropyl)thio)-4-oxobutyl)triphenylphosphonium bromide; |
| (5-(((*R*)-2-((*S*)-4-amino-5-methoxy-5-oxopentanamido)-3-((2-ethoxy-2-oxoethyl)amino)-3-oxopropyl)thio)-5-oxopentyl)triphenylphosphonium bromide; |
| (2-(((*R*)-2-((*S*)-4-amino-5-ethoxy-5-oxopentanamido)-3-((2-ethoxy-2-oxoethyl)amino)-3-oxopropyl)thio)-2-oxoethyl)triphenylphosphonium bromide; |
| (3-(((*R*)-2-((*S*)-4-amino-5-ethoxy-5-oxopentanamido)-3-((2-ethoxy-2-oxoethyl)amino)-3-oxopropyl)thio)-3-oxopropyl)triphenylphosphonium bromide; |
| (4-(((*R*)-2-((*S*)-4-amino-5-ethoxy-5-oxopentanamido)-3-((2-ethoxy-2-oxoethyl)amino)-3-oxopropyl)thio)-4-oxobutyl)triphenylphosphonium bromide; and/or (5-(((*R*)-2-((*S*)-4-amino-5-ethoxy-5-oxopentanamido)-3-((2-ethoxy-2-oxoethyl)amino)-3-oxopropyl)thio)-5-oxopentyl)triphenylphosphonium bromide. |

The compounds illustrated and named above are not limiting examples and the present disclosure contemplates compounds wherein linkers comprising more than five repeating alkylene oxide and/or methylene units are used. The present disclosure also contemplates compounds wherein any methyl or ethyl protecting group provided in the compound structures/names above is replaced with another protecting groups, such as aliphatic groups (e.g., propyl, butyl, pentyl, hexyl, septyl, octyl, nonyl, decyl, and the like) or other protecting groups disclosed herein.

Compound fragments that can be assembled to form the compounds described above can also be useful in the methods described herein. Some compounds can have structures satisfying any one of Formulas 7-12 illustrated below. 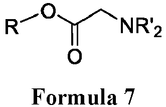 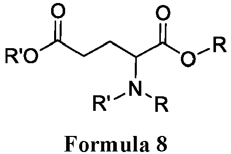 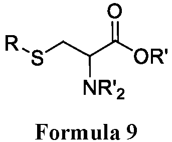 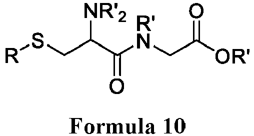 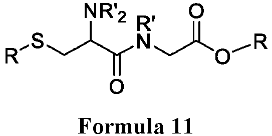 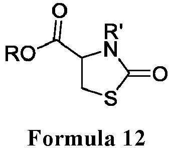 With reference to Formulas 7-12, each R independently can be selected from a protecting group or a linker-X group and each R' independently can be selected from hydrogen or a protecting group. In particular disclosed instances, the protecting group can be selected from aliphatic, aryl, heteroaliphatic, haloaliphatic, aliphatic-aryl, heteroaryl, aliphatic-heteroaryl, haloaliphatic-aryl, haloaliphatic-heteroaryl, heteroaliphatic-aryl, or heteroaliphatic-heteroaryl. In exemplary instances, the protecting group can be selected from alkyl, alkenyl, or alkynyl; heteroalkyl, heteroalkenyl, or heteroalkynyl; haloalkyl, haloalkenyl, or haloalkynyl; alkyl-aryl, alkenyl-aryl, or alkynyl-aryl; heteroalkyl-aryl, heteroalkenyl-aryl, or heteroalkynyl-aryl; haloalkyl-aryl, haloalkenyl-aryl, or haloalkynyl-aryl; heteroalkyl-heteroaryl, heteroalkenyl-heteroaryl, or heteroalkynyl-heteroaryl; alkyl-heteroaryl, alkenyl-heteroaryl, or alkynyl-heteroaryl; haloalkyl-heteroaryl, haloalkenyl-heteroaryl, or haloalkynyl-heteroaryl. In yet additional exemplary instances, the protecting group can be selected from methyl, ethyl, propyl, butyl, pentyl, hexyl, septyl, octyl, nonyl, decyl, phenyl, naphthyl, -(CH₂)ₘPh, wherein m is 1 to 20, fluorenylmethyl, silyl-oxyether (-[(CH₂)ₙO]ₘ-[(CH₂)ₚ]SiR^{a}₃, wherein n ranges from 1 to 10, m ranges from 0 to 10, and p ranges from 1 to 10), trihaloalkyl, diaryl, alkyldiaryl, or esters thereof.

The linker-X group can comprise a linker group selected from a carbonyl-containing group, an alkylene oxide, an imidoester, or linker group generated from a maleimide, a haloacetyl, or a pyridyl disulfide. In some instances, the linker-X group can be selected from -C(O)R^{c}X, -[(CH₂)₂O]ₘX, -C(=NH₂⁺)NR^{c}X, -CH₂C(O)NHR^{c}X, -SR^{c}X, or 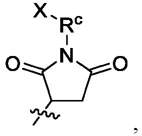 wherein each R^{c} independently can be selected from aliphatic, aryl, heteroaliphatic, aliphatic-aryl, heteroaryl, aliphatic-heteroaryl, heteroaliphatic-aryl, or heteroaliphatic-heteroaryl; each X independently can be selected from -P⁺(R^{d})₃, -N⁺(R^{d})₃, wherein each R^{d} independently is selected from hydrogen, aliphatic, aryl, or aliphatic-aryl; and m independently can be 1 to 30, such as 1 to 20, or 1 to 10, or 1 to 5. In particular disclosed instances, the linker-X group can be selected from -C(O)(CH₂)₁₋₃₀(P)⁺Ph₃Br⁻, -C(=NH₂⁺)N(CH₂)₁₋₃₀(P)⁺Ph₃Br⁻, -CH₂C(O)NH(CH₂)_{1.30}(P)⁺Ph₃Br⁻, -S(CH₂)₁₋₃₀(P)⁺Ph₃Br⁻, -(CH₂)₁₋₃₀(P)⁺Ph₃Br⁻, -C(O)[O(CH₂)₂]₁₋₃₀(P)⁺Ph₃Br⁻, -C(=NH₂⁺)NCH₂[O(CH₂)₂]₁₋₃₀(P)⁺Ph₃Br⁻, -CH₂C(O)NH[O(CH₂)₂]₁₋₃₀(P)⁺Ph₃Br⁻, -S[O(CH₂)₂]₁₋₃₀(P)⁺Ph₃Br⁻, -C(O)(CH₂)₁₋₃₀(N)⁺Me₃Br⁻, -C(=NH₂⁺)NCH₂[O(CH₂)₂]₁₋₃₀(N)⁺Me₃Br⁻, -CH₂C(O)NH(CH₂)₁₋₃₀(N)⁺Me₃Br⁻, -S(CH₂)₁₋₃₀(N)⁺Me₃Br⁻, -(CH₂)₃₋₃₀(N)⁺Me₃Br⁻,-S[O(CH₂)₂]₁₋₃₀(N)⁺Me₃Br⁻,

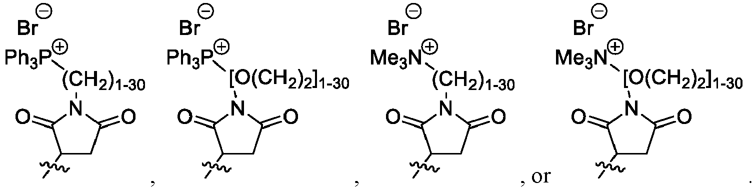

In exemplary aspects, the compound fragments can be selected from any one of the following illustrated below in Table 3.

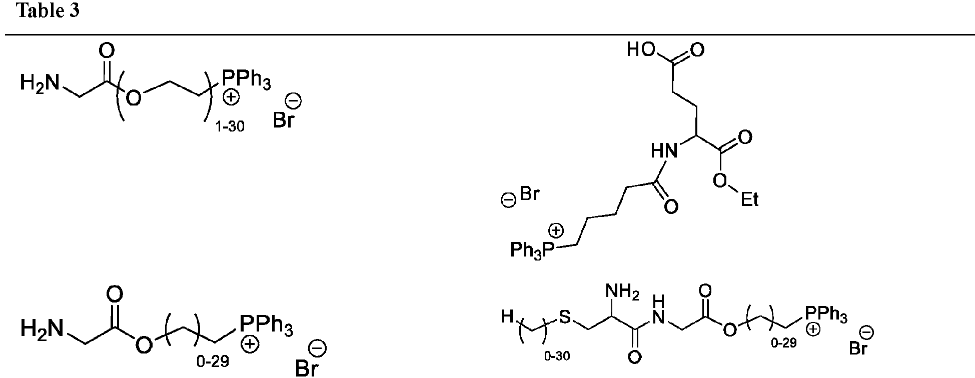

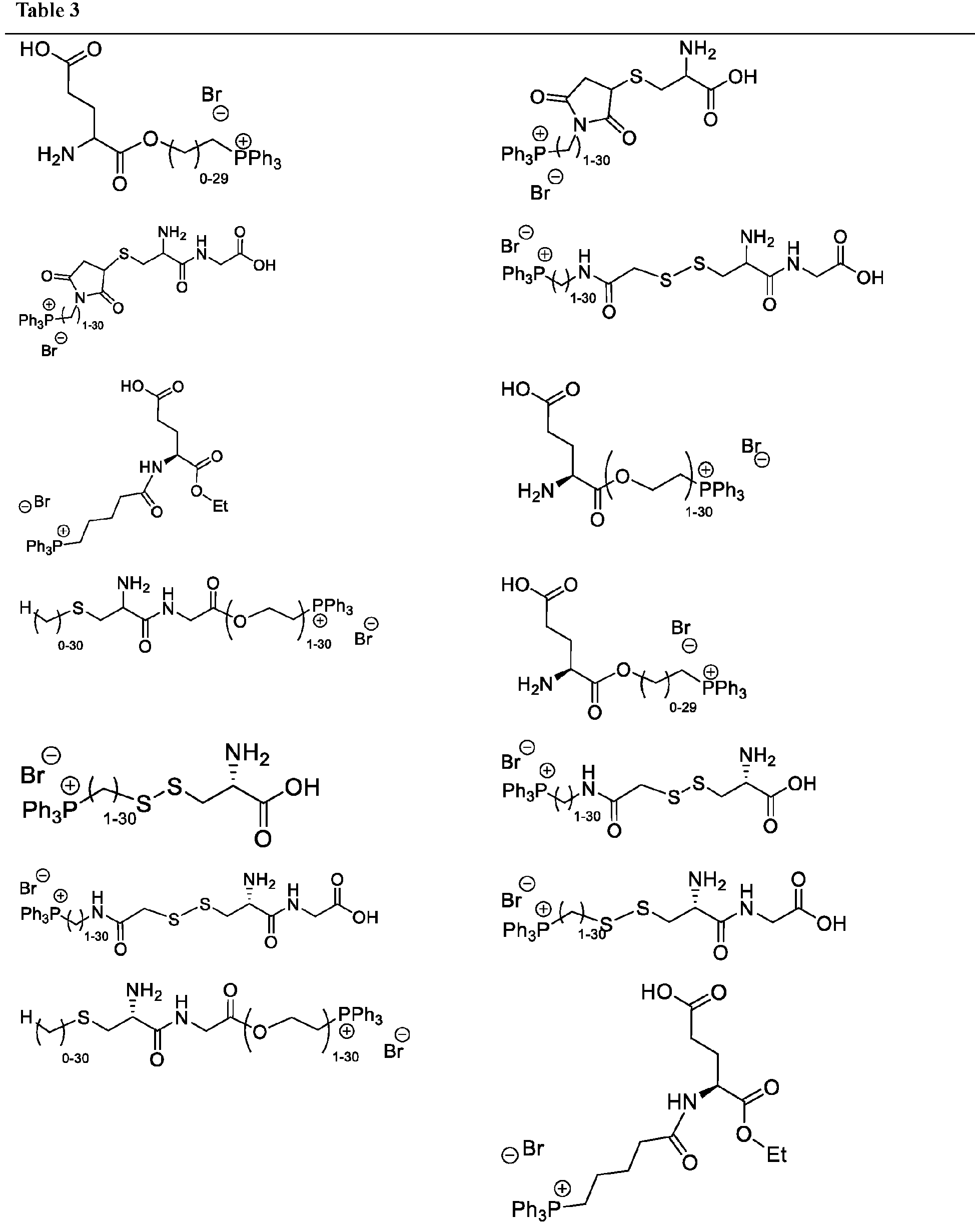

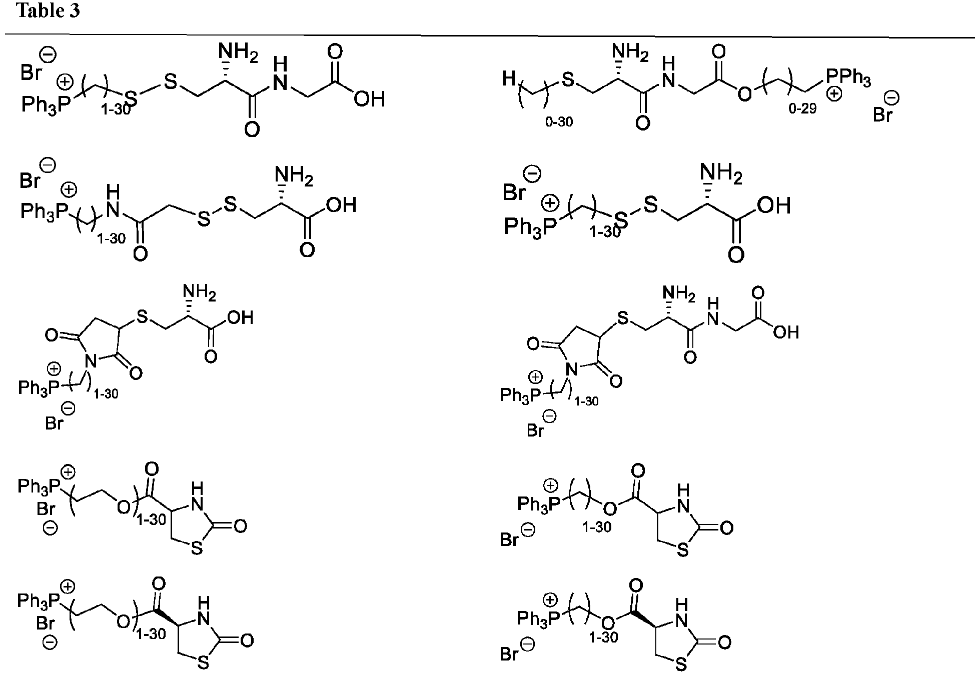

In some aspects, the compounds are selected from those listed below in Table 4.

**Table 4**

| |
|---|
| (2-(glycyloxy)ethyl)triphenylphosphonium bromide; |
| (2-(2-(glycyloxy)ethoxy)ethyl)triphenylphosphonium bromide; |
| (2-(2-(2-(glycyloxy)ethoxy)ethoxy)ethyl)triphenylphosphonium bromide; |
| (14-amino-13-oxo-3,6,9,12-tetraoxatetradecyl)triphenylphosphonium bromide; |
| (17-amino-16-oxo-3,6,9,12,15-pentaoxaheptadecyl)triphenylphosphonium bromide; |
| (5-((4-carboxy-1-ethoxy-1-oxobutan-2-yl)amino)-5 -oxopentyl)triphenylphosphonium bromide; |
| (3-(glycyloxy)propyl)triphenylphosphonium bromide; |
| (4-(glycyloxy)butyl)triphenylphosphonium bromide; |
| (5-(glycyloxy)pentyl)triphenylphosphonium bromide; |
| (6-(glycyloxy)hexyl)triphenylphosphonium bromide; |
| ((cysteinylglycyl)oxy)methyl)triphenylphosphonium bromide; |
| ((*L*-cysteinylglycyl)oxy)ethyl)triphenylphosphonium bromide; |
| (2-((cysteinylglycyl)oxy)ethyl)triphenylphosphonium bromide; |
| (2-((*L*-cysteinylglycyl)oxy)ethyl)triphenylphosphonium bromide; |
| (3-((cysteinylglycyl)oxy)propyl)triphenylphosphonium bromide; |
| (3-((*L*-cysteinylglycyl)oxy)propyl)triphenylphosphonium bromide; |
| (4-((cysteinylglycyl)oxy)butyl)triphenylphosphonium bromide; |
| (4-((*L*-cysteinylglycyl)oxy)butyl)triphenylphosphonium bromide; |
| (5-((cysteinylglycyl)oxy)pentyl)triphenylphosphonium bromide; |
| (5-((*L*-cysteinylglycyl)oxy)pentyl)triphenylphosphonium bromide; |
| (6-((cysteinylglycyl)oxy)hexyl)triphenylphosphonium bromide; |
| (6-((*L*-cysteinylglycyl)oxy)hexyl)triphenylphosphonium bromide; |
| ((glutamyloxy)methyl)triphenylphosphonium bromide; |
| (2-(glutamyloxy)ethyl)triphenylphosphonium bromide; |
| (3-(glutamyloxy)ethyl)triphenylphosphonium bromide; |
| (4-(glutamyloxy)butyl)triphenylphosphonium bromide; |
| (5-(glutamyloxy)pentyl)triphenylphosphonium bromide; |
| (6-(glutamyloxy)hexyl)triphenylphosphonium bromide; |
| ((3-((2-amino-2-carboxyethyl)thio)-2,5 -dioxopyrrolidin-1-yl)methyl)triphenylphosphonium bromide; |
| ((3-(((*R*)-2-amino-2-carboxyethyl)thio)-2,5-dioxopyrrolidin-1-yl)methyl)triphenylphosphonium bromide; |
| (2-(3-((2-amino-2-carboxyethyl)thio)-2,5-dioxopyrrolidin-1-yl)ethyl)triphenylphosphonium bromide; |
| (2-(3-(((*R*)-2-amino-2-carboxyethyl)thio)-2,5-dioxopyrrolidin-1-yl)ethyl)triphenylphosphonium bromide; |
| (3-(3-((2-amino-2-carboxyethyl)thio)-2,5-dioxopyrrolidin-1-yl)propyl)triphenylphosphonium bromide; |
| (3-(3-(((*R*)-2-amino-2-carboxyethyl)thio)-2,5-dioxopyrrolidin-1-yl)propyl)triphenylphosphonium bromide; |
| (4-(3-((2-amino-2-carboxyethyl)thio)-2,5 -dioxopyrrolidin-1-yl)butyl)triphenylphosphonium bromide; |
| (4-(3-(((*R*)-2-amino-2-carboxyethyl)thio)-2,5-dioxopyrrolidin-1-yl)butyl)triphenylphosphonium bromide; |
| (5-(3-((2-amino-2-carboxyethyl)thio)-2,5-dioxopyrrolidin-1-yl)pentyl)triphenylphosphonium bromide; |
| (5-(3-(((*R*)-2-amino-2-carboxyethyl)thio)-2,5-dioxopyrrolidin-1-yl)pentyl)triphenylphosphonium bromide; |
| ((3-((2-amino-3-((carboxymethyl)amino)-3-oxopropyl)thio)-2,5-dioxopyrrolidin-1-yl)methyl)triphenylphosphonium bromide; |
| ((3-(((*R*)-2-amino-3-((carboxymethyl)amino)-3-oxopropyl)thio)-2,5-dioxopyrrolidin-1-yl)methyl)triphenylphosphonium bromide; |
| (2-(3-((2-amino-3-((carboxymethyl)amino)-3-oxopropyl)thio)-2,5-dioxopyrrolidin-1-yl)ethyl)triphenylphosphonium bromide; |
| (2-(3-(((*R*)-2-amino-3-((carboxymethyl)amino)-3-oxopropyl)thio)-2,5-dioxopyrrolidin-1-yl)ethyl)triphenylphosphonium bromide; |
| (3-(3-((2-amino-3-((carboxymethyl)amino)-3-oxopropyl)thio)-2,5-dioxopyrrolidin-1-yl)propyl)triphenylphosphonium bromide; |
| (3-(3-(((*R*)-2-amino-3-((carboxymethyl)amino)-3-oxopropyl)thio)-2,5-dioxopyrrolidin-1-yl)propyl)triphenylphosphonium bromide; |
| (4-(3-((2-amino-3-((carboxymethyl)amino)-3-oxopropyl)thio)-2,5-dioxopyrrolidin-1-yl)butyl)triphenylphosphonium bromide; |
| (4-(3-(((*R*)-2-amino-3-((carboxymethyl)amino)-3-oxopropyl)thio)-2,5-dioxopyrrolidin-1-yl)butyl)triphenylphosphonium bromide; |
| (5-(3-((2-amino-3-((carboxymethyl)amino)-3-oxopropyl)thio)-2,5-dioxopyrrolidin-1-yl)pentyl)triphenylphosphonium bromide; |
| (5-(3-(((*R*)-2-amino-3-((carboxymethyl)amino)-3-oxopropyl)thio)-2,5-dioxopyrrolidin-1-yl)pentyl)triphenylphosphonium bromide; |
| ((2-((2-amino-3-((carboxymethyl)amino)-3-oxopropyl)disulfanyl)acetamido)methyl)triphenylphosphonium bromide; |
| (*R*)-((2-((2-amino-3-((carboxymethyl)amino)-3-oxopropyl)disulfanyl)acetamido)methyl)triphenylphosphonium bromide; |
| (2-((2-((2-amino-3-((carboxymethyl)amino)-3 - oxopropyl)disulfanyl)acetamido)ethyl)triphenylphosphonium bromide; |
| (*R*)-(2-((2-((2-amino-3-((carboxymethyl)amino)-3-oxopropyl)disulfanyl)acetamido)ethyl)triphenylphosphonium bromide; |
| (3-(2-((2-amino-3-((carboxymethyl)amino)-3-oxopropyl)disulfanyl)acetamido)propyl)triphenylphosphonium bromide; |
| (*R*)-(3-(2-((2-amino-3-((carboxymethyl)amino)-3-oxopropyl)disulfanyl)acetamido)propyl)triphenylphosphonium bromide; |
| (4-(2-((2-amino-3 -((carboxymethyl)amino)-3 - oxopropyl)disulfanyl)acetamido)butyl)triphenylphosphonium bromide; |
| (*R*)-(4-(2-((2-amino-3-((carboxymethyl)amino)-3-oxopropyl)disulfanyl)acetamido)butyl)triphenylphosphonium bromide; |
| (5-(2-((2-amino-3-((carboxymethyl)amino)-3-oxopropyl)disulfanyl)acetamido)pentyl)triphenylphosphonium bromide; |
| (*R*)-(5-(2-((2-amino-3-((carboxymethyl)amino)-3-oxopropyl)disulfanyl)acetamido)pentyl)triphenylphosphonium bromide; |
| (*S*)-(5-((4-carboxy-1-ethoxy-1-oxobutan-2-yl)amino)-5-oxopentyl)triphenylphosphonium bromide; |
| (2-((2-oxothiazolidine-4-carbonyl)oxy)ethyl)triphenylphosphonium bromide; |
| (*R*)-(2-((2-oxothiazolidine-4-carbonyl)oxy)ethyl)triphenylphosphonium bromide; |
| (2-(2-((cysteinylglycyl)oxy)ethoxy)ethyl)triphenylphosphonium bromide; |
| (R)-(2-(2-((cysteinylglycyl)oxy)ethoxy)ethyl)triphenylphosphonium bromide; |
| (14-amino-15-mercapto-10,13-dioxo-3,6,9-trioxa-12-azapentadecyl)triphenylphosphonium bromide; |
| (*R*)-(14-amino-15-mercapto-10,13-dioxo-3,6,9-trioxa-12-azapentadecyl)triphenylphosphonium bromide; |
| (17-amino-18-mercapto-13,16-dioxo-3,6,9,12-tetraoxa-15-azaoctadecyl)triphenylphosphonium bromide; |
| (*R*)-(17-amino-18-mercapto-13,16-dioxo-3,6,9,12-tetraoxa-15-azaoctadecyl)triphenylphosphonium bromide; |
| (2-(2-((2-oxothiazolidine-4-carbonyl)oxy)ethoxy)ethyl)triphenylphosphonium bromide; |
| (*R*)-(2-(2-((2-oxothiazolidine-4-carbonyl)oxy)ethoxy)ethyl)triphenylphosphonium bromide; |
| (2-(2-(2-((2-oxothiazolidine-4-carbonyl)oxy)ethoxy)ethoxy)ethyl)triphenylphosphonium bromide; |
| (*R*)-(2-(2-(2-((2-oxothiazolidine-4-carbonyl)oxy)ethoxy)ethoxy)ethyl)triphenylphosphonium bromide; |
| ((2-oxothiazolidine-4-carbonyl)oxy)methyl)triphenylphosphonium bromide; |
| (*R*)-((2-oxothiazolidine-4-carbonyl)oxy)methyl)triphenylphosphonium bromide; |
| (2-((2-oxothiazolidine-4-carbonyl)oxy)ethyl)triphenylphosphonium bromide; |
| (*R*)-(2-((2-oxothiazolidine-4-carbonyl)oxy)ethyl)triphenylphosphonium bromide; |
| (3-((2-oxothiazolidine-4-carbonyl)oxy)propyl)triphenylphosphonium bromide; |
| (*R*)-(3-((2-oxothiazolidine-4-carbonyl)oxy)propyl)triphenylphosphonium bromide; |
| (4-((2-oxothiazolidine-4-carbonyl)oxy)butyl)triphenylphosphonium bromide; |
| (*R*)-(4-((2-oxothiazolidine-4-carbonyl)oxy)butyl)triphenylphosphonium bromide; |
| (5-((2-oxothiazolidine-4-carbonyl)oxy)pentyl)triphenylphosphonium bromide; and/or |
| (*R*)-(5-((2-oxothiazolidine-4-carbonyl)oxy)pentyl)triphenylphosphonium bromide. |

### III. Methods of Making Compounds

Examples of the compounds disclosed herein can be made using the methods described below. To the extent that the disclosed methods are methods of making compounds that are not compounds of the invention, then the methods are described herein for reference purposes only.

In particular disclosed aspects, the compounds can be made using peptide coupling conditions in combination with the addition of one or more protecting groups and/or targeting moieties. 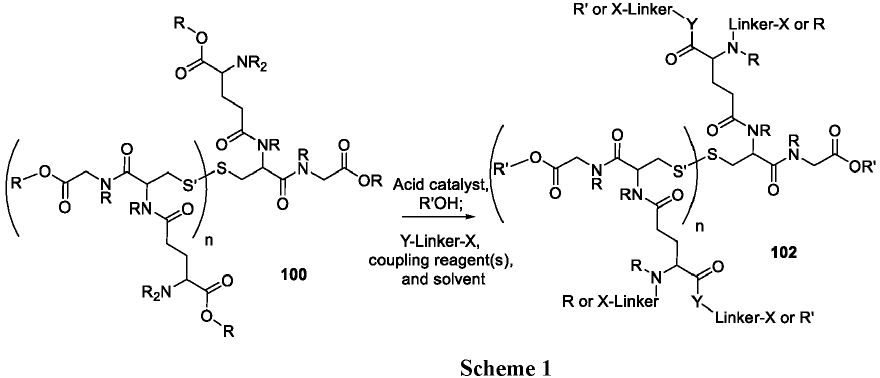 With reference to Scheme 1, an acid precursor compound **100** can be exposed to an acid catalyst and an alcohol; a targeting moiety reagent (e.g., Y-Linker-X, wherein Y is a group that can be displaced upon reaction of the linker group with an amine to form an N-Linker-X group as illustrated in Scheme 1, or Y is an nitrogen-containing group that becomes bound to a carbonyl moiety as illustrated in Scheme 1), a coupling reagent, and a base; or a combination thereof to form compound 102. The alcohol can be an alcohol having a formula R'OH, wherein R' is protecting group selected from aliphatic, aryl, heteroaliphatic, aliphatic-aryl, heteroaryl, aliphatic-heteroaryl, heteroaliphatic-aryl, or heteroaliphatic-heteroaryl an the acid catalyst can be selected from, for example, thionyl chloride, tosyl chloride, mesyl chloride, or the like. Any suitable targeting moiety reagent can be used, with certain examples having a formula Y-linker-X, wherein the linker-X group is as described herein; or a Y-linker group, which can then be reacted with a targeting moiety, as described herein. Suitable coupling reagents include, but are not limited to 2-(7-aza-1H-benzotriazol-1-yl)-N,N,N',N'-tetramethylaminium hexafluorophosphate, 2-(1H-benzotriazol-1-yl)-N,N,N',N'-hexafluorophosphate, 2-(6-chloro-1H-benzotriazol-1-yl)-N,N,N',N'-tetramethylaminium hexafluorophosphate, 1-hydroxybenzotriazole, dicyclohexylcarbodiimide, diisopropylcarbodiimide, N-(3-dimethylaminopropyl)-N'-ethylcarbodiimide·HCl, benzotriazol-1-yloxy-tris(dimethylamino)-phosphonium hexafluorophosphate, benzotriazol-1-yloxy-tripyrrolidino-phosphonium hexafluorophosphate, bromo-tripyrrolidino-phosphonium hexafluorophosphate, and the like, or a combination thereof. Suitable bases include, but are not limited to an amine base, such as di-isopropylethyl amine, isopropyl amine, and the like. With reference to Scheme 1 (and the schemes illustrated below), each R independently can be selected from hydrogen or a protecting group as described for the formulas provided herein; each R' independently can be a protecting group as described herein; and each linker-X group is as described for the formulas provided herein. The order in which the steps illustrated in Scheme 1 are performed can vary. For example, in some aspects, acid precursor compound **100** can first be reacted with the alcohol and acid catalyst to provide intermediate **104** and then the targeting moiety reagent to provide product **102** (see Scheme 1A). In other aspects, acid precursor compound **100** can first be reacted with the targeting moiety reagent and the coupling reagent to provide intermediate **106** and subsequently reacted with the alcohol and acid catalyst to provide product **102** (see Scheme 1B).

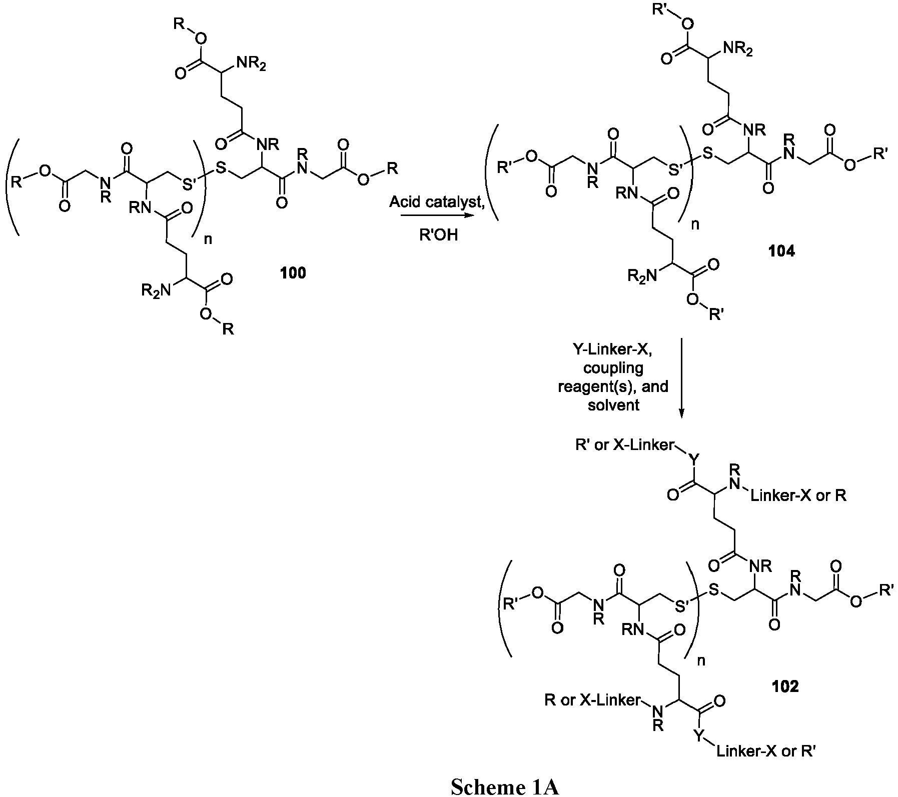

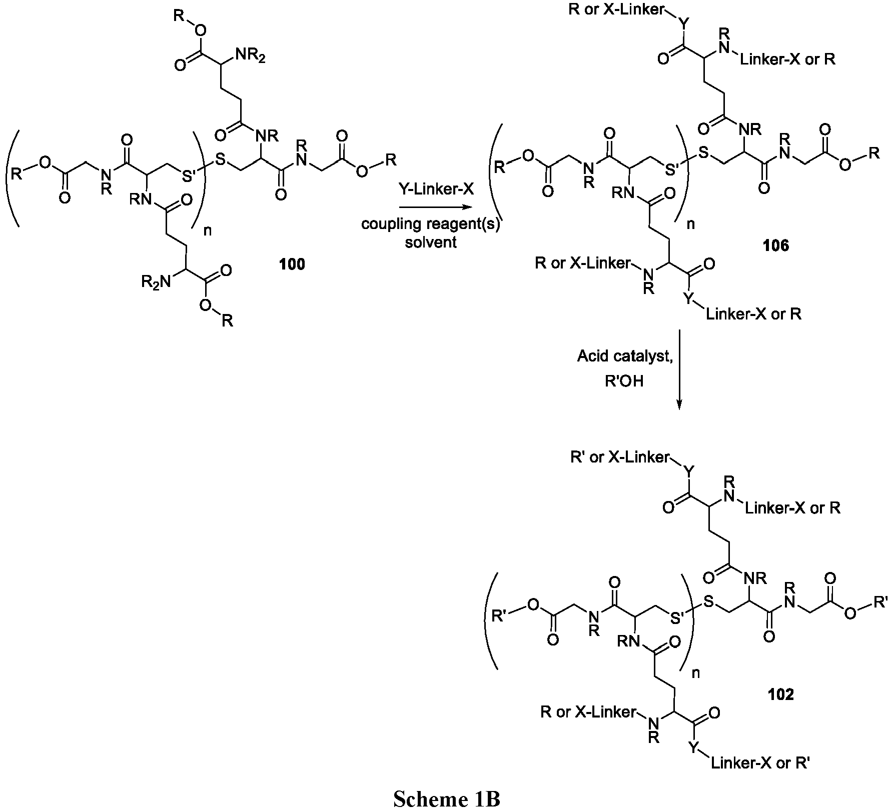

An exemplary method of making the compounds disclosed herein is illustrated below in Scheme 2. As illustrated in Scheme 2, GSSG (glutathione disulfide) **200** is reacted with thionyl chloride and an alcohol (e.g., methanol and/or ethanol) to provide ester products **202A** and/or **202B.** Ester products **202A** and **202B** are then reacted with the phosphonium salt **204** and coupling reagents shown in Scheme 2 to provide the TPP-containing glutathione compounds **206A** and **206B.**

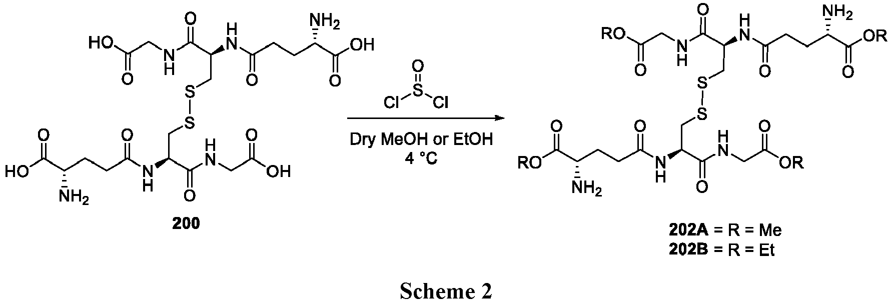

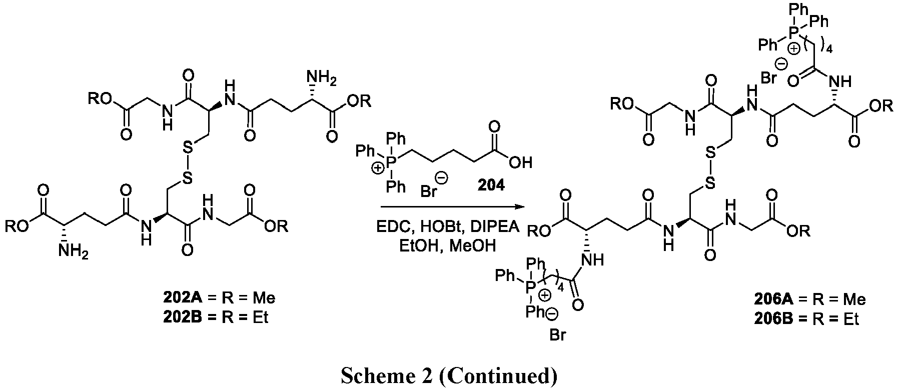

Additional methods for making the compounds described herein are illustrated in Scheme 3. With reference to Scheme 3, the disulfide precursor **300** can undergo a peptide coupling with ethyl glycinate hydrochloride to provide amide product **302.** Deprotection of the Boc protecting groups on amide product **302** can be carried out with a suitable acid to provide the free amine product **304,** which can then be coupled with protected peptide **306** to make the protected disulfide product **308.** Deprotection of the acids of protected disulfide product **308** results in free acid product **310,** which can be reacted with triphosphonium salt **312** to provide a target moiety-containing product **314.** Upon treatment with a base, the amine moieties of product **314** can be deprotected selectively to provide the targeting compound **316.**

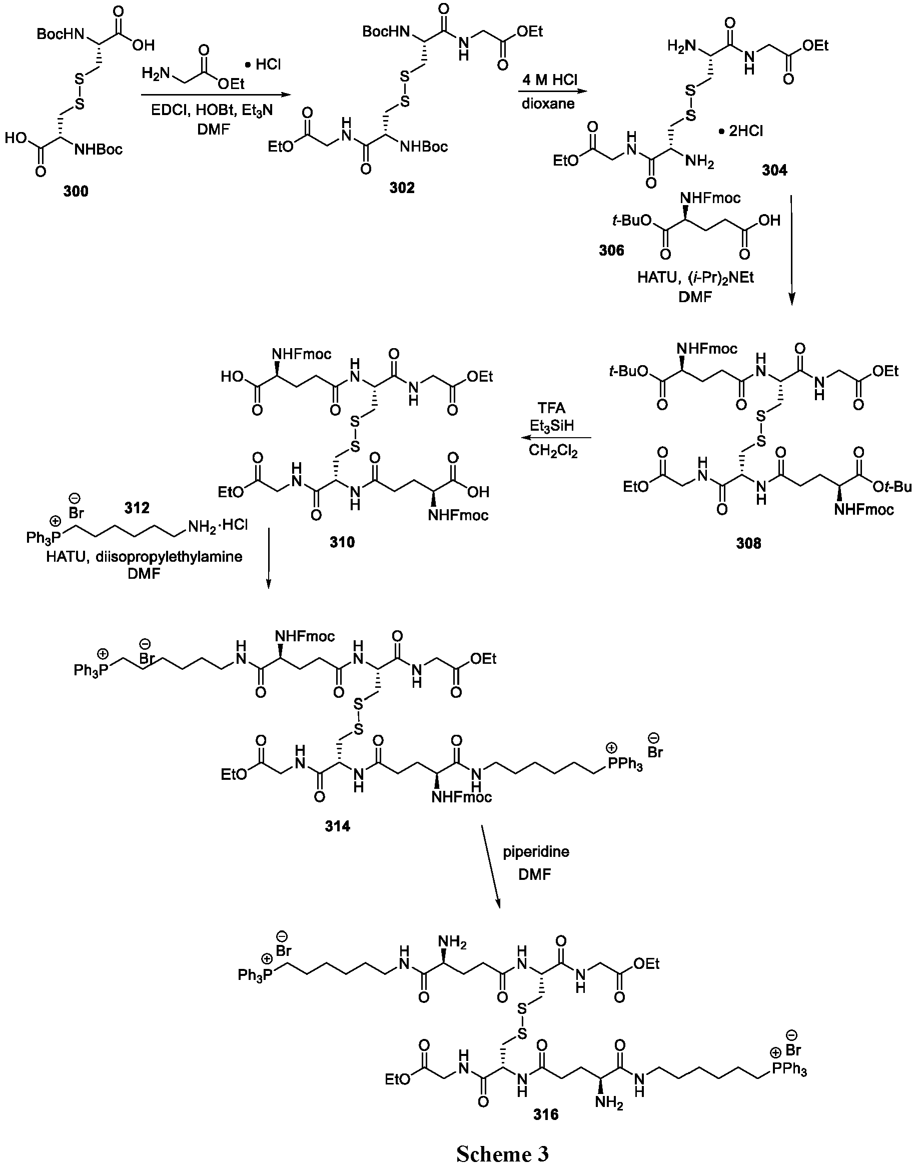

In additional examples, compounds having structures satisfying any one of Formulas 7-12 can be made by exposing one or more peptides selected from glycine, cysteine, or glutamic acid to a targeting moiety reagent, a coupling reagent, and a base as described herein. In some aspects, one or more peptides selected from glycine, cysteine, or glutamic acid can first be coupled together using suitable peptide coupling reagents, such as those described herein, and then the coupled di- or tri-peptide can be exposed to a targeting moiety reagent, a coupling reagent, and a base. In yet additional aspects, the one or more peptides can first be exposed to a targeting moiety reagent, a coupling reagent, and base and then coupled to another peptide to form a targeting di- or tri-peptide.

### IV. Methods of Use

The compounds disclosed herein can be used to maintain or increase glutathione levels in a cell, subject, or sample. Methods of using the compounds can comprise administering a therapeutically effective amount of the compound to a cell, subject, or a sample. In particular disclosed embodiments, the compounds are for use in a method to treat, ameliorate, and/or prevent diseases or conditions associated with low or reduced glutathione levels. In some embodiments, the compounds are for use in a method to treat, ameliorate, and/or prevent diseases and/or conditions associated with low mitochondrial glutathione levels, such as diseases associated with age-related glutathione loss or toxicological insults that target mitochondria. In some embodiments, the compounds are for use in a method to prevent or mitigate any one or more of the following: (1) attenuation of electron transport chain (ETC) activity, especially through oxidation of critical thiols on ETC complexes; (2) allosteric inhibition of dicarboxylate transport, which adversely slows the TCA cycle; (3) uncoupling of the GSH/GSSG redox network from the NADH/NAD⁺ redox couple, lowering mitochondrial membrane potential and ATP synthesis; (4) mitochondria susceptibility to oxidants emanating from the ETC, and preventing GSH-dependent peroxidases from eliminating hydrogen peroxide and lipid hydroperoxides; (5) enhanced susceptibility to drugs and toxins that can contribute to "polypharmacy" in the elderly; and (6) initiating permeability pore opening and release of pro-apoptotic factors (cytochrome c, AIF). In yet additional embodiments, the compounds are for use in a method to treat, ameliorate, or prevent neurodegeneration (e.g., Parkinson's disease, amyotrophic lateral sclerosis, dementia, Alzheimer's disease, Huntington's disease, or the like), stroke, diabetes, cardiovascular disease (or other heart-related diseases, such as chronic heart failure), cancer and side effects associated with chemotherapy, muscle injuries and myopathies, viral infections, periodontal disease, vascular impairment, kidney disease, ischemia-reperfusion injury, wound repair issues, inflammation, schizophrenia and other neuro-psychological disorders, as well as acetaminophen toxicity or acute alcohol toxicity.

The compounds disclosed herein can be administered to a subject. In some embodiments, the compounds can be formulated as a pharmaceutical composition. Pharmaceutical compositions contemplated by the present disclosure can include, but are not limited to, pharmaceutical compositions comprising at least one compound disclosed herein, and a pharmaceutically-acceptable excipient selected from, for example, an adjuvant, a carrier, a stabilizer, or combinations thereof. The pharmaceutical compositions also can include diluents, fillers, binding agents, moisturizing agents, preservatives, acids, and the like. The compounds described herein may be used alone, in combination with one or more additional compounds, or as an adjunct to, or in combination with, an established therapy. In some embodiments, the compounds may be for use in combination with other therapeutic agents useful for the disorder or condition being treated. These compounds may be administered simultaneously, sequentially in any order, by the same route of administration, or by a different route. Examples of such compounds include N-acetyl cysteine, vitamin E, vitamin C, and diacetyl-bis(4-methylthiosemicarbazonato)copper(II) (CuATSM).

Pharmaceutical compositions comprising the compounds disclosed herein can be administered as pharmaceutically acceptable formulations in the form of solids, liquids, and/or lotions. Suitable solid forms of administration include, but are not limited to, tablets, capsules, powders, solid dispersions, and the like. Suitable liquid or lotion forms include, but are not limited to, oil-in-water or water-in-oil emulsions, aqueous gel compositions, or liquids or lotions formulated for use as foams, films, sprays, ointments, pessary forms, suppository forms, creams, liposomes or in other forms embedded in a matrix for the slow or controlled release of the compound to the skin or surface onto which it has been applied or is in contact.

Compositions comprising the compounds or pharmaceutically acceptable components may be formulated so as to be suitable for a variety of modes of administration, including, but not limited to, topical, ocular, oral, buccal, systemic, nasal, injection (such as intravenous, intraperitoneal, subcutaneous, intramuscular, or intrathecal), transdermal, rectal, vaginal, etc., or a form suitable for administration by inhalation or insufflation.

For oral or buccal administration, the pharmaceutical compositions may take the form of lozenges, tablets or capsules prepared by conventional means with pharmaceutically acceptable excipients. The tablets or capsules may be coated by methods well known in the art with, for example, sugars, films, or enteric coatings.

Liquid preparations of the compounds disclosed herein for oral administration may take the form of, for example, elixirs, solutions, syrups or suspensions, or they may be presented as a dry product for constitution with water or other suitable vehicle before use. Preparations for oral administration also may be suitably formulated to give controlled release of the compound.

For topical administration, the compounds can be formulated as solutions, lotions, gels, ointments, creams, suspensions, etc. For transmucosal administration, penetrants appropriate to the barrier to be permeated can be used in the formulation.

Systemic formulations include those designed for administration by injection, e.g., subcutaneous, intravenous, intramuscular, intrathecal or intraperitoneal injection, as well as those designed for transdermal, transmucosal oral or pulmonary administration. Useful injectable preparations include sterile suspensions, solutions or emulsions of the compound in aqueous or oily vehicles. The pharmaceutical compositions may also contain formulating agents, such as suspending, stabilizing and/or dispersing agents.

For rectal and vaginal routes of administration, the compounds or compositions thereof may be formulated as solutions (for retention enemas) suppositories or ointments containing conventional suppository bases, such as cocoa butter or other glycerides.

For nasal administration or administration by inhalation or insufflation, the compounds and/or compositions thereof can be conveniently delivered in the form of an aerosol spray from pressurized packs or a nebulizer with the use of a suitable propellant. In the case of a pressurized aerosol, the dosage unit may be determined by providing a valve to deliver a metered amount. Capsules and cartridges for use in an inhaler or insufflator (for example capsules and cartridges comprised of gelatin) may be formulated containing a powder mix of the compound and a suitable powder base such as lactose or starch.

The dosage of compounds as disclosed herein will depend on various factors, including the age, weight, general health, and severity of the condition of the subject being treated. Dosage may also need to be tailored to the sex of the individual. Dosage, and frequency of administration of the compounds will also depend on whether the compounds are formulated for treatment of acute episodes of a condition or for the prophylactic treatment of a disorder.

Effective dosages can be estimated initially from *in vitro* assays. For example, an initial dosage for use in subjects can be formulated to achieve a circulating blood or serum concentration of active compound that is at or above an IC₅₀ or EC₅₀ of the particular compound as measured in an *in vitro* assay. Dosages can be calculated to achieve such circulating blood or serum concentrations taking into account the bioavailability of the particular compound.

Dosage amounts, such as therapeutically effective amounts, of the disclosed compounds or pharmaceutical compositions thereof will typically be in the range of from about greater than 0 mg/kg/day, such as 0.0001 mg/kg/day or 0.001 mg/kg/day or 0.01 mg/kg/day, up to about 100 mg/kg/day. More typically, the dosage (or effective amount) may range from about 0.0025 mg/kg to about 1 mg/kg administered at least once per day, such as from 0.01 mg/kg to about 0.5 mg/kg or from about 0.05 mg/kg to about 0.15 mg/kg. The total daily dosage typically ranges from about 0.1 mg/kg to about 5 mg/kg or to about 20 mg/kg per day, such as from 0.5 mg/kg to about 10 mg/kg per day or from about 0.7 mg/kg per day to about 2.5 mg/kg/day.

Compositions comprising one or more of the compounds disclosed herein typically comprise from greater than 0 up to 99% of the compound by total weight percent. More typically, compositions comprising one or more of the compounds disclosed herein comprise from about 1 to about 20 total weight percent of the compound, and from about 80 to about 99 weight percent of at least one pharmaceutically-acceptable component, such as an adjuvant.

### V. Examples

Any examples relating to compounds outside the scope of the claims are included herein for reference purposes only.

Chemical reactions were performed under an argon atmosphere employing oven-dried glassware. Analytical thin layer chromatography (TLC) was performed using silica plates (60 Å) with a fluorescent indicator (254 nm), which were visualized with a UV lamp and ceric ammonium molybdate (CAM) solution. Chromatographic purification of products was performed on silica gel (60 Å, 72-230 mesh). Proton NMR spectra were recorded on Bruker 400 MHz spectrometers. Proton chemical shifts are reported in parts per million (ppm) (δ) relative to the residual solvent signals as the internal standard (CDCl₃: δH 7.26; MeOH-d₄: δH 3.31; DMSO-d₆: δH 2.50, DMF-d₇: δH 8.03). Multiplicities in the 1H NMR spectra are described as follows: s = singlet, bs = broad singlet, d = doublet, t = triplet, bt = broad triplet, q = quartet, m = multiplet; coupling constants are reported in hertz (Hz). Carbon NMR spectra were recorded on a Bruker 700 (175 MHz) spectrometer with complete proton decoupling. Carbon chemical shifts are reported in parts per million (ppm) (δ) relative to the residual solvent signal as the internal standard (CDCl₃: δC 77.16; MeOH-d₄: δC 49.0; DMSO-d₆: δC 39.52, DMF-d₇: δC 163.15). High-resolution electrospray mass spectra were recorded on a Waters/Micromass LCT spectrometer. Glutathione disulfide is available from commercial sources and/or can be made by oxidizing glutathione in the presence of a peroxide (e.g., hydrogen peroxide or the like).

In some embodiments, purification was conducted using high performance liquid chromatography implementing the following conditions: The sample was diluted in 25%ACN in MilliQ water, 0.1%TFA and separated by RP-HPLC. The column was a C18 Phenomenex 4µm, 150mm x 4.6mm. A gradient of Buffer A (99.9% MilliQ water, 0.1% TFA) and B (99.9% HPLC-grade CAN, 0.1% TFA) was run at 1mL/min (%B): 0-5 min, 5-15%; 5-31 min, 15-100%; 31-40 min, 100-5%. A 100µL sample loop and ~10 nmol sample load was used in some embodiments. Peaks were detected at 220nm using a Waters 2487 UV-spectrophotometer. In some embodiments, a semi-prep column can be used to enhance fraction collection, such as a Supelco C8 Supelcosil SPLC-8-D8 5 µm, 250mm x 10mm. The sample loop used was a 100 µL loop. In such embodiments, a gradient of Buffer A (99.9% MilliQ water, 0.1% TFA) and B (99.9% HPLC-grade CAN, 0.1% TFA) can be run at 3.8mL/min (%B): 0-11 min, 5-15%; 11-65 min, 15-95%; 65-73 min, 95%, 73-83 min, 95-5%. In yet other embodiments, a gradient of Buffer A (99.9% MilliQ water, 0.1% TFA) and B (99.9% HPLC-grade CAN, 0.1% TFA) can be run at 3.8mL/min (%B): 0-5 min, 5-50%; 5-40 min, 50-95%; 40-45 min, 95%, 45-50 min, 95-5%.

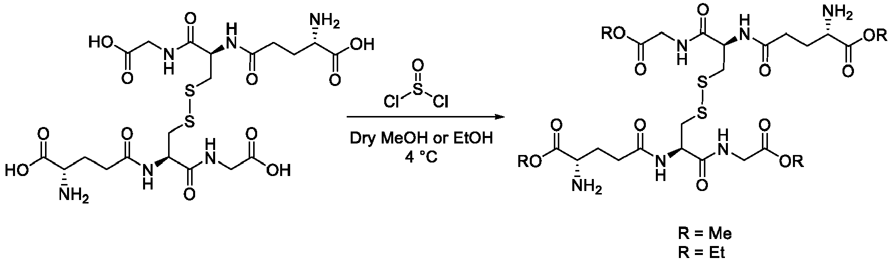

Glutathione disulfide (500 mg, 0.817 mmol) was dissolved in room temperature dry ethanol (100mL), cooled on ice, and thionyl chloride (5.0 mL, 68.8 mmol) was added drop-wise slowly from a syringe. The solution was swirled and allowed to react for 7 days at 4 °C. The reaction mixture was concentrated under reduced pressure to remove thionyl chloride to yield a whitish oily residue.

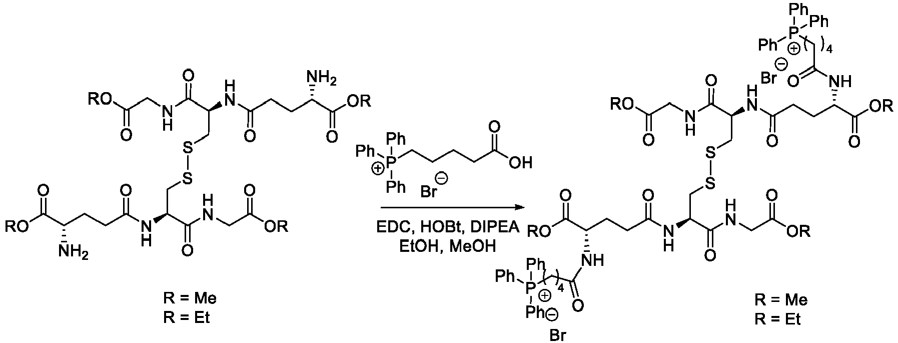

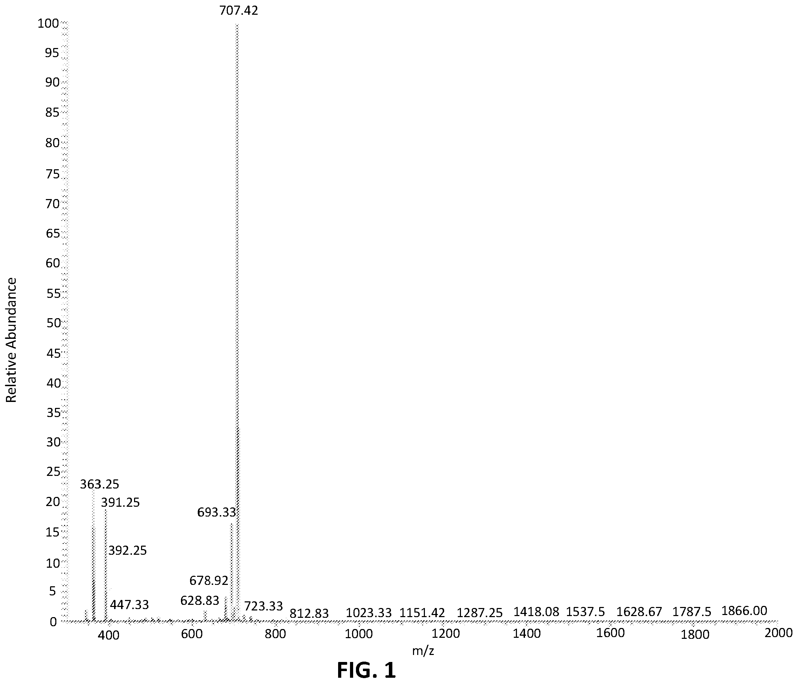

**Triphenylphosphonium glutathione disulfide tetraethyl ester.** A standard peptide coupling reaction such as HATU was used to link the amino groups of tetramethylGSSG with the lipophilic triphenylphosphonium (TPP) cation. The oily residue from the glutathione disulfide tetraethyl ester synthesis above (~ 591 mg, 0.816 mmol) was dissolved in dichloromethane (80 mL) and then carboxybutyl-triphenylphosphonium bromide (709 mg, 1.6 mmol), 1-[Bis(dimethylamino)methylene]-1H-1,2,3-triazolo[4,5-b]pyridinium 3-oxid hexafluorophosphate (HATU 608 mg, 1.6 mmol), and *N,N-*Diisopropylethylamine (600 µL, 3.4 mmol) were added. The reaction mixture was stirred at room temperature for 30 minutes. The organic layer was washed with saturated aqueous NaHCO₃ (120 mL), saturated aqueous NaBr (120 mL), dried over Na₂SO₄, and concentrated under reduced pressure to yield an oily crude residue. This was dissolved in a minimal volume of dichloromethane and then triturated with ethylacetate. The solvents were removed under reduced pressure to yield the final compound as a white powder (802 mg, minimum yield of 62.5% yield). FIG. 1 provides a mass spectrum for Et₄GSSG-TPP₂ (triphenylphosphonium tetraethyl glutathione disulfide). With reference to FIG. 1, the peak at m/z 707.42 is a doubly-charged peak that reflects an actual mass of 1415 (not including the mass of the counter ion).

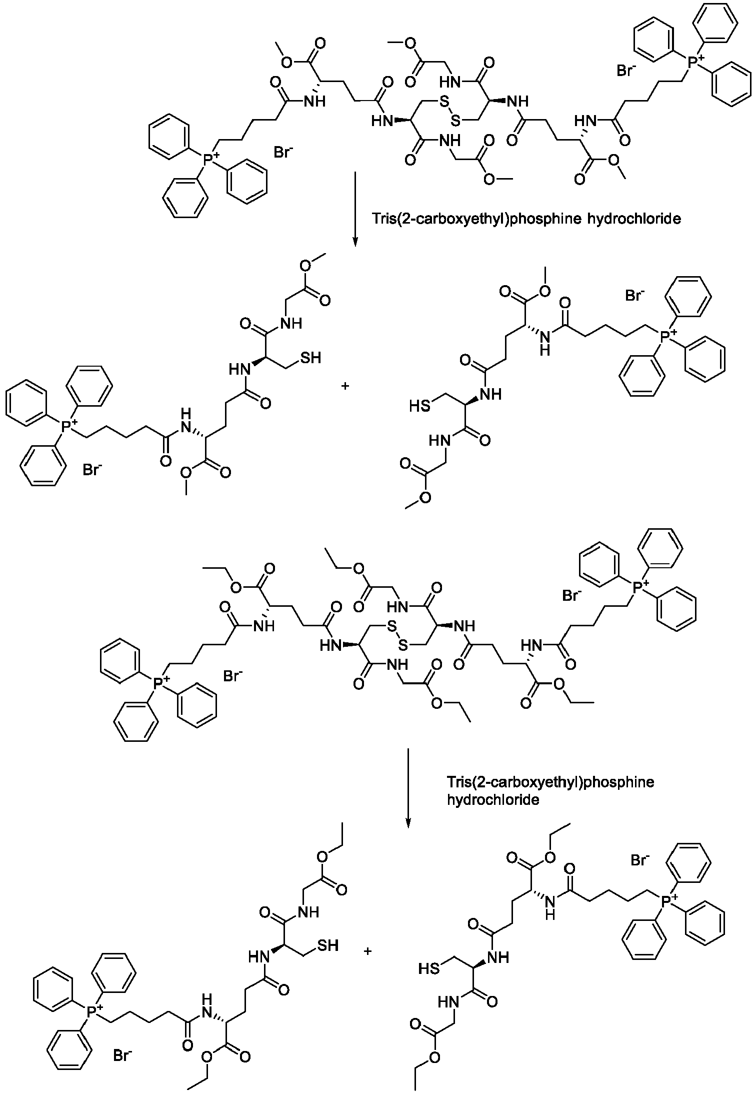

In some embodiments, the compounds described herein can be made using the procedures described below. 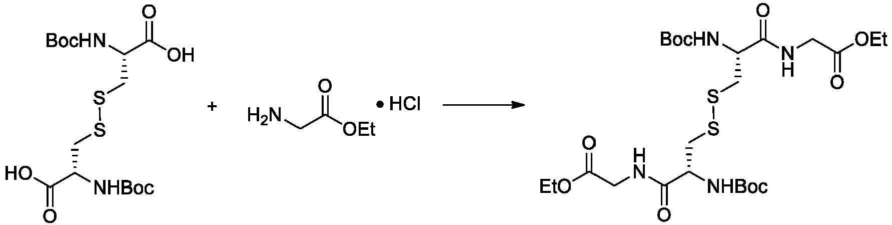 ***N,N*'-Di-Boc-L-cystinyldiglycine diethyl ester.** To a solution of *N,N*-di-Boc-L-cystine (1.0 g, 2.27 mmol), glycine ethyl ester hydrochloride (697 mg, 4.99 mmol), and HOBt (675 mg, 4.99 mmol) in DMF (25 mL) were added EDCI (957 mg, 4.99 mmol) and triethylamine (1.40 mL, 9.99 mmol) at 0 °C and the reaction mixture was stirred for 24 h at RT. DMF was removed using a high vacuum pump, and the oily crude was diluted with EtOAc (50 mL), and *aq.* sat. NaHCO₃ (50 mL). The aqueous layer further extracted with EtOAc (2 X 50 mL). The combined organic layer was washed the brine (100 mL), dried over Na₂SO₄, filtered, and concentrated under reduced pressure to give the white crude. Column chromatography (silica gel, EtOAc : Hex = 2 : 1) yielded the title compound (1.314 g, 94.5%); ¹H NMR (400 MHz, CDCl₃): δ 1.26 (t, *J=* 7.2 Hz, 6H), 2.92 (dd, *J* = 14.8, 10.8 Hz, 2H), 3.08 (dd, *J=* 14.8, 3.8 Hz, 2H), 3.86 (dd, *J=* 17.6, 5.2 Hz, 2H), 4.13 (d, *J=* 6.4 Hz, 2H), 4.18 (q, *J=* 7.2 Hz, 4H), 4.95 (m, 2H), 5.53 (d, *J=* 9.2 Hz, 2H); ¹³C NMR (175 MHz, CDCl₃): δ 14.3, 28.4, 41.2, 54.7, 61.5, 80.5, 156.0, 169.4, 171.0. 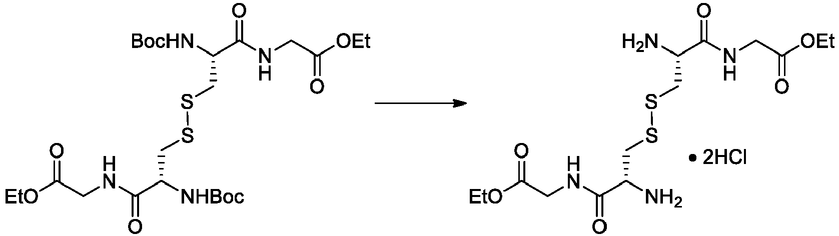 **L-Cystinyldiglycine diethyl ester dihydrochloride.** To a round bottom flask containing *N,N*'-di-Boc-L-cystinyldiglycine diethyl ester (1.2 g, 1.97 mmol), was added 4M HCl in dioxane (14 mL) at 0 °C and further stirring for 45 min. at RT. The solvent was removed using a rotavapor under the hood, and the crude was completely dried using a high vacuum pump to give the title compound (942 mg, 99%); ¹H NMR (400 MHz, MeOH-*d*₄): δ 1.28 (t, *J=* 7.2 Hz, 6H), 3.14 (dd, *J=* 14.4, 8.8 Hz, 2H), 3.52 (dd, *J=* 8.8, 4.8 Hz, 2H); ¹³C NMR (175 MHz, MeOH-*d*₄): δ 14.6, 39.0, 42.1, 52.6, 62.6, 169.2, 171.0. 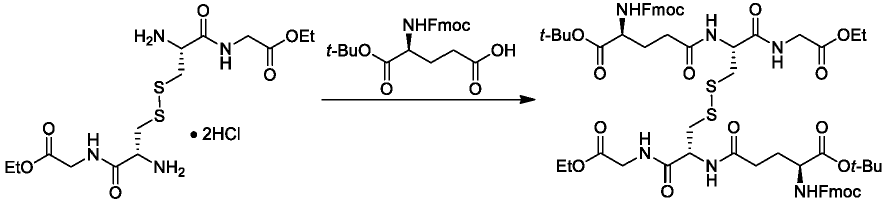 ***N,N*'-Di-Fmoc-L-glutathione disulfide di-*tert*-butyl ester diethyl ester.** To a solution of L-cystinyldiglycine diethyl ester dihydrochloride (700 mg, 1.45 mmol), and *N*-Fmoc-L-glutamic acid *α-tert-*butyl ester (1.417 g, 3.33 mmol) in DMF (25 mL), were added diisopropylamine (656 µL, 3.76 mmol), and HATU (1.431 g, 3.76 mmol) at RT. After stirred at RT for 6 h, an additional amount of HATU (275 mg, 0.124 mmol) and diisopropylamine (126 µL, 0.124 mmol) was added to the reaction mixture. After further stirring for 15 h at RT, DMF was removed using a high vacuum pump. The crude was diluted with CH₂Cl₂ (120 mL), and *aq.* sat. NaHCO₃ (120 mL), and aqueous layer further extracted with EtOAc (2 X 100 mL). The combined organic layer was washed the brine (150 mL), dried over Na₂SO₄, filtered, and concentrated under reduced pressure to give the crude. Fractions containing the desired product from 1^{st} column chromatography (silica gel, CH₂Cl₂ : MeOH = 15 : 1) were combined, and subjected to the 2nd column chromatography (silica gel, CH₂Cl₂ : EtOAc: MeOH = 20 : 2 : 1) to yield the title compound (1.669 g, 94.1%); ¹H NMR (400 MHz, CDCl₃): δ 1.22 (t, *J* = 7.2 Hz, 6H), 1.45 (s, 18H), 1.97-2.15 (m, 4H), 2.32-2.44 (m, 4H), 2.89-3.01 (m, 4H), 3.94 (d, *J=* 17.6 Hz, 2H), 4.10-4.24 (m, 8H), 4.33-4.43 (m, 6H), 5.53 (m, 2H), 6.41 (d, *J=* 8.4 Hz, 2H), 7.17 (d, *J=* 8.8 Hz, 2H), 7.27 (m, 2H), 7.38 (t, *J=* 7.6, 4H), 7.59 (d, *J=* 6.8 Hz, 4H), 7.74 (d, *J=* 7.6 Hz, 4H), 8.38 (s, 2H); ¹³C NMR (175 MHz, CDCl₃): δ 14.2, 28.2, 31.7, 41.8, 45.8, 47.3, 53.1, 54.6, 61.9, 67.3, 81.1, 120.1, 125.3, 127.1, 127.9, 141.4, 143.9, 144.1, 156.6, 169.9, 170.1, 172.4, 172.7. HRMS (ESI) *m*/*z* 1225.4807 (calcd for C₆₂H₇₆N₆O₁₆S₂ [M + H]⁺: 1225.4832). 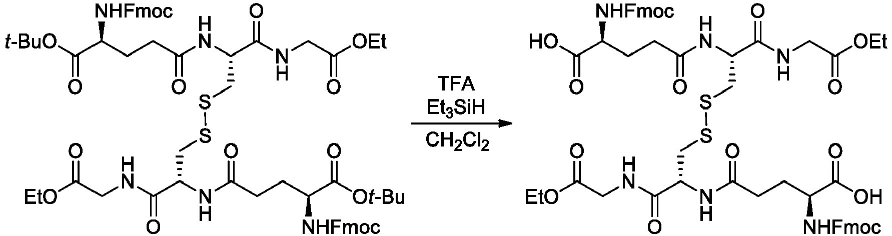 **N,N'-Di-Fmoc-L-glutathione disulfide diethyl ester.** To a solution of *N,N'*-di-Fmoc-L-glutathione disulfide di-*tert*-butyl ester diethyl ester (620 mg, 0.51 mmol) in CH₂Cl₂ (7.0 mL), were added TFA (7.0 mL) and triethylsilane (178 µL, 1.11 mmol) at 0 °C, and stirred for 3 h at RT. The solvent was removed using a rotavapor under the hood, and the crude was further dried with CH₂Cl₂. The white crude was suspended with EtOAc (10 mL), and filtered to give the title compound (544 mg, 97%); ¹H NMR (400 MHz, DMF-*d*₇): δ 1.19 (t, *J=* 7.2 Hz, 6H), 1.85-2.23 (m, 4H), 2.57 (t, *J=* 2.0 Hz, 4H), 3.10 (dd, *J=* 9.4, 14.0 Hz, 2H), 3.30 (dd, *J=* 4.4, 13.6 Hz, 2H), 3.90 (d, *J=* 5.2 Hz, 4H), 4.06-4.12 (m, 4H), 4.25-4.35 (m, 8H), 4.87 (m, 2H), 7.35 (t, *J=* 7.6 Hz, 4H), 7.44 (t, *J=* 7.2 Hz, 4H), 7.71 (d, *J=* 7.2, 2H), 7.77 (t, *J=* 8.4 Hz, 4H), 7.93 (d, *J=* 7.6 Hz, 4H), 8.39 (t, *J=* 6.0 Hz, 2H), 8.44 (d, *J=* 8.0 Hz, 2H); ¹³C NMR (175 MHz, DMF-*d*₇): δ 14.8, 28.7, 31.3, 41.7, 42.2, 48.1, 53.5, 55.9, 61.7, 67.6, 121.2, 126.6, 128.3, 128.8, 142.2, 145.2, 145.4, 157.8, 170.7, 171.7, 173.2, 175.2. HRMS (ESI) *m*/*z* 1113.3583 (calcd for C₅₄H₆₀N₆O₁₆S₂ [M + H]⁺: 1113.3580). 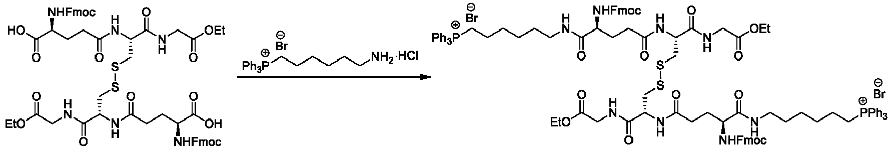 **Bis-triphenylphosphoniumhexylamide-*N,N'*-di-Fmoc-L-glutathione disulfide diethyl ester dibromide.** To a solution of *N,N*'-di-Fmoc-L-glutathione disulfide diethyl ester (200 mg, 0.18 mmol) in DMF (7.0 mL), were added 6-(triphenylphosphonium)hexan-1-amine chloride hydrochloride¹ (203 mg, 0.47 mmol) in DMF (7 mL), diisopropylethylamine (81 µL, 0.47 mmol), and HATU (178 mg, 0.47 mmol) at RT, and further stirred for 6 h at RT. The solvent was removed using a high vacuum pump, and the crude was dissolved in CH₂Cl₂ (150 mL). The organic layer was washed with sat. *aq.* NaHCO₃ (150 mL), 20% *aq.* NaBr, dried over NaSO₄, filtered, and concentrated under reduced pressure to give the crude. The oily crude was dissolved in the minimum amount of CH₂Cl₂, and triturated with EtOAc, and the white precipitate was filtered to give the title compound (261 mg, 74%); ¹H NMR (400 MHz, DMF-*d*₇): δ 1.17 (t, *J=* 7.2 Hz, 6H), 1.33-1.47 (m, 8H), 1.52-1.59 (m, 4H), 1.65-1.75 (m, 4H), 1.97-2.17 (m, 4H), 2.36 (t, *J=* 7.2 Hz, 4H), 3.06-3.16 (m, 6H), 3.30 (dd, *J=* 13.6, 4.4 Hz, 2H), 3.63-3.71 (m, 4H), 3.96 (d, *J=* 5.6 Hz, 4H), 4.08 (q, *J=* 7.2 Hz, 4H), 4.22-4.32 (m, 8H), 4.83-4.87 (m, 2H), 7.34 (t, *J=* 7.6 Hz, 4H), 7.44 (t, *J=* 7.2 Hz, 4H), 7.73-7.85 (m, 20H), 7.92-7.98 (m, 20H), 8.42 (t, *J=* 5.6 Hz, 2H), 8.47 (d, *J=* 8.0 Hz, 2H); ¹³C NMR (175 MHz, DMF-*d*₇): δ 14.8, 21.9 (d, *J=* 50.8 Hz, -CH₂-P), 23.2, 27.0, 29.4, 33.1, 39.8, 41.9, 42.2, 48.1, 53.5 (d, *J=* 8.8 Hz, -CH₂-CH₂-P), 56.2 (d, *J=* 10.5 Hz, -CH₂-CH₂-CH₂-P), 61.7, 67.6, 120.0 (d, *J=* 84.0 Hz, Ph₃P *ipso* C), 121.2, 126.5 (d, *J=* 10.5 Hz, Ph₃P *ortho*/*meta*), 128.3, 128.8, 131.4 (d, *J=* 12.3 Hz, Ph₃P *ortho*/*meta*), 134.9 (d, *J=* 10.5 Hz, Ph₃P *para),* 136.1, 142.2, 145.2 (d, *J* = 42.0 Hz), 157.7, 170.7, 171.8, 173.0, 173.3. HRMS (ESI) *m*/*z* 900.3662 (calcd for C₁₀₂H₁₁₄N₈ O₁₄ P₂ S₂ [M]²⁺: 900.3680). 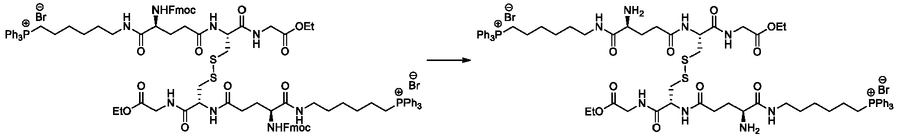 **Bis-triphenylphosphoniumhexylamide-L-glutathione disulfide diethyl ester dibromide.** To a solution of bis-triphenylphosphoniumhexylamide-*N,N'*-di-Fmoc-L-glutathione disulfide diethyl ester dibromide (100 mg, 50.97 µmol) in DMF (4.5 mL), was added piperidine (0.5 ml), and stirred for 15 min at RT. The solvent was removed using a high vacuum pump, and the crude was washed with Et₂O. The crude was dissolved in the minimum amount of MeOH, and triturated with Et₂O, and the white precipitate was filtered to give the title compound (53 mg, 77%); ¹H NMR (400 MHz, MeOH-*d*₄): δ 1.24 (t, *J=* 7.2 Hz, 6H), 1.33-1.39 (m, 4H), 1.42-1.47 (m, 4H), 1.53-1.60 (m, 4H), 1.63-1.70 (m, 4H), 1.84-2.01 (m, 4H), 2.28 (t, *J* = 7.6 Hz, 4H), 2.99 (dd, *J=* 14.0, 9.2 Hz, 2H), 3.13 (t, *J=* 6.8 Hz, 4H), 3.23 (dd, *J=* 14.0, 4.8 Hz, 2H), 3.37-3.45 (m, 4H), 3.94 (d, *J=* 4.8 Hz, 4H), 4.14 (q, *J=* 7.2 Hz, 4H), 7.73-7.91 (m, 30H); ¹³C NMR (175 MHz, MeOH-*d*₄): δ 14.5, 22.5 (d, *J* = 50.75 Hz, -CH₂-P), 23.4, 27.1, 30.1, 31.1 (d, *J* = 15.75 Hz, -CH₂-CH₂-P), 32.2, 33.2, 40.1, 42.0, 42.2, 53.7, 54.0, 55.5, 62.4, 119.9 (d, *J* = 85.8 Hz, Ph₃P *ipso* C), 131.5 (d, *J=* 12.5 Hz, Ph₃P *ortho*/*meta*), 134.8 (d, *J* = 8.8 Hz, Ph₃P *ortho*/*meta),* 136.3, 171.7, 172.8, 175.2, 176.9. HRMS (ESI) *m*/*z* 678.2978 (calcd for C₇₂H₉₄N₈ O₁₀ P₂ S₂ [M]²⁺: 678. 2999).

### Effect of mitoGSH on drug-induced damage to hepatocytes

Hepatocytes isolated from an old rat have 60% less mitochondrial GSH than that seen in young adult animals. In this example, menadione was used as a redox cycler, to stress mitochondria in hepatocytes, as GSH-dependent mechanisms can be involved its detoxification. Aged hepatocytes are markedly more susceptible to menadione compared to young animals (LC50 young = ~500 µM; LC50 old = 300 µM). The increased vulnerability to menadione has been linked to the loss of GSH resulting from the decline in GSH synthesis capacity.

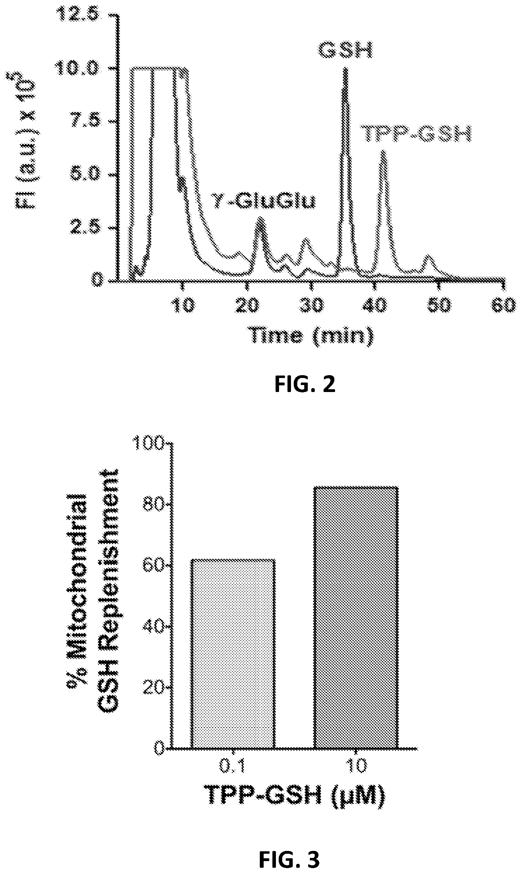

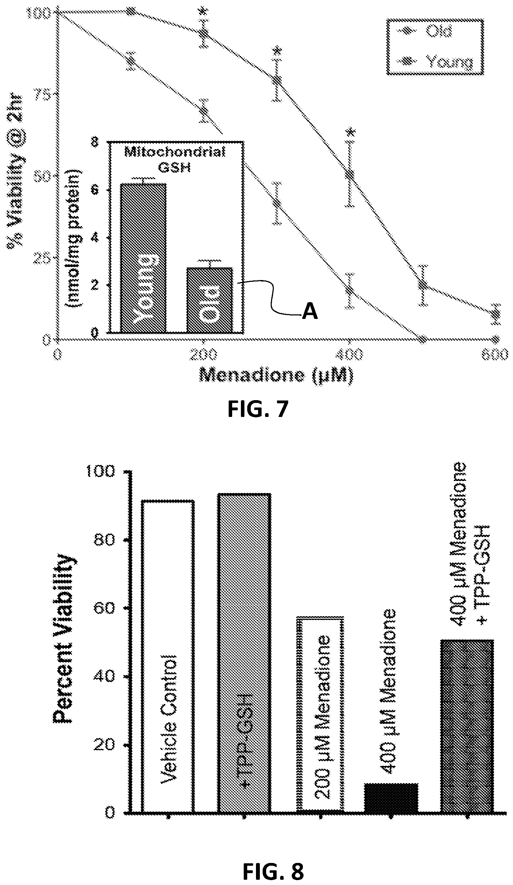

After cell isolation, hepatocytes were treated with increasing concentrations of TPP-GSH for 30 minutes prior to the addition of 300 µM Menadione (see FIGS. 2 and 3, which show that TPP-GSH replenishes GSH depleted mitochondria within 30 minutes). Menadione treatment alone served as the positive control. Viability in untreated hepatocytes also was measured over two hours. The 1 µM TPP pretreatment was not protective, while 10 and 100 µM TPP-GSH lowered the susceptibility of hepatocytes from aged rats to menadione. Triphenylphosphonium alone did not affect viability. FIGS. 7 and 8 provide results establishing that hepatocytes from old rats are nearly twice as susceptible to menadione as cells from young animals (LCso = 232 µM in old vs 401 µM in young; n=6, p<0.05). This higher vulnerability is coincident with an age-related loss of mitochondrial GSH (FIG. 7, "A"). But pretreating cells with 50 µM TPP-GSH 30 minutes prior to menadione protects against hepatotoxicity (FIG. 8). The LCso after TPP-GSH treatment is no different than *untreated* cells from young rats (n=1).

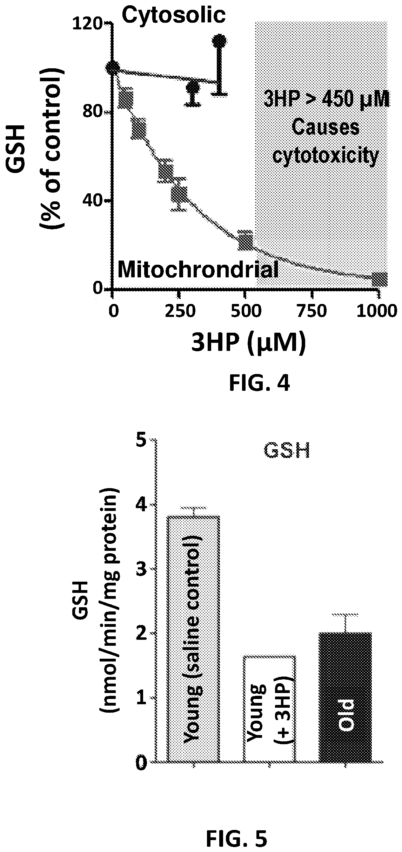

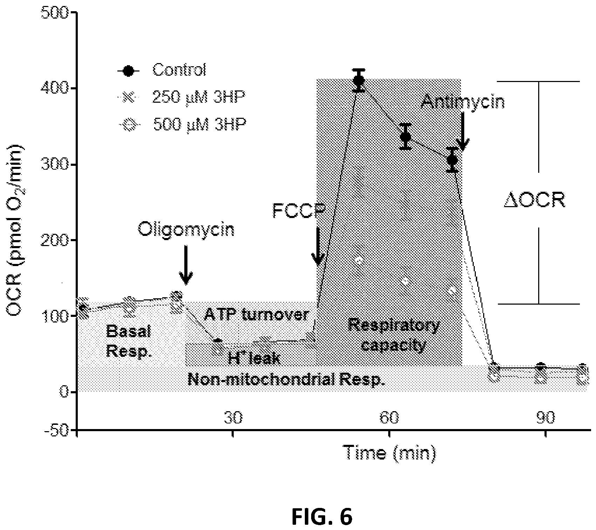

In separate experiments, isolated mitochondria were neither uncoupled nor was electron transport inhibited by treatment with TPP-GSH. The respiratory control ratio (RCR) for 8-month-old male Fischer 344 rat kidney mitochondria was not significantly different between vehicle controls (avg 2.6), 10 µM TPP-GSH treatment (2.8), and 100 µM TPP-GSH treatment (2.3). The information provided by FIG. 4 demonstrates the impact of 3HP on mitochondrial (represented with "■") and cytosolic (represented with "●") GSH. FIG. 5 shows the impact of 3HP on mitochondrial GSH levels in young rat liver vs. mitochondrial GSH in young and old rat liver. Additionally, FIG. 6 illustrates that 3HP-dependent GSH loss does not alter (1) basal respiration, (2) ATP turnover, (3) proton leak, or (4) non-mitochondrial respiration; however, respiratory capacity is increasingly lost with higher levels of 3HP treatment indicating that through the loss of mitochondrial GSH, cells can no longer respond to increasing energy demands.

### Effect of mitoGSH in animals

Three seven-month-old female non-transgenic mice (approximately 30g each) were injected i.p. with 3.8 mg/kg TPP-GSSG in 100 µL/mouse. TPP-GSSG was initially solubilized in DMF and later diluted in millipure water to 5.3% (v/v) dimethylformamide for injection. All mice remained healthy and active for 48 hours with no detrimental effects observed. All mice gained weight (0.1g - 2.3g) over the 48 hour period. Blood was collected via brachial cut, spun down to collect plasma and then delivered to OSU Veterinary Diagnostic Laboratory for a small animal liver chemistry profile. No liver damage or abnormalities were detected.

## Claims

1. A compound having a structure that satisfies any of Formulas 2A-2D or 5A or 5B 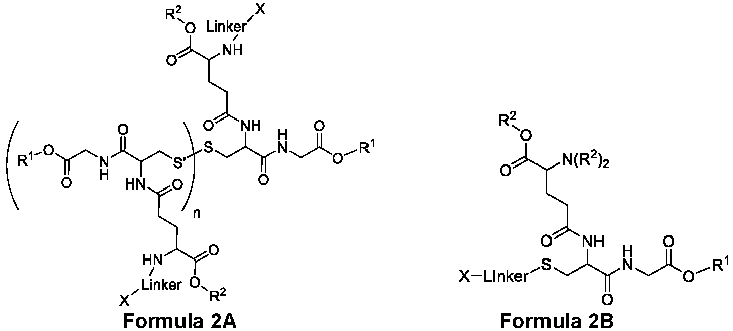 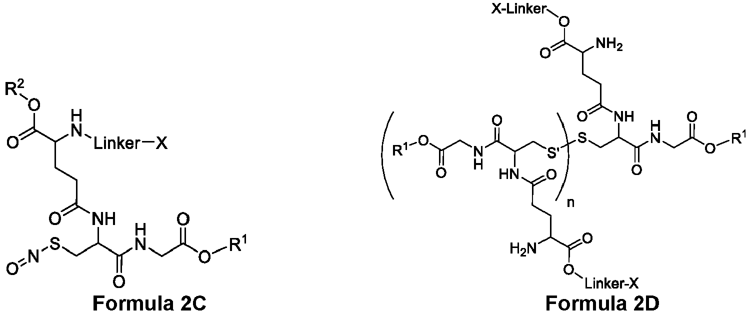 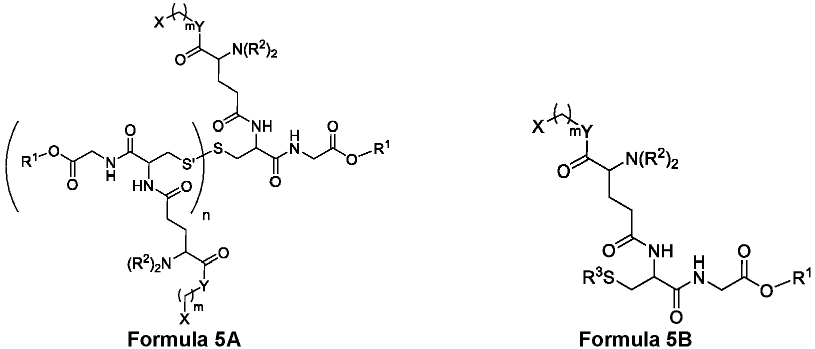 wherein
the compound comprises a pharmaceutically acceptable, negatively charged counter ion;
n is 0 or 1 and when n is 0, the S atom is bound to a hydrogen atom, a nitroso group to provide a thio-nitroso group, an aliphatic group to provide a thioether-containing compound, or a ketone group to provide a thioester-containing compound;
each R¹ independently is selected from aliphatic, aryl, heteroaliphatic, aliphatic-aryl, heteroaryl, aliphatic-heteroaryl, heteroaliphatic-aryl, or heteroaliphatic-heteroaryl;
each R² independently is selected from hydrogen or aliphatic, aryl, heteroaliphatic, aliphatic-aryl, heteroaryl, aliphatic-heteroaryl, heteroaliphatic-aryl, or heteroaliphatic-heteroaryl;
R³ is selected from hydrogen, a protecting group, a nitroso group, or a linker-X group, wherein the protecting group is selected from aliphatic, aryl, heteroaliphatic, aliphatic-aryl, heteroaryl, aliphatic-heteroaryl, heteroaliphatic-aryl, or heteroaliphatic-heteroaryl;
each linker-X group is selected from -C(O)R^{c}X, -[(CH₂)₂O]ₘX, -C(=NH₂⁺)NR^{c}X, -CH₂C(O)NHR^{c}X, -SR^{c}X, or 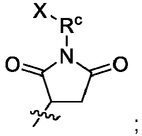
wherein each R^{c} independently is selected from aliphatic, aryl, heteroaliphatic, aliphatic-aryl, heteroaryl, aliphatic-heteroaryl, heteroaliphatic-aryl, or heteroaliphatic-heteroaryl; each X independently is selected from -P⁺(R^{d})₃, wherein each R^{d} independently can be selected from hydrogen, aliphatic, aryl, or aliphatic-aryl; Y is oxygen or -NH; and each of m independently is 1 to 30; optionally wherein
(i) each R¹ and R² independently is methyl or ethyl;
(ii) each linker independently is selected from -C(O)R^{c}-, -[(CH₂)₂O]ₘ-, -C(=NH₂⁺)NR^{c}-, -CH₂C(O)NHR^{c}-, -SR^{c}-, wherein each R^{c} independently is selected from aliphatic, aryl, heteroaliphatic, aliphatic-aryl, heteroaryl, aliphatic-heteroaryl, heteroaliphatic-aryl, or heteroaliphatic-heteroaryl; and m is 1 to 30; or
(iii) X is selected from -P⁺(Ph)₃.

2. The compound of claim 1, wherein the pharmaceutically acceptable, negatively charged counter ion is a mesylate.

3. The compound of claim 1, wherein the compound satisfies any one of Formulas 3B, 3A, 4A, or 4B

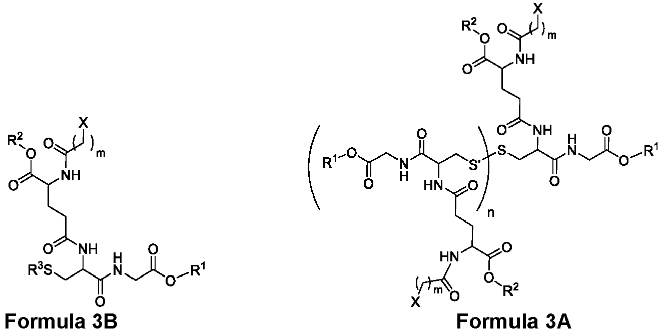

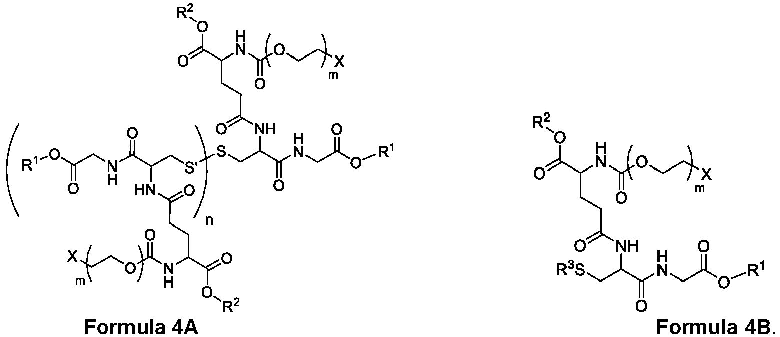

4. The compound of claim 1, wherein the compound is selected from

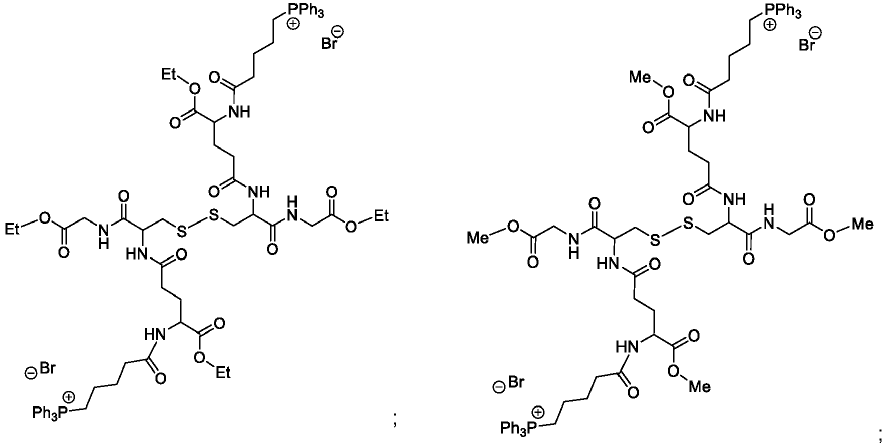

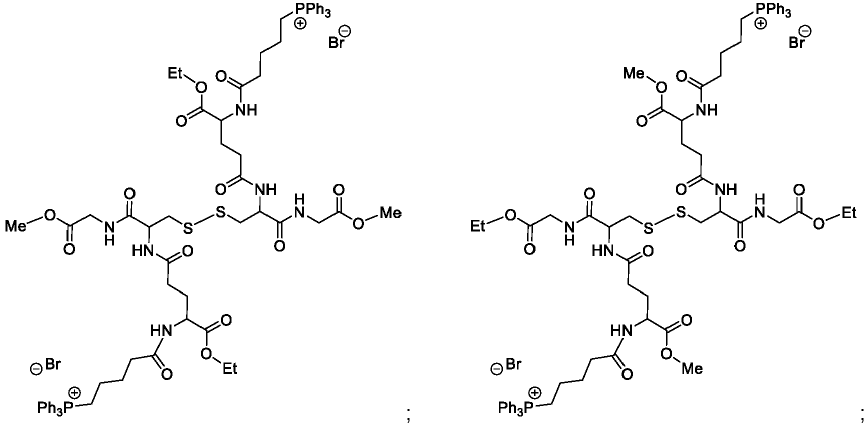

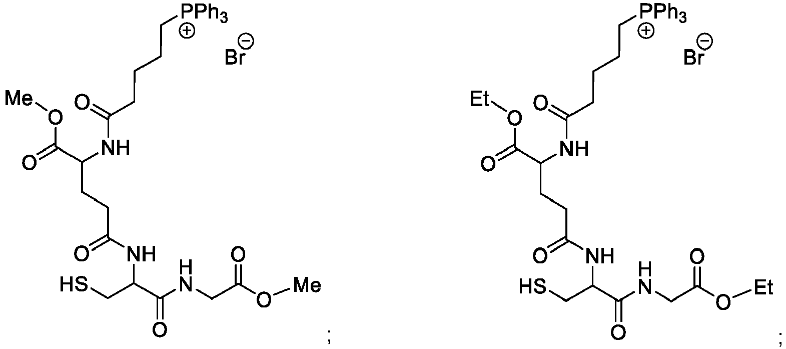

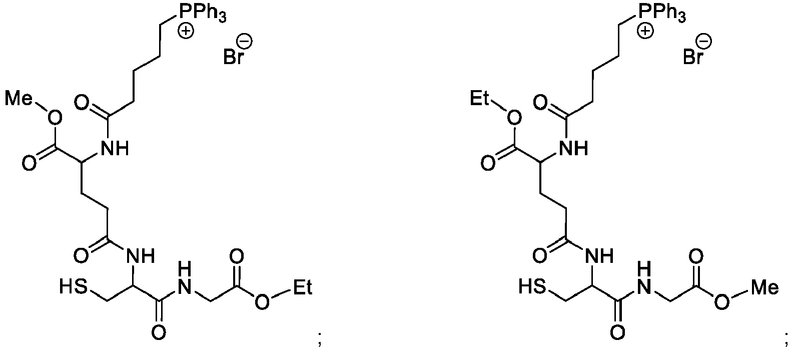

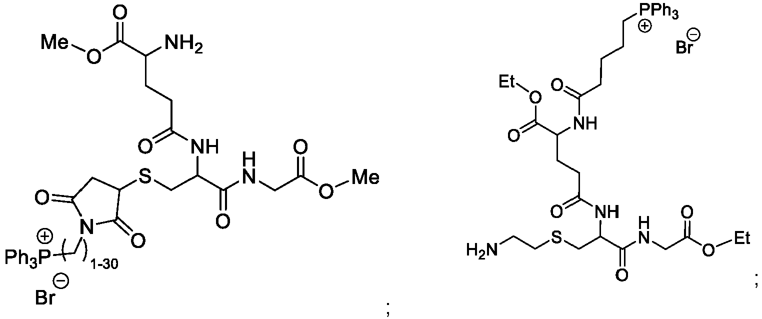

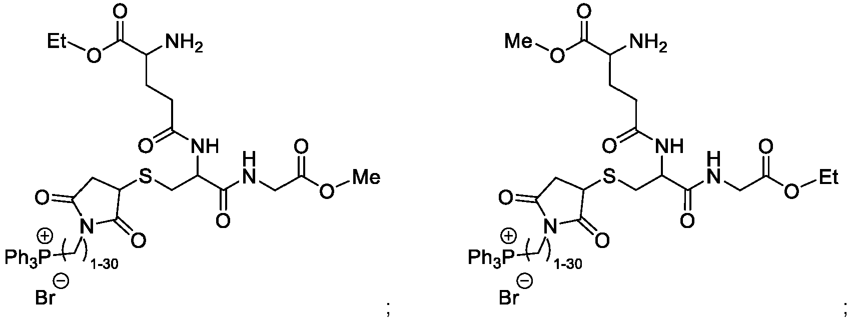

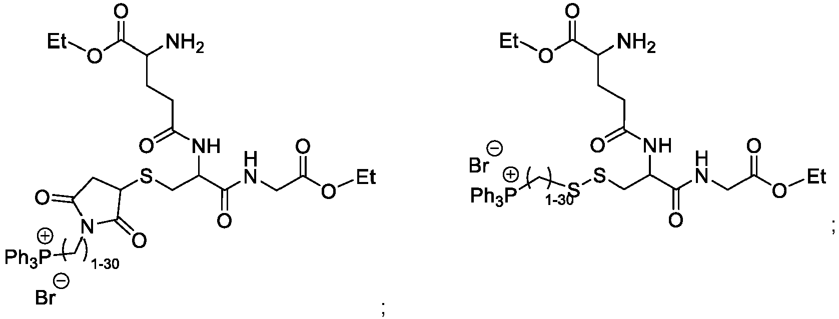

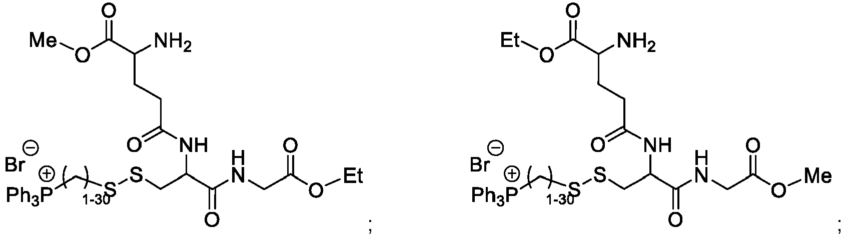

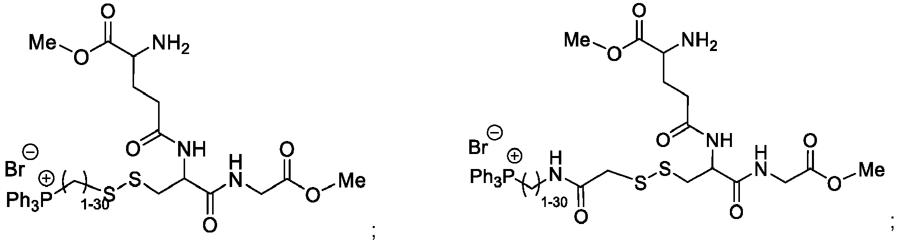

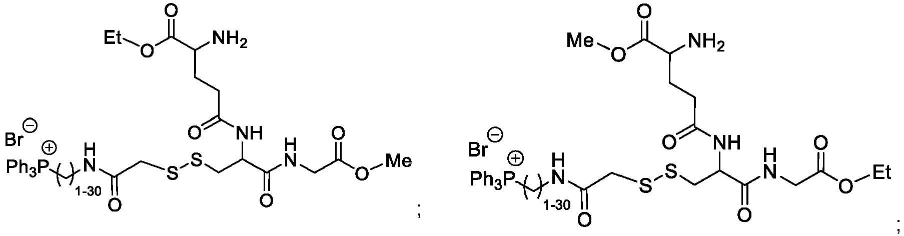

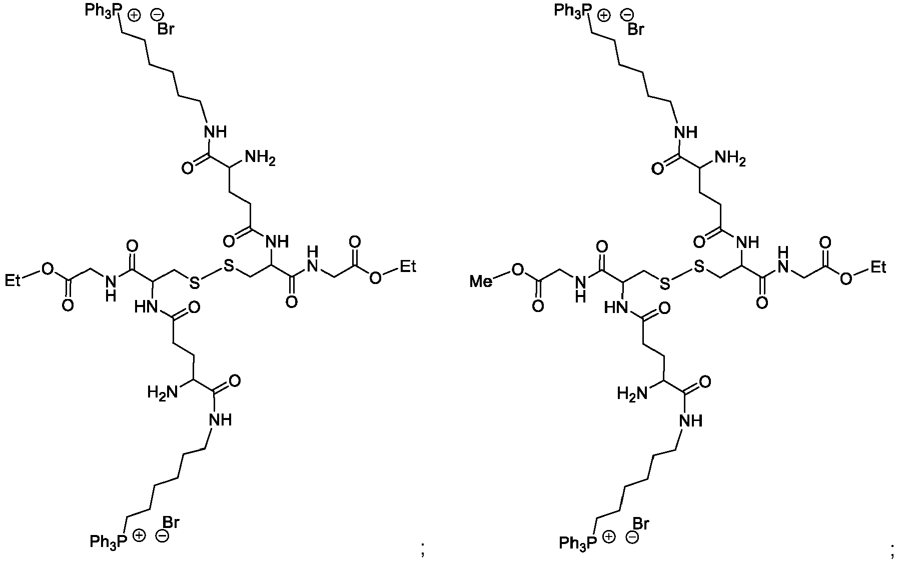

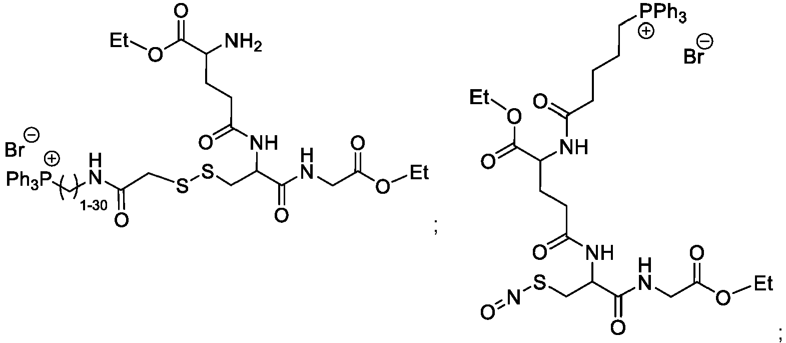

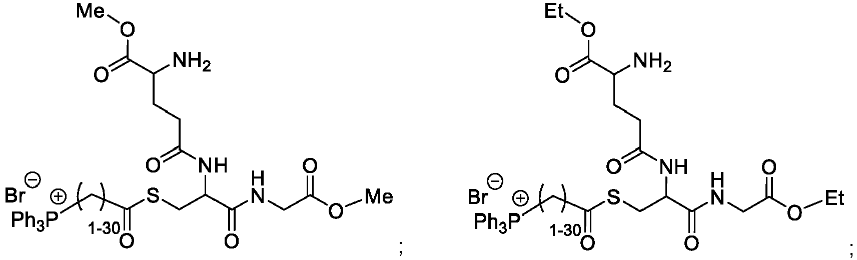

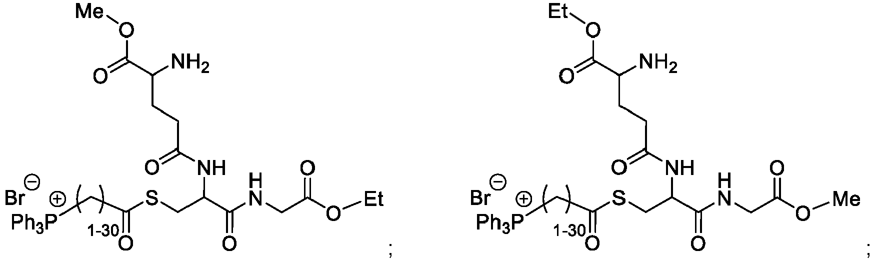

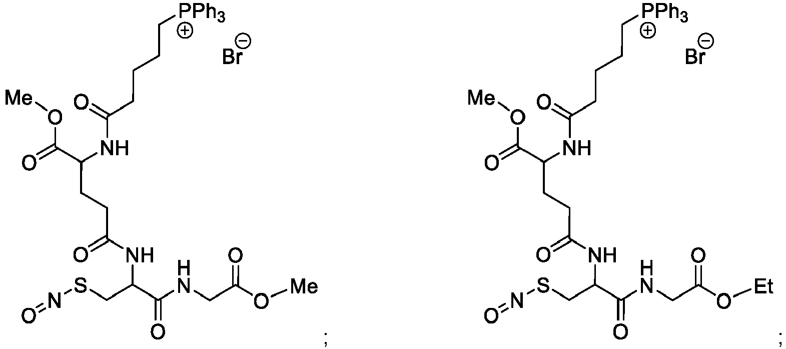

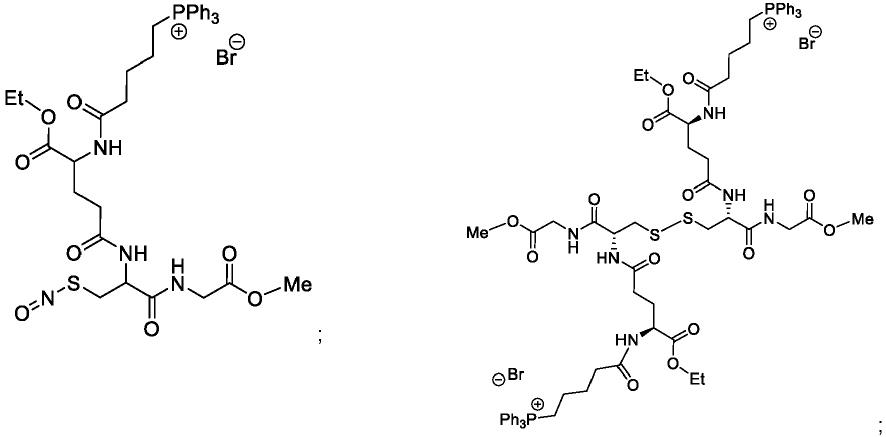

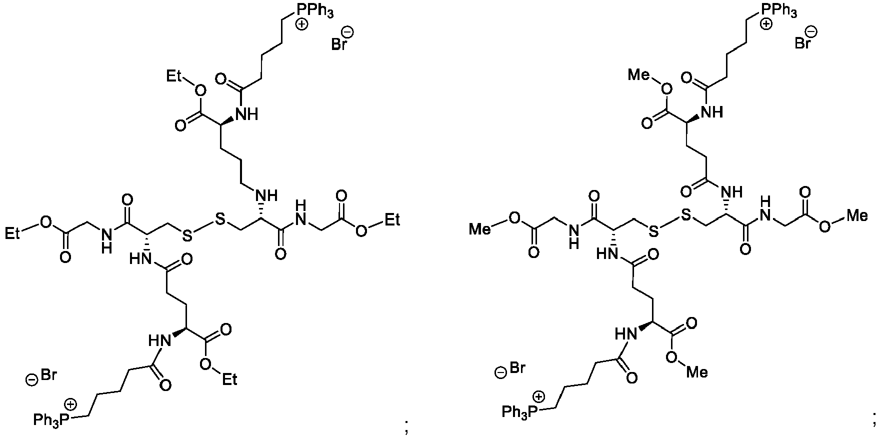

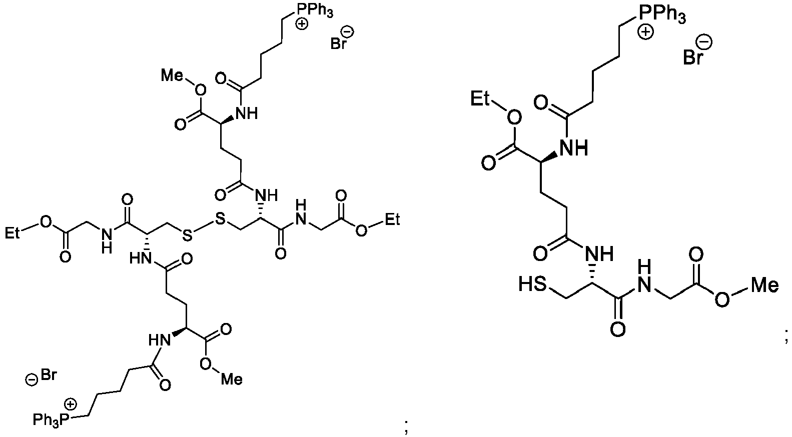

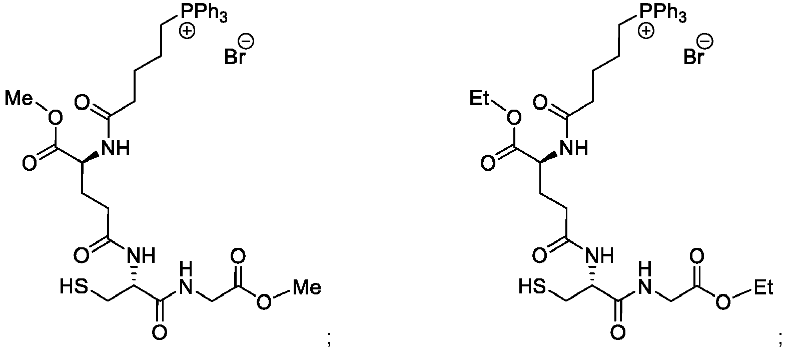

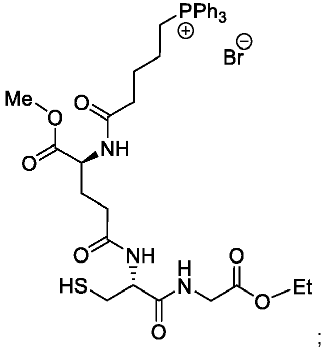

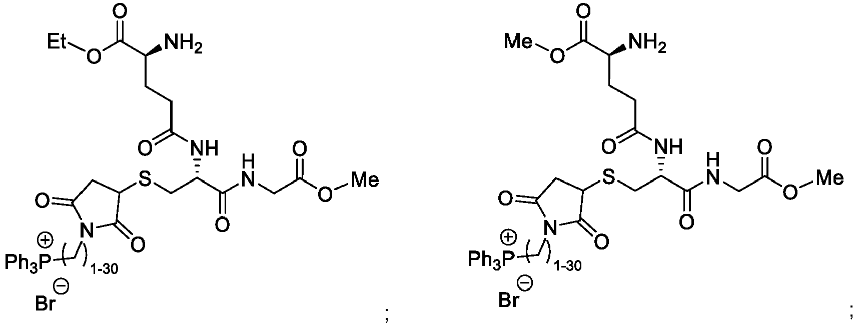

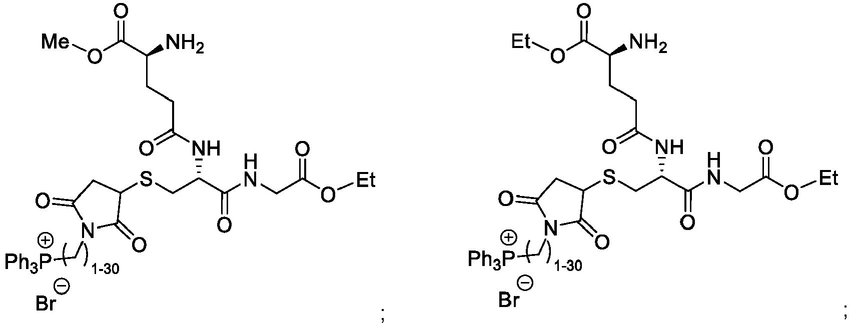

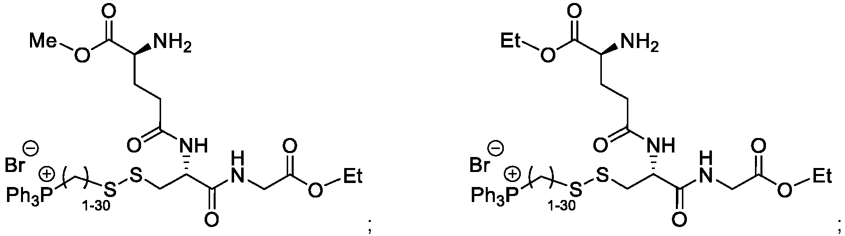

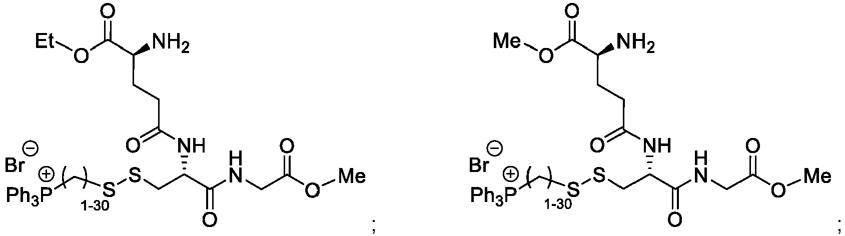

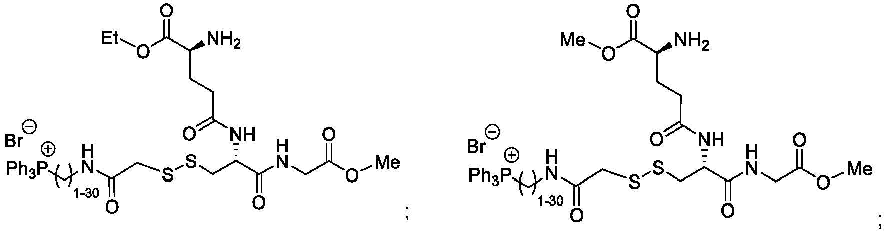

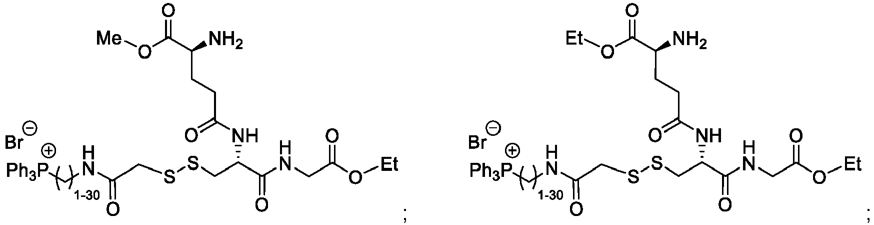

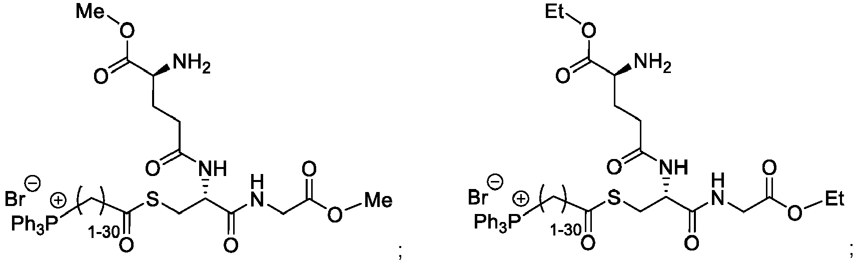

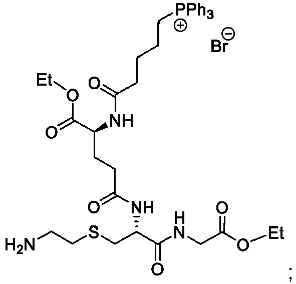

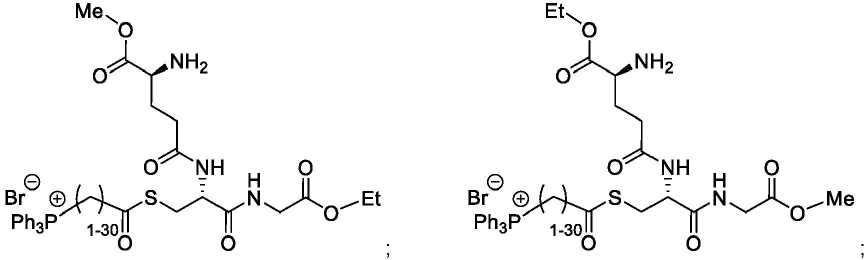

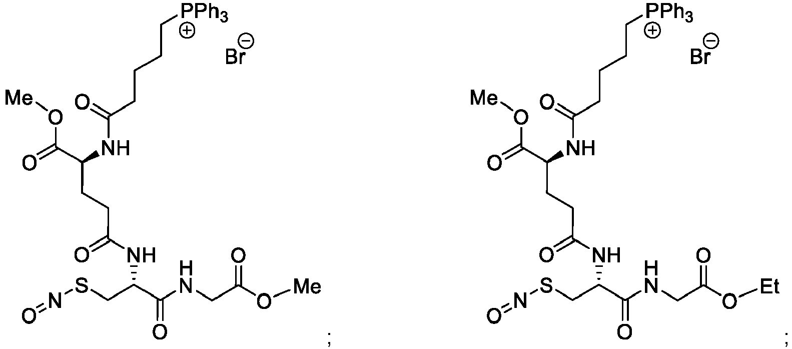

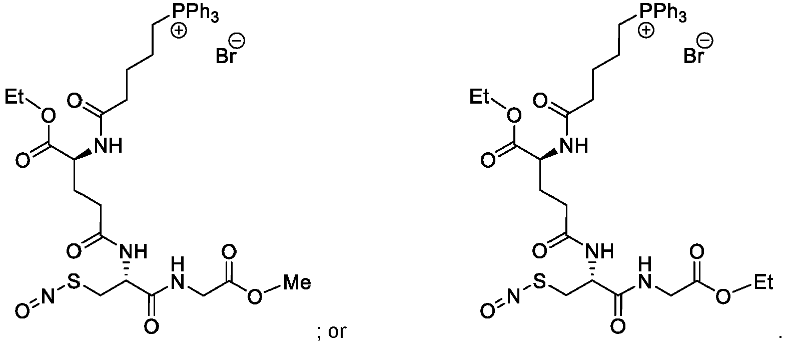

5. A pharmaceutical composition, comprising:
a compound according to any one of claims 1 to 4; and
a pharmaceutically acceptable carrier.

6. The pharmaceutical composition of claim 5, wherein the composition further comprises one or more additional therapeutic agents,
optionally wherein the one or more additional therapeutic agents is selected from N-acetyl cysteine, vitamin E, vitamin C, and diacetyl-bis(4-methylthiosemicarbazonato)copperll.

7. A compound as claimed in any one of claims 1 to 4 or a composition of claim 5 or 6 for use in a method of treatment of a human or animal.

8. A compound as claimed in any one of claims 1 to 4 or a pharmaceutical composition as claimed in claim 5 or 6 for use
(i) to treat, ameliorate, and/or prevent a disease or condition associated with low or reduced glutathione levels, optionally caused by low mitochondrial glutathione levels; or
(ii) to prevent or mitigate attenuation of electron transport chain (ETC) activity, allosteric inhibition of dicarboxylate transport, uncoupling of a GSH/GSSG redox network from a NADH/NAD⁺ redox couple, mitochondria susceptibility to oxidants emanating from an ETC, enhanced susceptibility to drugs and toxins, initiating permeability pore opening and release of pro-apoptotic factors, or a combination thereof; or
(iii) to treat, ameliorate, or prevent neurodegeneration, stroke, diabetes, cardiovascular disease, cancer, muscle injuries, muscle myopathies, viral infections, periodontal disease, vascular impairment, kidney disease, ischemia-reperfusion injury, wound repair issues, inflammation, schizophrenia and other neuro-psychological disorders, acetaminophen toxicity, acute alcohol toxicity, chronic alcohol toxicity, or a combination thereof.

9. A method of making a compound of claim 1, comprising:
exposing glutathione disulfide to thionyl chloride and an alcohol selected from methanol or ethanol to form a protected glutathione disulfide;
exposing the protected glutathione disulfide to (4-carboxybutyl)triphenylphosphonium bromide, N-(3-dimethylaminopropyl)-N'-ethylcarbodiimide·HCl, hydroxybenzotriazole, di-isopropylethyl amine, and an alcohol solvent to form a compound having a structure 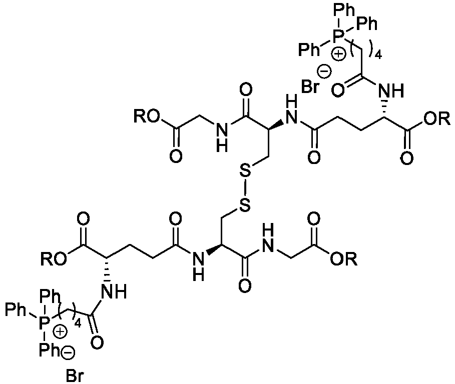
wherein R is methyl or ethyl.

## Patentansprüche

1. Verbindung mit einer Struktur, die jegliche der Formeln 2A-2D oder 5A oder 5B erfüllt 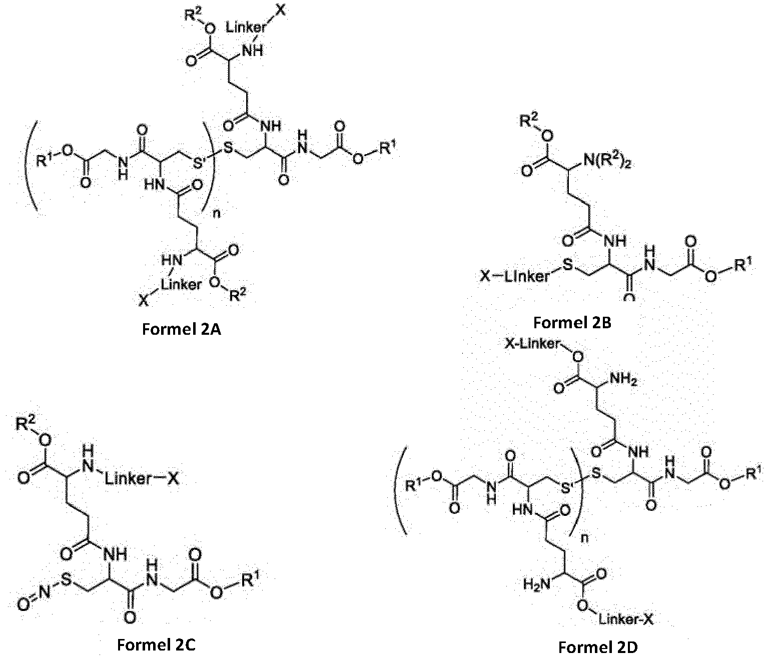 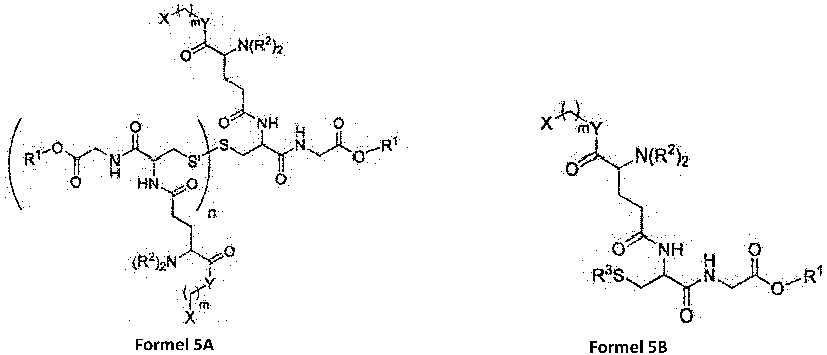 wobei
die Verbindung ein pharmazeutisch annehmbares, negativ geladenes Gegenion umfasst;
n 0 oder 1 ist und wenn n 0 ist, das S-Atom an ein Wasserstoffatom, eine Nitrosogruppe, um eine Thio-Nitrosogruppe bereitzustellen, eine aliphatische Gruppe, um eine Thioether-haltige Verbindung bereitzustellen, oder eine Ketongruppe, um eine Thioesterhaltige Verbindung bereitzustellen, gebunden ist;
jedes R¹ unabhängig ausgewählt ist aus aliphatisch, Aryl, heteroaliphatisch, aliphatischem Aryl, Heteroaryl, aliphatischem Heteroaryl, heteroaliphatischem Aryl oder heteroaliphatischem Heteroaryl;
jedes R² unabhängig ausgewählt ist aus Wasserstoff oder aliphatisch, Aryl, heteroaliphatisch, aliphatischem Aryl, Heteroaryl, aliphatischem Heteroaryl, heteroaliphatischem Aryl oder heteroaliphatischem Heteroaryl;
R³ ausgewählt ist aus Wasserstoff, einer Schutzgruppe, einer Nitrosogruppe oder einer Linker-X-Gruppe, wobei die Schutzgruppe ausgewählt ist aus aliphatisch, Aryl, heteroaliphatisch, aliphatischem Aryl, Heteroaryl, aliphatischem Heteroaryl, heteroaliphatischem Aryl oder heteroaliphatischem Heteroaryl;
jede Linker-X-Gruppe ausgewählt ist aus -C(O)R^{c}X, -[(CH₂)₂O]ₘX, -C(=NH₂⁺)NR^{c}X, -CH₂C(O)NHR^{c}X, -SR^{c}X oder 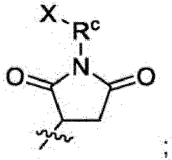
wobei jedes R^{c} unabhängig ausgewählt ist aus aliphatisch, Aryl, heteroaliphatisch, aliphatischem Aryl, Heteroaryl, aliphatischem Heteroaryl, heteroaliphatischem Aryl oder heteroaliphatischem Heteroaryl; jedes X unabhängig ausgewählt ist aus -P⁺(R^{d})₃, wobei jedes R^{d} unabhängig ausgewählt sein kann aus Wasserstoff, aliphatisch, Aryl oder aliphatischem Aryl; Y Sauerstoff oder -NH ist; und jedes aus m unabhängig 1 bis 30 ist; wobei optional
(i) jedes R¹ und R² unabhängig Methyl oder Ethyl ist;
(ii) jeder Linker unabhängig ausgewählt ist aus -C(O)R^{c}-, -[(CH₂)₂O]ₘ-, - C(=NH₂⁺)NR^{c}-, -CH₂C(O)NHR^{c}-, -SR^{c}-, wobei jedes R^{c} unabhängig ausgewählt ist aus aliphatisch, Aryl, heteroaliphatisch, aliphatischem Aryl, Heteroaryl, aliphatischem Heteroaryl, heteroaliphatischem Aryl oder heteroaliphatischem Heteroaryl; und m 1 bis 30 ist; oder
(iii) X ausgewählt ist aus -P⁺(Ph)₃.

2. Verbindung nach Anspruch 1, wobei das pharmazeutisch annehmbare, negativ aufgeladene Gegenion ein Mesylat ist.

3. Verbindung nach Anspruch 1, wobei die Verbindung jegliche der Formeln 3B, 3A, 4A oder 4B erfüllt

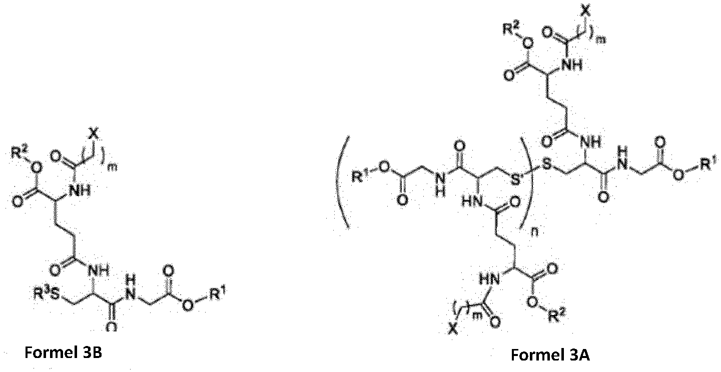

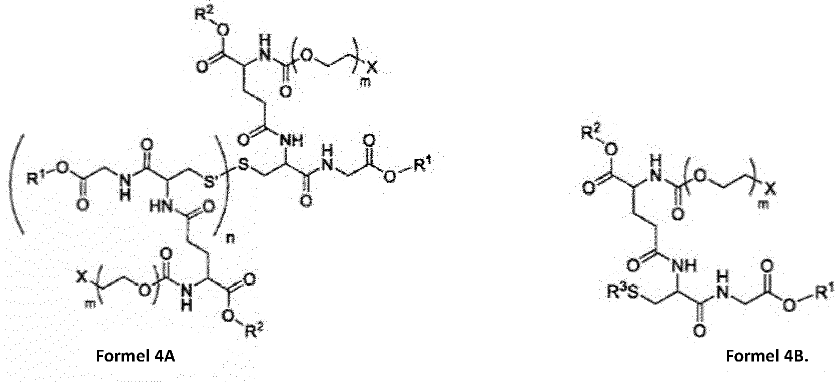

4. Verbindung nach Anspruch 1, wobei die Verbindung ausgewählt ist aus

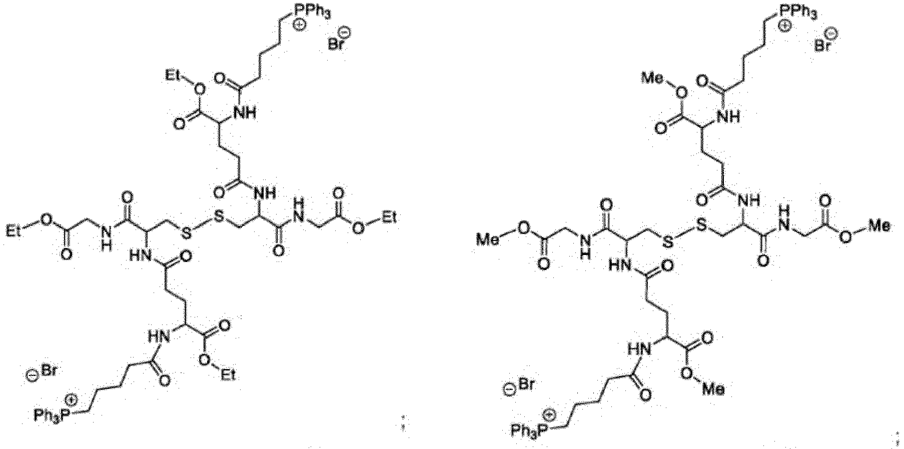

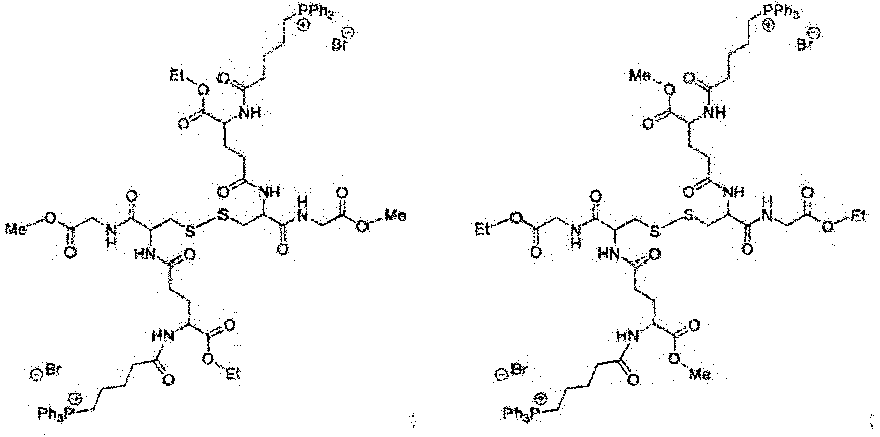

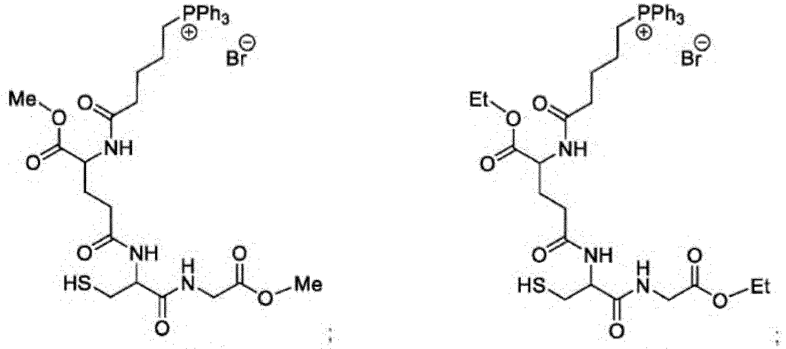

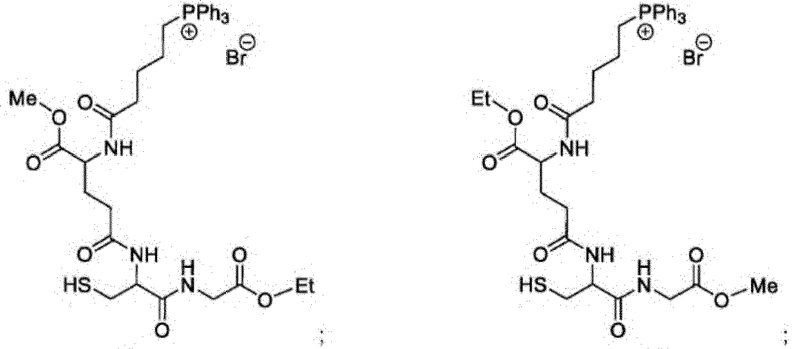

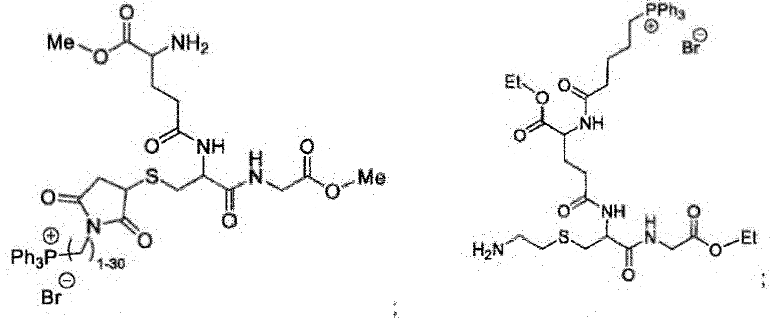

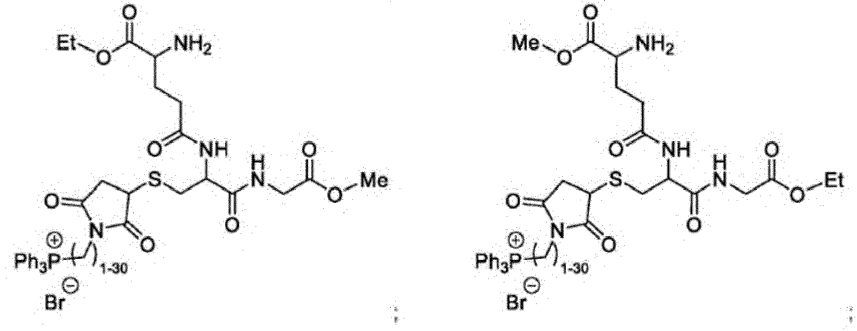

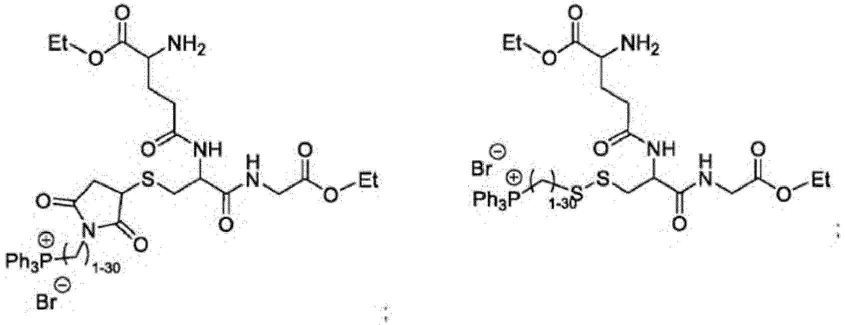

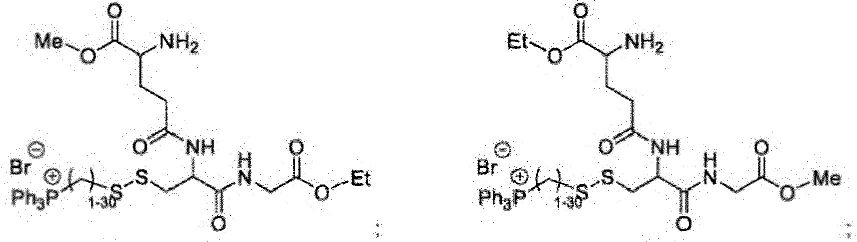

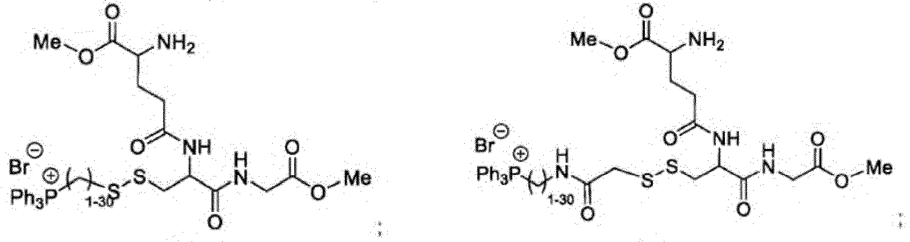

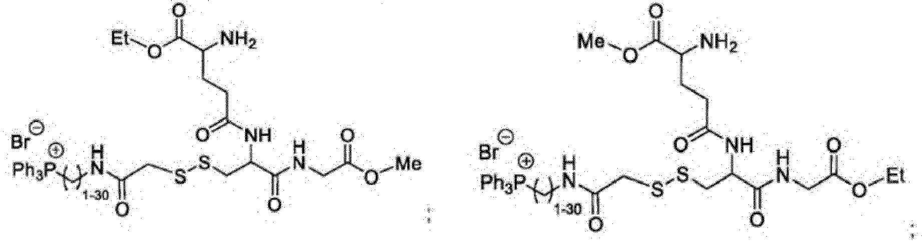

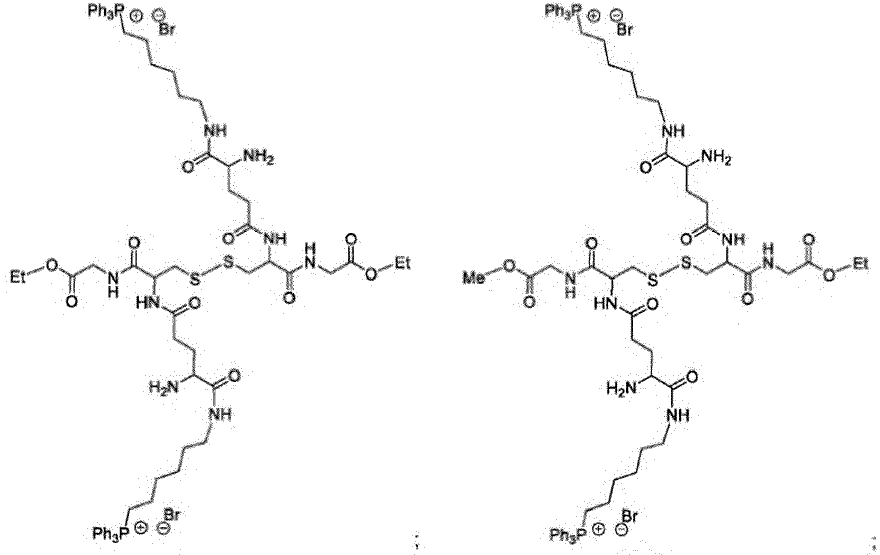

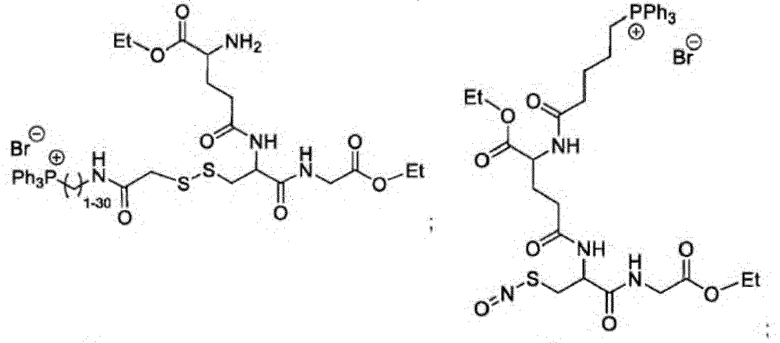

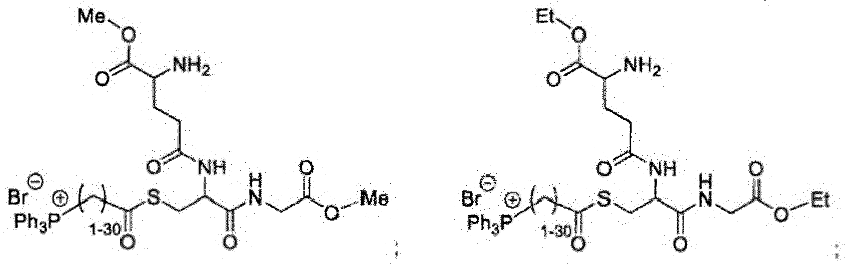

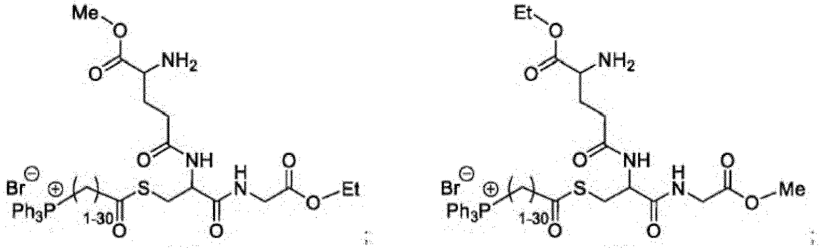

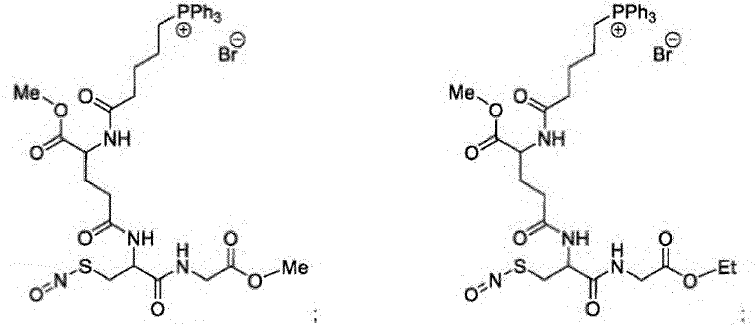

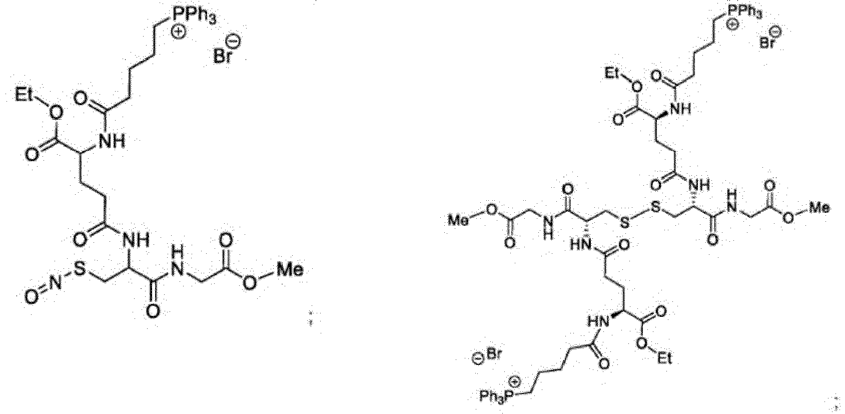

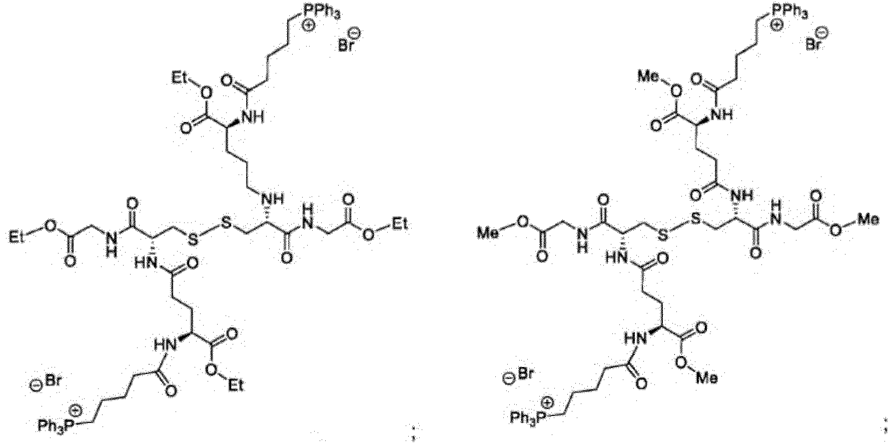

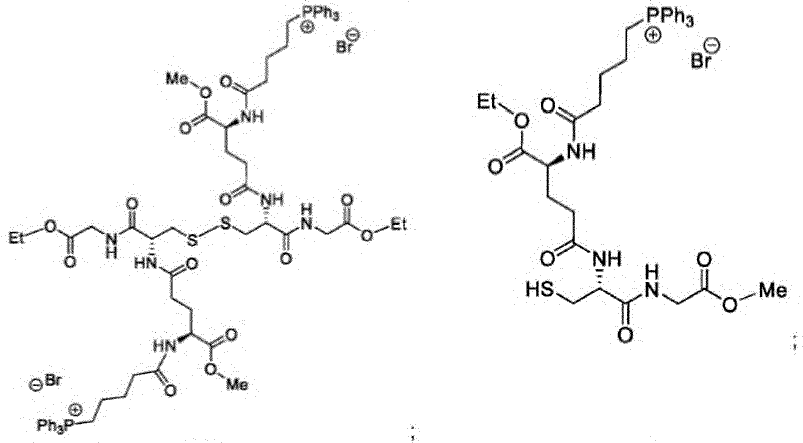

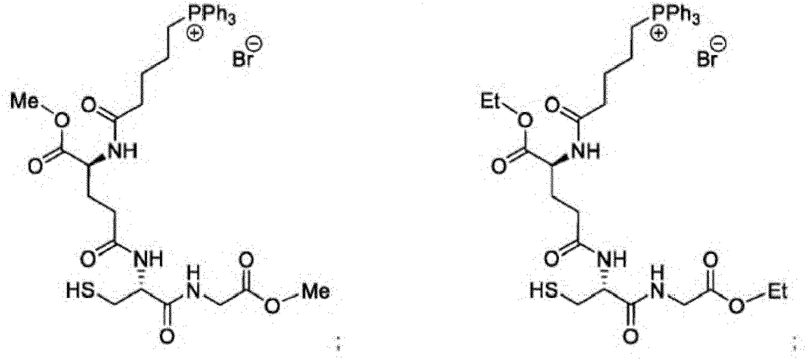

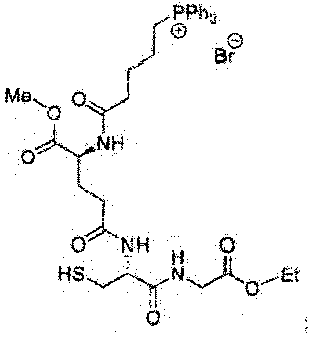

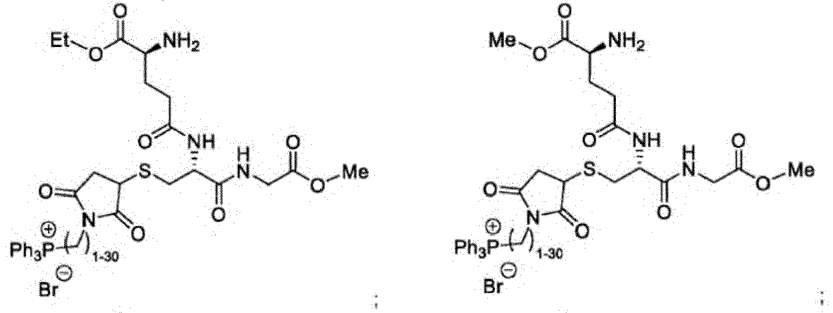

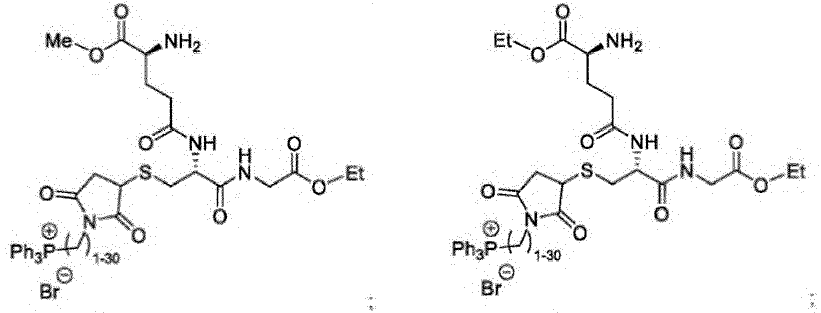

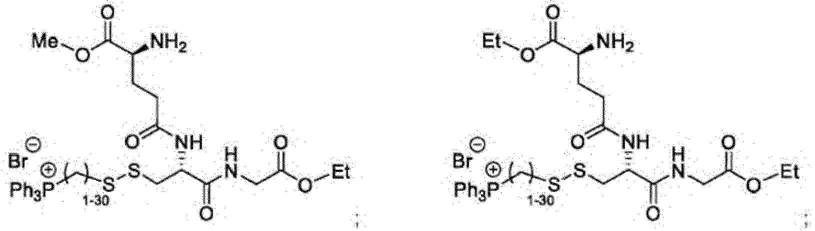

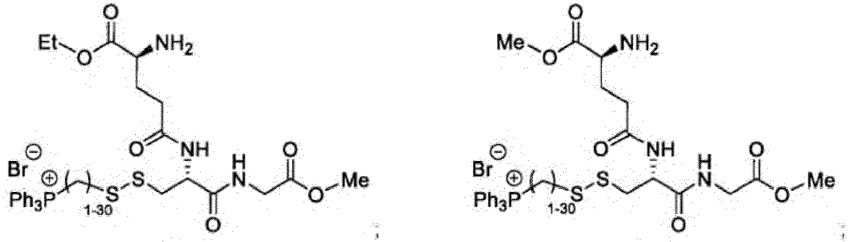

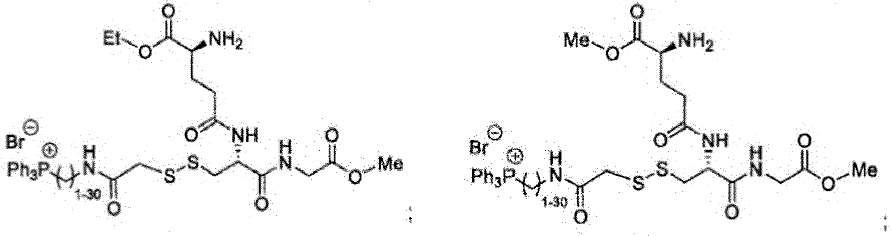

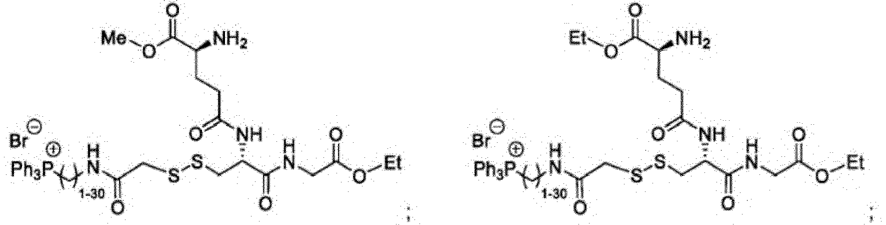

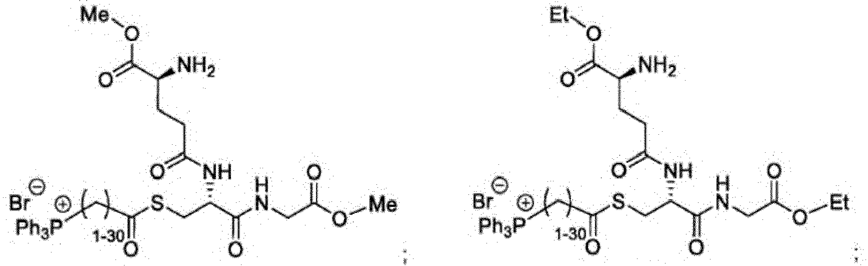

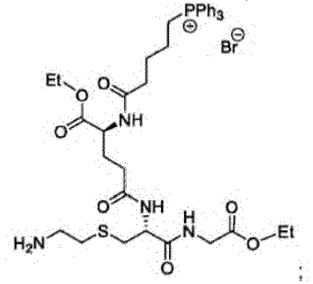

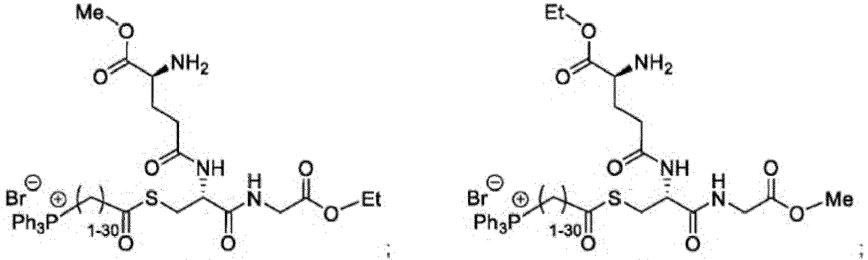

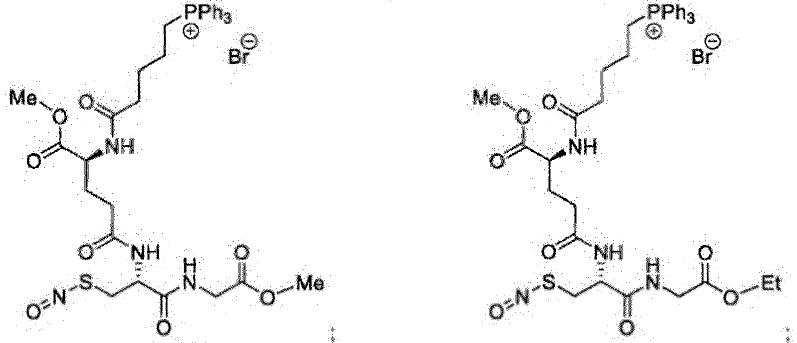

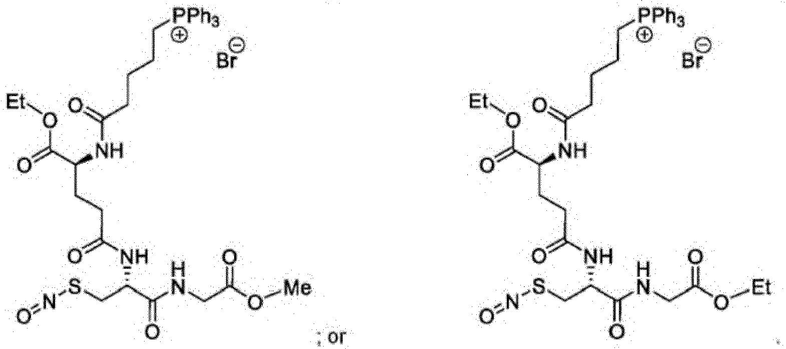

5. Pharmazeutische Zusammensetzung, umfassend:
eine Verbindung nach einem der Ansprüche 1 bis 4; und
einen pharmazeutisch annehmbaren Träger.

6. Pharmazeutische Zusammensetzung nach Anspruch 5, wobei die Zusammensetzung ferner einen oder mehrere zusätzliche Wirkstoffe umfasst,
wobei optional der eine oder die mehreren zusätzlichen Wirkstoffe ausgewählt ist aus N-Acetylcystein, Vitamin E, Vitamin C und Diacetyl-bis(4-methylthiosemicarbazonato)-kupferII.

7. Verbindung nach einem der Ansprüche 1 bis 4 oder eine Zusammensetzung nach Anspruch 5 oder 6 zur Verwendung in einem Verfahren der Behandlung eines Menschen oder eines Tiers.

8. Verbindung nach einem der Ansprüche 1 bis 4 oder eine pharmazeutische Zusammensetzung nach Anspruch 5 oder 6 zur Verwendung
(i) zur Behandlung, Linderung und/oder Prävention einer Krankheit oder eines Zustands im Zusammenhang mit einem niedrigen oder verringerten Glutathionspiegel, optional verursacht durch einen niedrigen oder mitochondrialen Glutathionspiegel; oder
(ii) zur Prävention oder Linderung der Attenuation der Elektronentransportkette (ETC) -Aktivität, der allosterischen Hemmung von Dicarboxylat-Transport, der Entkopplung eines GSH/GSSG-Redox-Netzwerks von einem NADH/NAD⁺-Redoxpaar, der Mitochondrien-Anfälligkeit für Oxidantien, die aus einer ETC entstammen, der erhöhten Anfälligkeit für Medikamente und Toxine, der Initiierung von Permeabilität-Porenöffnung und Freisetzung von pro-apoptotischen Faktoren oder einer Kombination davon; oder
(iii) zur Behandlung, Linderung oder Prävention von neurodegenerativer Erkrankung, Schlaganfall, Diabetes, Herz-Kreislauf-Erkrankung, Krebs, Muskelverletzungen, Muskelmyopathien, Virusinfektionen, Parodontose, Gefäßschäden, Nierenerkrankung, Ischämie-Reperfusionsschaden, Wundheilungsstörungen, Entzündung, Schizophrenie und anderen neuropsychologischen Störungen, Acetaminophen-Toxizität, akuter Alkoholtoxizität, chronischer Alkoholtoxizität oder einer Kombination davon.

9. Verfahren des Herstellens einer Verbindung nach Anspruch 1, umfassend:
Aussetzen von Glutationdisulfid unter Thionylchlorid und einen Alkohol, ausgewählt aus Methanol oder Ethanol, um ein geschütztes Glutathiondisulfid zu bilden;
Aussetzen des geschützten Glutationdisulfids unter (4-Carboxybutyl)triphenylphosphoniumbromid, N-(3-Dimethylaminopropyl)-N'-ethylcarbodiimid·HCl, Hydroxybenzotriazol, di-Isopropylethylamin und ein Alkohollösungsmittel, um eine Verbindung mit der folgenden Struktur zu bilden 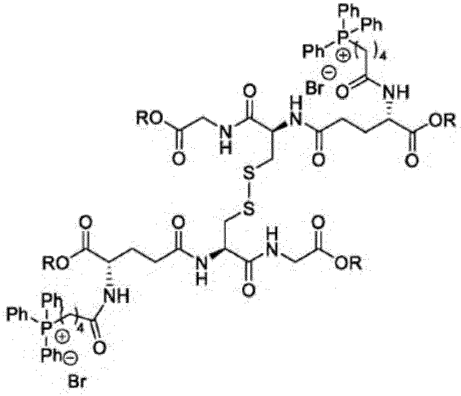
wobei R Methyl oder Ethyl ist.

## Revendications

1. Composé ayant une structure qui satisfait à l'une quelconque des formules 2A-2D ou 5A ou 5B 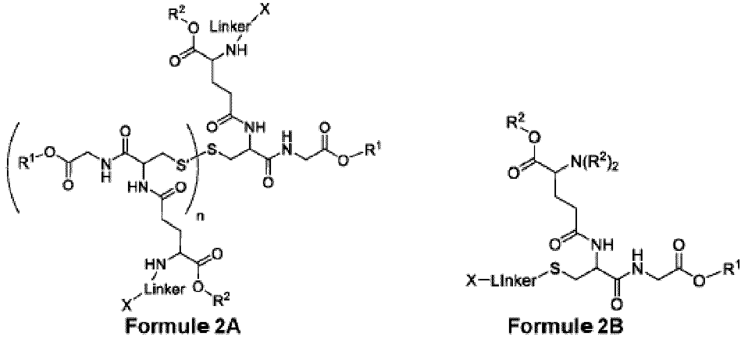 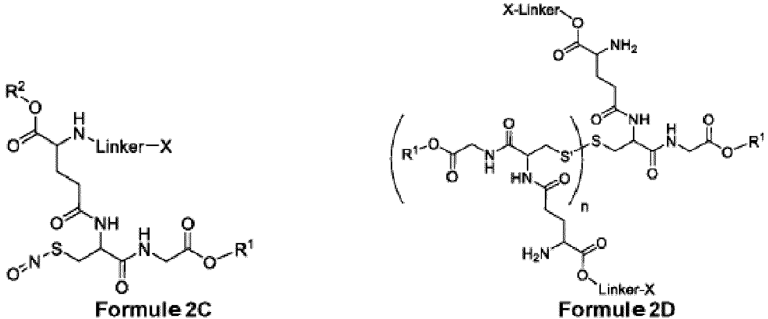 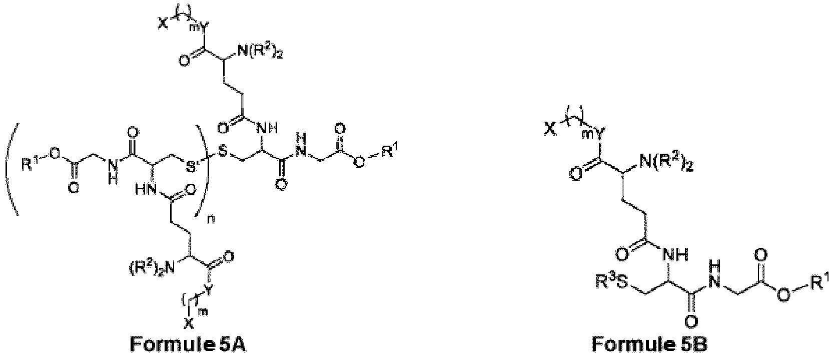 dans lequel
le composé comprend un contre-ion négativement chargé pharmaceutiquement acceptable, ;
n est 0 ou 1 et lorsque n est 0, l'atome S est lié à un atome d'hydrogène, un groupe nitroso pour fournir un groupe thio-nitroso, un groupe aliphatique pour fournir un composé contenant thioéther, ou un groupe cétone pour fournir un composé contenant thioester ;
chaque R¹ est indépendamment sélectionné parmi aliphatique, aryle, hétéroaliphatique, aliphatique-aryle, hétéroaryle, aliphatique-hétéroaryle, hétéroaliphatique-aryle, ou hétéroaliphatique-hétéroaryle ;
chaque R² est indépendamment sélectionné parmi hydrogène ou aliphatique, aryle, hétéroaliphatique, aliphatique-aryle, hétéroaryle, aliphatique-hétéroaryle, hétéroaliphatique-aryle, ou hétéroaliphatique-hétéroaryle ;
R³ est sélectionné parmi hydrogène, un groupe protecteur, un groupe nitroso, ou un groupe linker-X, dans lequel le groupe protecteur est sélectionné parmi aliphatique, aryle, hétéroaliphatique, aliphatique-aryle, hétéroaryle, aliphatique-hétéroaryle, hétéroaliphatique-aryle, ou hétéroaliphatique-hétéroaryle ;
chaque groupe linker-X est sélectionné parmi -C(O)R^{c}X, -[(CH₂)₂O]ₘX, -C(=NH₂⁺)NR^{c}X, -CH₂C(O)NHRC²X, -SR^{c}X, ou 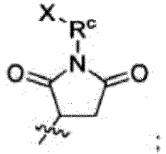
dans lequel chaque R^{c} est indépendamment sélectionné parmi aliphatique, aryle, hétéroaliphatique, aliphatique-aryle, hétéroaryle, aliphatique-hétéroaryle, hétéroaliphatique-aryle, ou hétéroaliphatique-hétéroaryle ; chaque X est indépendamment sélectionné parmi -P⁺(R^{d})₃, dans lequel chaque R^{d} peut indépendamment être sélectionné parmi hydrogène, aliphatique, aryle, ou aliphatiquearyle ; Y est oxygène ou -NH ; et chaque de m est indépendamment 1 à 30 ; optionnellement dans lequel
(i) chaque R¹ et R² est indépendamment méthyle ou éthyle ;
(ii) chaque linker est indépendamment sélectionné parmi -C(O)R^{c}-, -[(CH_{c})₂O]ₘ-, -C(=NH₂⁺)NR^{c}-, -CH₂C(O)NHR^{c}-, -SR^{c}-, dans lequel chaque R^{c} est indépendamment sélectionné parmi aliphatique, aryle, hétéroaliphatique, aliphatique-aryle, hétéroaryle, aliphatique-hétéroaryle, hétéroaliphatique-aryle, ou hétéroaliphatique-hétéroaryle ; et m est de 1 à 30 ; ou
(iii) X est sélectionné parmi -P⁺(Ph)₃.

2. Composé selon la revendication 1, dans lequel le contre-ion négativement chargé pharmaceutiquement acceptable est un mésylate.

3. Composé selon la revendication 1, dans lequel le composé satisfait à l'une quelconque des formules 3B, 3A, 4A, ou 4B

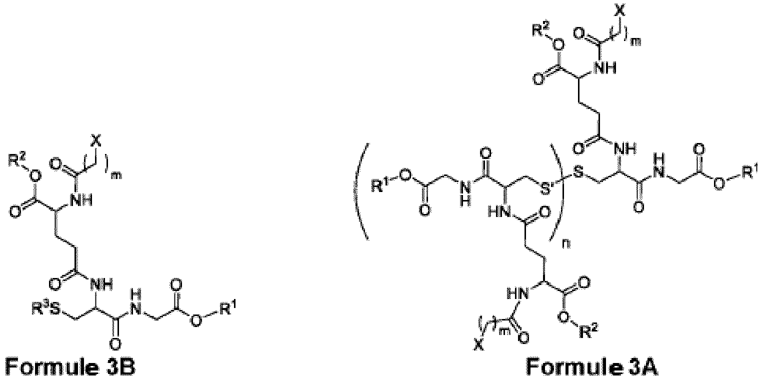

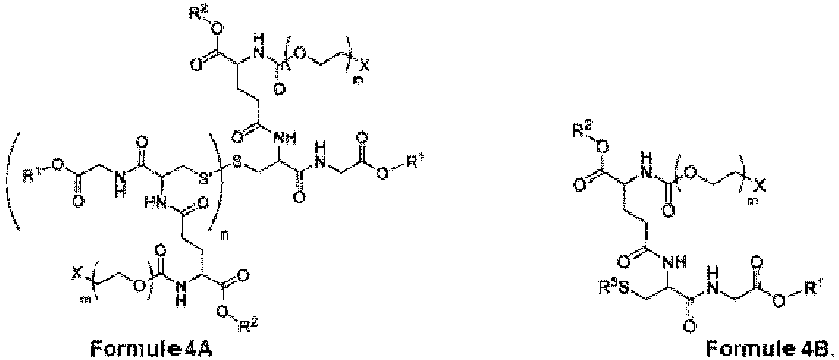

4. Composé selon la revendication 1, dans lequel le composé est sélectionné parmi

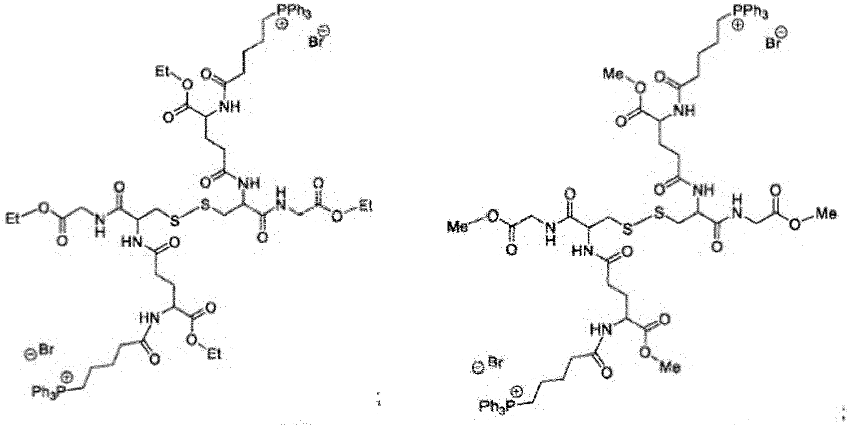

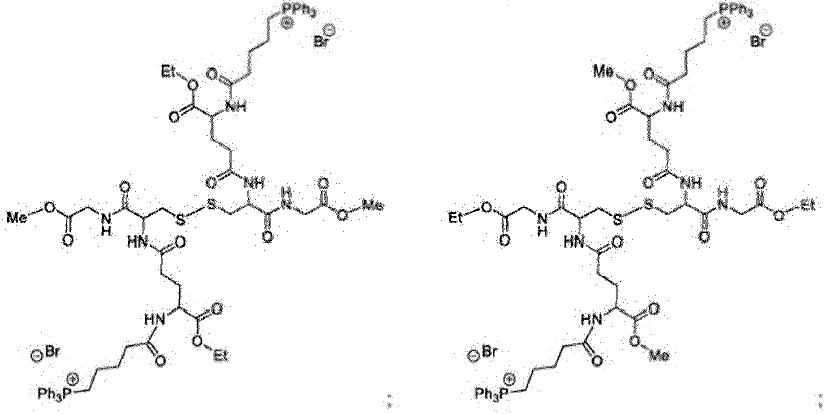

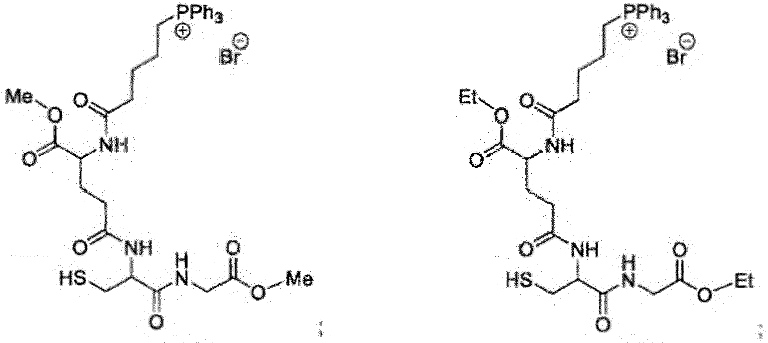

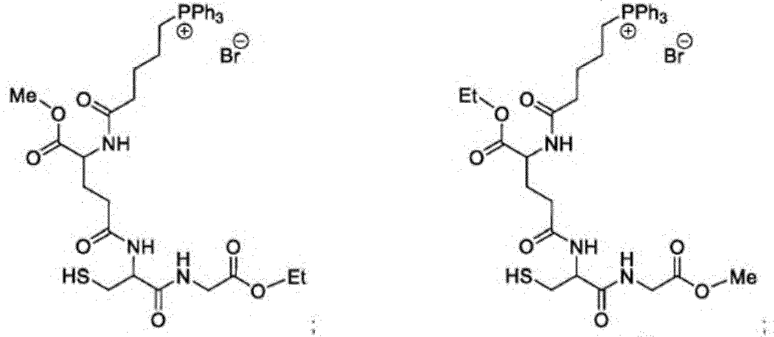

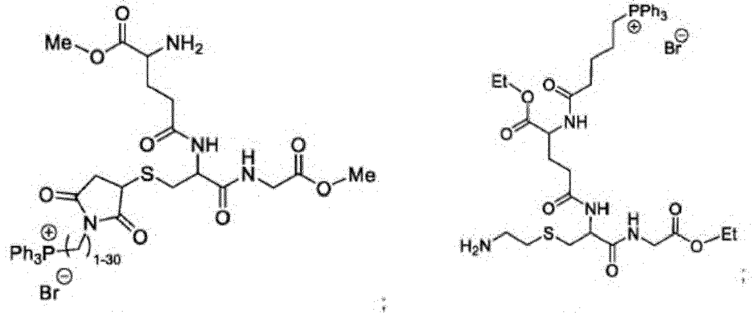

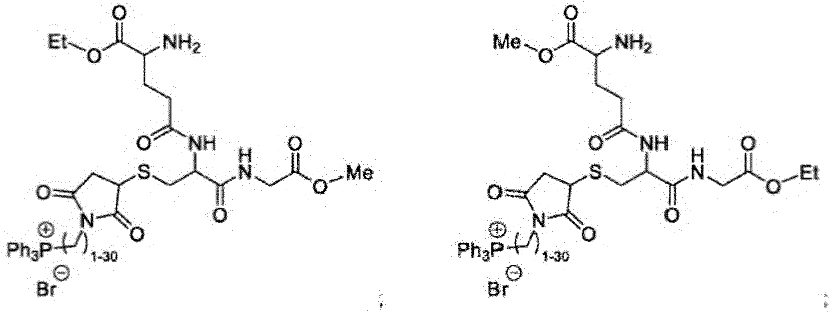

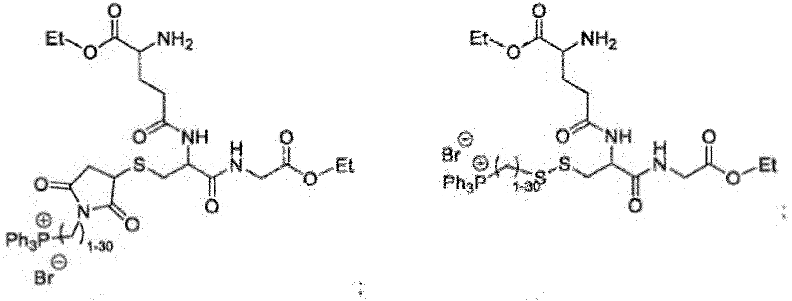

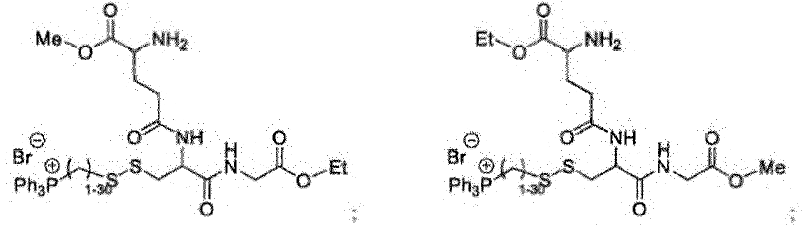

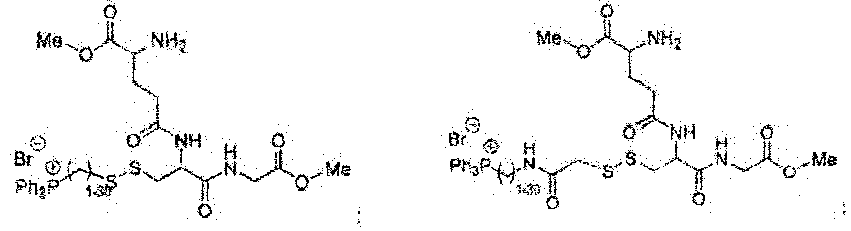

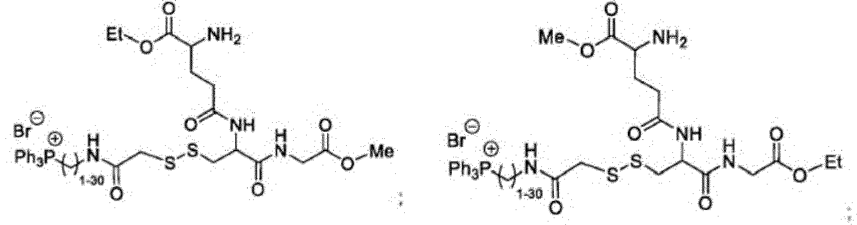

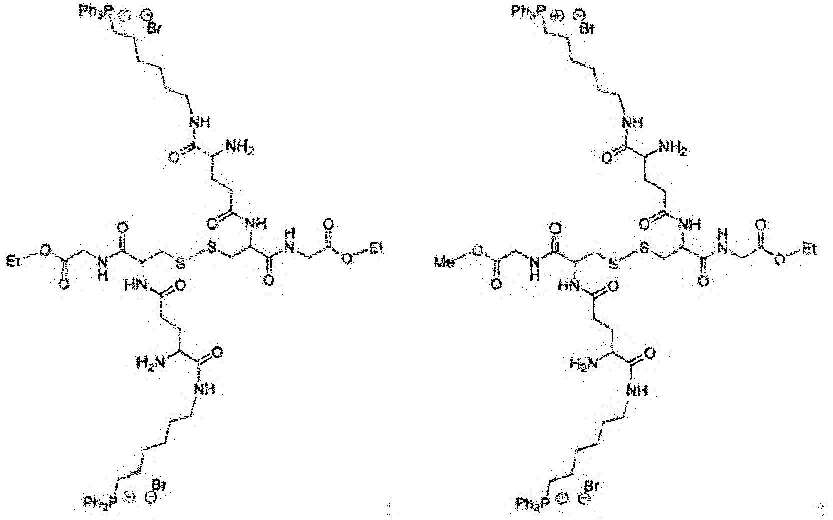

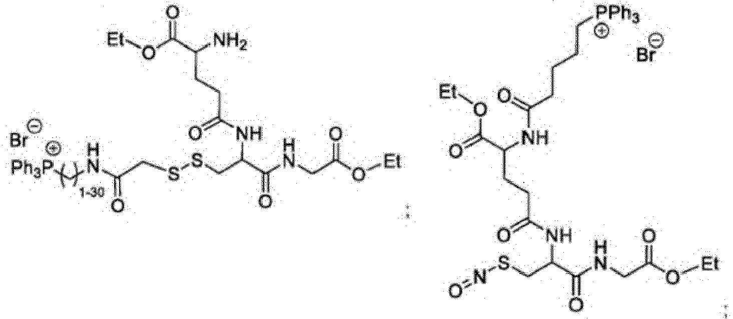

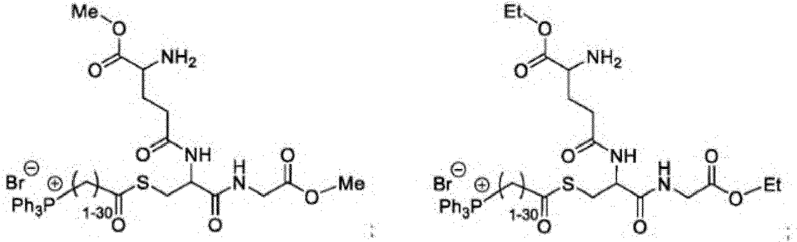

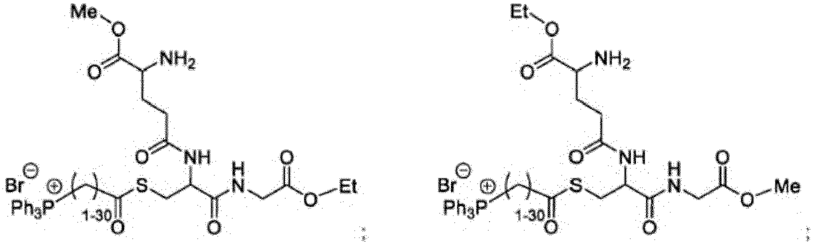

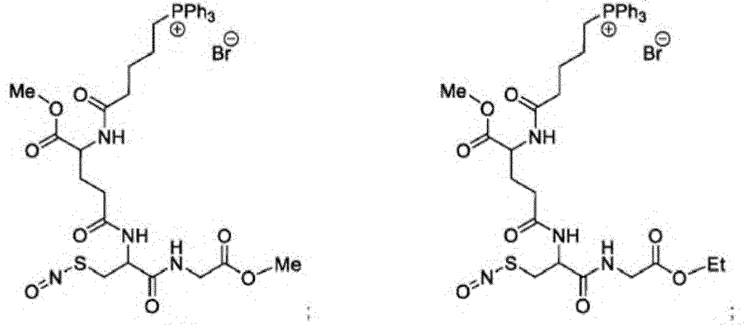

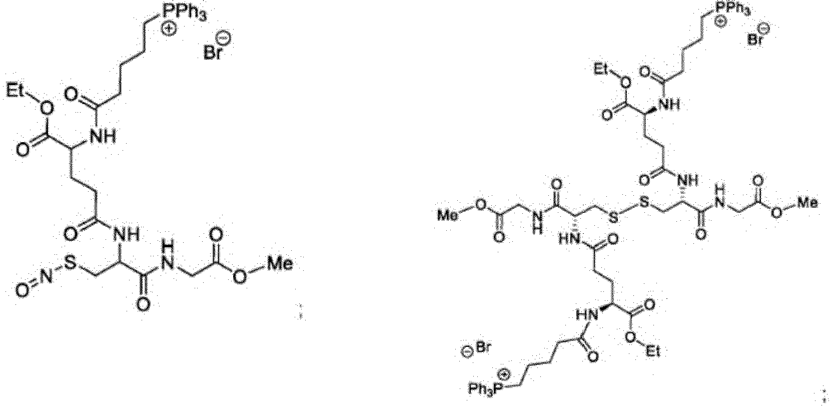

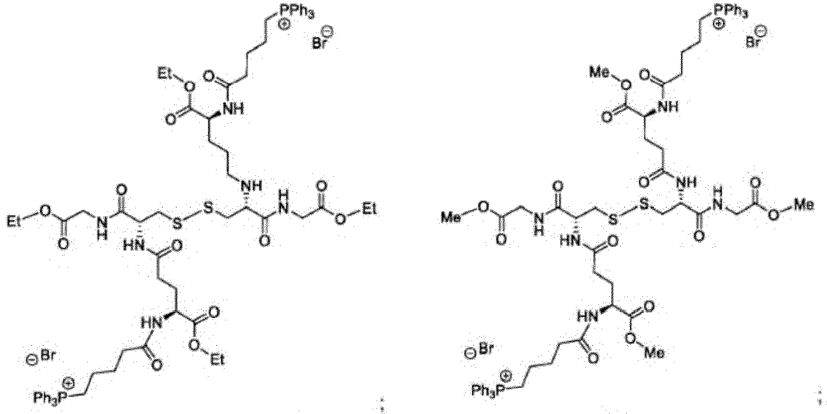

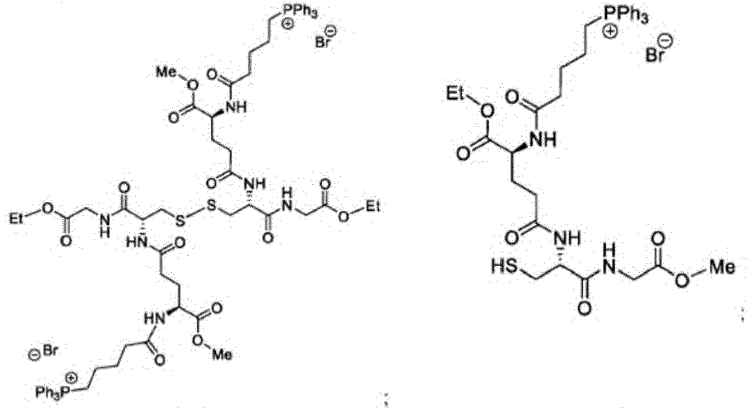

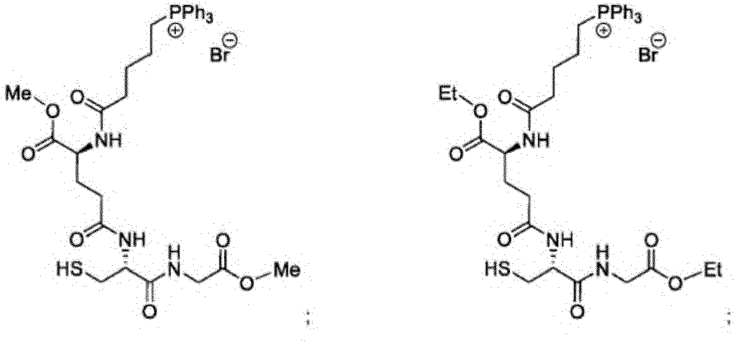

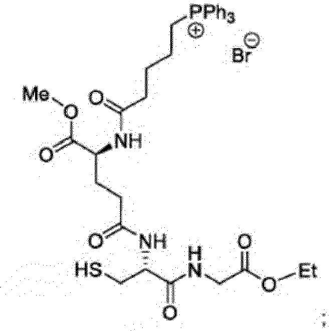

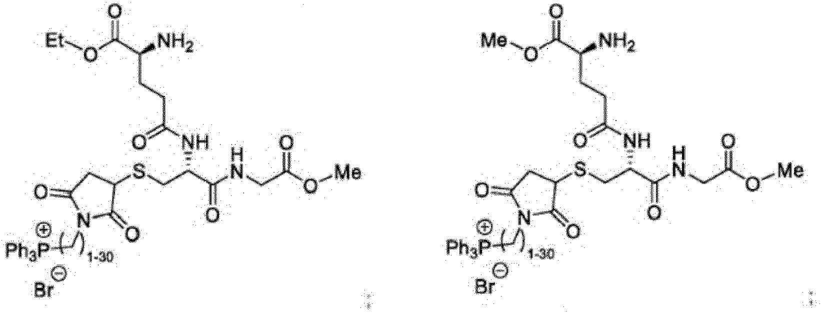

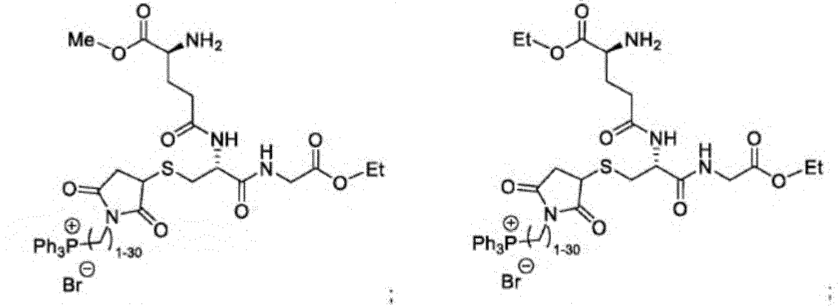

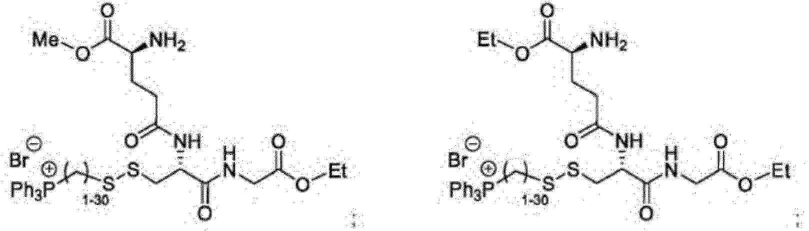

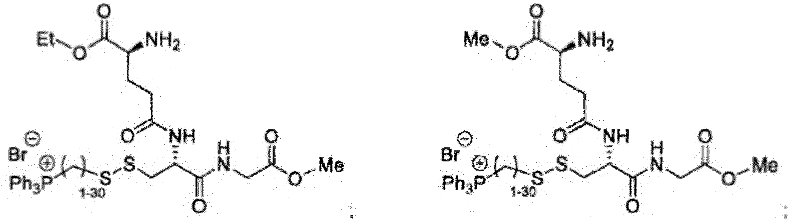

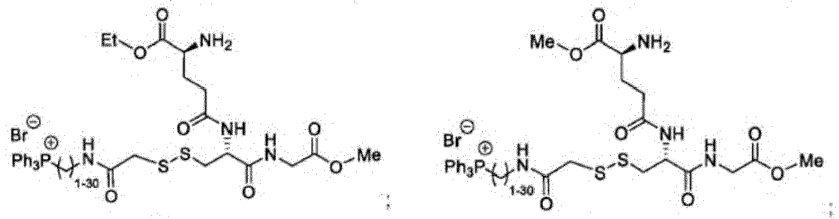

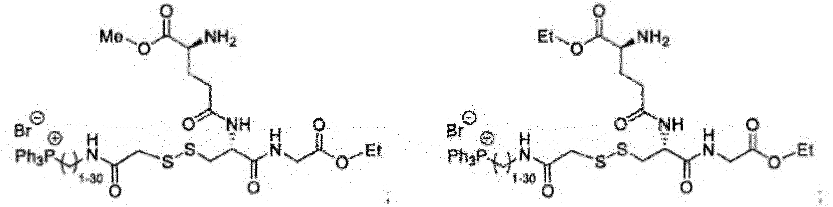

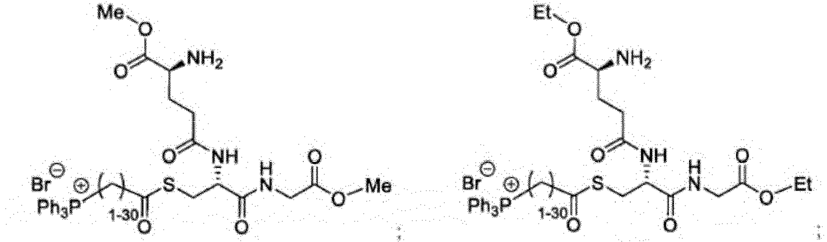

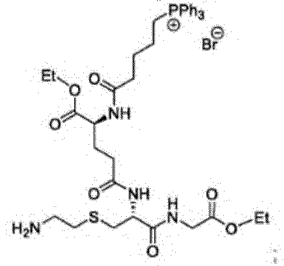

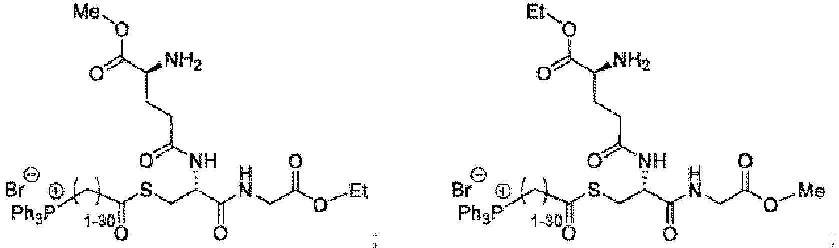

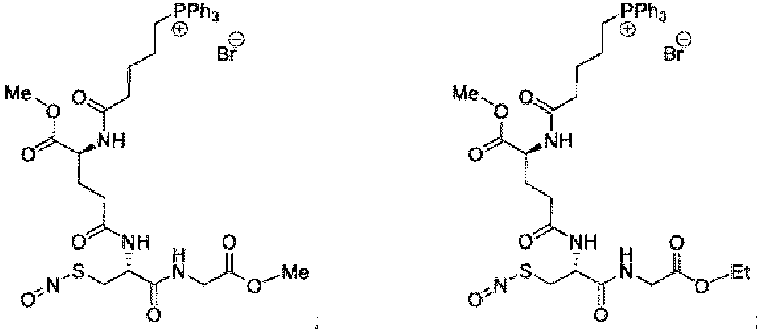

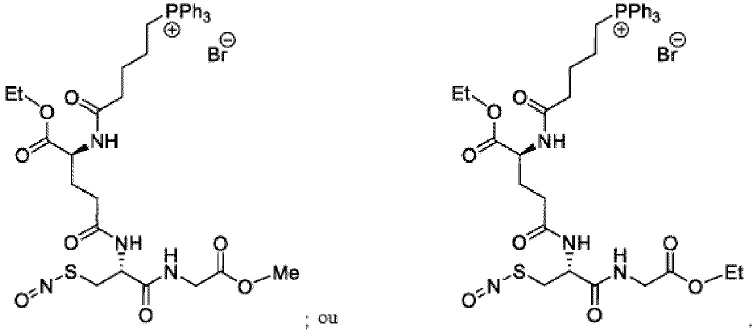

5. Composition pharmaceutique, comprenant :
un composé selon l'une quelconque des revendications 1 à 4 ; et
un vecteur pharmaceutiquement acceptable.

6. Composition pharmaceutique selon la revendication 5, dans laquelle la composition comprend en outre un ou plusieurs agents thérapeutiques supplémentaires,
optionnellement dans laquelle l'un ou les plusieurs agents thérapeutiques supplémentaires sont sélectionnés parmi N-acétylcystéine, vitamine E, vitamine C, et diacétyl-bis(4-méthylthiosemicarbazonato)cuivreII.

7. Composé selon l'une quelconque des revendications 1 à 4 ou composition selon la revendication 5 ou 6 pour l'utilisation dans un procédé de traitement d'un humain ou d'un animal.

8. Composé selon l'une quelconque des revendications 1 à 4 ou composition pharmaceutique selon la revendication 5 ou 6 pour l'utilisation
(i) pour traiter, améliorer, et/ou empêcher une maladie ou condition associée à des niveaux faibles ou réduits de glutathion, optionnellement causée par des niveaux faibles de glutathion mitochondriale ; ou
(ii) pour empêcher ou mitiger l'atténuation d'activité de chaîne de transport d'électrons (ETC), l'inhibition allostérique de transport de dicarboxylate, le découplage d'un réseau redox GSH/GSSG à partir d'un couple redox NADH/NAD⁺, la susceptibilité mitochondriale à des oxydants provenant d'une ETC, la susceptibilité améliorée aux médicaments et toxines, l'initiation d'ouverture de pores à perméabilité et libération de facteurs pro-apoptotiques, ou une association de ceux-ci ; ou
(iii) pour traiter, améliorer, ou empêcher : neuro-dégénérescence, accident vasculaire cérébral, diabète, maladie cardiovasculaire, cancer, lésions musculaires, myopathies musculaires, infections virales, maladie parodontale, déficience vasculaire, maladie rénale, lésion ischémie-reperfusion, problèmes de cicatrisation de blessure, inflammation, schizophrénie et autres troubles neuropsychologiques, toxicité acétaminophène, toxicité alcoolique aigue, toxicité alcoolique chronique, ou association de ceux-ci.

9. Procédé de fabrication d'un composé selon la revendication 1, comprenant :
l'exposition de disulfure de glutathion à du chlorure de thionyle et un alcool sélectionné parmi méthanol ou éthanol pour former un disulfure de glutathion protégé ;
l'exposition du disulfure de glutathion protégé à bromure de (4-carboxybutyl)triphénylphosphonium, N-(3-diméthylaminopropyl)-N'-éthylcarbodiimide·HCl, hydroxybenzotriazole, di-isopropyléthylamine, et un solvant alcoolique pour former un composé ayant une structure 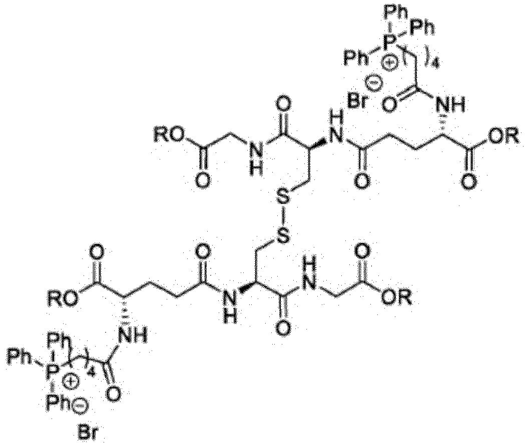
R étant méthyle ou éthyle.
